# EUROPEAN PATENT APPLICATION

(11) **EP 1 728 862 A1**
(43) Date of publication of application: **06.12.2006**
(21) Application number: 06005274.3
(22) Date of filing: 16.03.2001
(51) Int. Cl.: C12N 15/11, C12N 15/63, C12Q 1/68, A61K 31/7105, A01K 67/027

(54) **Methods and compositions for RNA interference**

(30) Priority: 16.03.2000 US 189739 P; 24.10.2000 US 243097 P
(62) Divisional of application: 01918752.5
(71) Applicant: Genetica, Inc., Cambridge, MA 02139 (US); COLD SPRING HARBOR LABORATORY, Cold Spring Harbor New York 11724 (US)
(72) Inventor: Beach, David, Huntington Bay, NY 11743 (US); Caudy, Amy, Melville, NY 11747 (US); Bernstein, Emily, Huntington, NY 11743 (US); Hammond, Scott, Huntington, NY 11743 (US); Hannon, Gregory, Huntington, NY 11743 (US)
(74) Representative: Chapman, Paul Gilmour

(57) **Abstract**

The present invention provides methods for attenuating gene expression in a cell using gene-targeted double stranded RNA (dsRNA). The dsRNA contains a nucleotide sequence that hybridizes under physiologic conditions of the cell to the nucleotide sequence of at least a portion of the gene to be inhibited (the "target" gene).

## Description

### Government Support

Work described herein was supported by National Institutes of Health Grant R01-GM62534. The United States Government may have certain rights in the invention.

### Background of the Invention

"RNA interference", "post-transcriptional gene silencing", "quelling" ― these different names describe similar effects that result from the overexpression or misexpression of transgenes, or from the deliberate introduction of double-stranded RNA into cells (reviewed in Fire A (1999) Trends Genet 15:358-363; Sharp PA (1999) Genes Dev 13:139-141; Hunter C (1999) Curr Biol 9:R440-R442; Baulcombe DC (1999) Curr Biol 9:R599-R601; Vaucheret et al. (1998) Plant J 16:651-659). The injection of double-stranded RNA into the nematode *Coenorhabditis elegans,* for example, acts systemically to cause the post-transcriptional depletion of the homologous endogenous RNA (Fire et al. (1998) Nature 391: 806-811; and Montgomery et al. (1998) PNAS 95:15502-15507). RNA interference, commonly referred to as RNAi, offers a way of specifically and potently inactivating a cloned gene, and is proving a powerful tool for investigating gene function. But the phenomenon is interesting in its own right; the mechanism has been rather mysterious, but recent research ― the latest reported by Smardon et al. (2000) Curr Biol 10:169-178― is beginning to shed light on the nature and evolution of the biological processes that underlie RNAi.

RNAi was discovered when researchers attempting to use the antisense RNA approach to inactivate a *C, elegans* gene found that injection of sense-strand RNA was actually as effective as the antisense RNA at inhibiting gene function. Guo et al. (1995) Cell 81:611-620, Further investigation revealed that the active agent was modest amounts of double-stranded RNA that contaminate *in vitro* RNA preparations. Researchers quickly determined the 'rules' and effects of RNAi. Exon sequences are required, whereas introns and promoter sequences, while ineffective, do not appear to compromise RNAi (though there may be gene-specific exceptions to this rule). RNAi acts systemically ― injection into one tissue inhibits gene function in cells throughout the animal. The results of a variety of experiments, in C. *elegans* and other organisms, indicate that RNAi acts to destabilize cellular RNA after RNA processing.

The potency of RNAi inspired Timmons and Fire (1998 Nature 395: 854) to do a simple experiment that produced an astonishing result. They fed to nematodes bacteria that had been engineered to express double-stranded RNA corresponding to the C. *elegans unc-22* gene. Amazingly, these nematodes developed a phenotype similar to that of *unc-22* mutants that was dependent on their food source. The ability to conditionally expose large numbers of nematodes to gene-specific double-stranded RNA formed the basis for a very powerful screen to select for RNAi-defective *C. elegans* mutants and then to identify the corresponding genes.

Double-stranded RNAs (dsRNAs) can provoke gene silencing in numerous in vivo contexts including *Drosophila, Caenorhabditis elegans,* planaria, hydra, trypanosomes, fungi and plants. However, the ability to recapitulate this phenomenon in higher eukaryotes, particularly mammalian cells, has not be accomplished in the art. Nor has the prior art demonstrated that this phenomena can be observe in cultured eukaryotes cells.

### Summary of the Invention

One aspect of the present invention provides a method for attenuating expression of a target gene in a non-embryonic cell suspended in culture, comprising introducing into the cell a double stranded RNA (dsRNA) in an amount sufficient to attenuate expression of the target gene, wherein the dsRNA comprises a nucleotide sequence that hybridizes under stringent conditions to a nucleotide sequence of the target gene.

Another aspect of the present invention provides a method for attenuating expression of a target gene in a mammalian cell, comprising
(i) activating one or both of a Dicer activity or an Argonaut activity in the cell, and
(ii) introducing into the cell a double stranded RNA (dsRNA) in an amount sufficient to attenuate expression of the target gene, wherein the dsRNA comprises a nucleotide sequence that hybridizes under stringent conditions to a nucleotide sequence of the target gene.
In certain embodiments, the cell is suspended in culture; while in other embodiments the cell is in a whole animal, such as a non-human mammal.

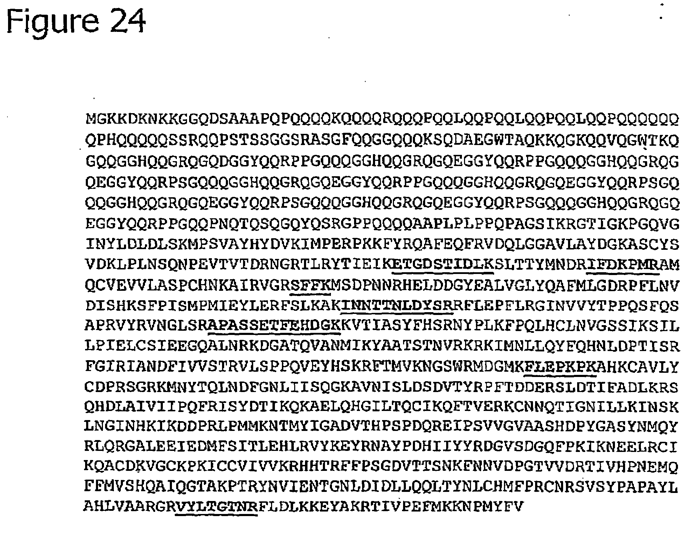

In certain preferred embodiments, the cell is engineered with (i) a recombinant gene encoding a Dicer activity, (ii) a recombinant gene encoding an Argonaut activity, or (iii) both. For instance, the recombinant gene may encode, for a example, a protein which' includes an amino acid sequence at least 50 percent identical to SEQ ID No. 2 or 4; or be defined by a coding sequence hybridizes under wash conditions of 2 x SSC at 22°C to SEQ ID No. 1 or 3. In certain embodiments, the recombinant gene may encode, for a example, a protein which includes an amino acid sequence at least 50 percent identical to the Argonaut sequence shown in Figure 24.

In certain embodiments, rather than use a heterologous expression construct(s), an endogenous Dicer gene or Argonaut gene can be activated, e.g, by gene activation technology, expression of activated transcription factors or other signal transduction protein, which induces expression of the gene, or by treatment with an endogenous factor which upregualtes the level of expression of the protein or inhibits the degradation of the protein.

In certain preferred embodiments, the target gene is an endogenous gene of the cell. In other embodiments, the target gene is an heterologous gene relative to the genome of the cell, such as a pathogen gene, e.g., a viral gene.

In certain embodiments, the cell is treated with an agent that inhibits protein kinase RNA-activated (PKR) apoptosis, such as by treatment with agents which inhibit expression of PKR, cause its destruction, and/or inhibit the kinase activity of PKF.

In certain preferred embodiments, the cell is a primate cell, such as a human cell.

In certain embodiments, the dsRNA is at least 50 nucleotides in length, and preferably 400-800 nucleotides in length.

Still another aspect of the present invention provides an assay for identifying nucleic acid sequences responsible for conferring a particular phenotype in a cell, comprising
(i) constructing a variegated library of nucleic acid sequences from a cell in an orientation relative to a promoter to produce double stranded DNA;
(ii) introducing the variegated dsRNA library into a culture of target cells, which cells have an activated Dicer activity or Argonaut activity;
(iii) identifying members of the library which confer a particular phenotype on the cell, and identifying the sequence from a cell which correspond, such as being identical or homologous, to the library member.

Yet another aspect of the present invention provides a method of conducting a drug discovery business comprising:
(i) identifying, by the assay of claim 16, a target gene which provides a phenotypically desirable response when inhibited by RNAi;
(ii) identifying agents by their ability to inhibit expression of the target gene or the activity of an expression product of the target gene;
(iii) conducting therapeutic profiling of agents identified in step (b), or further analogs thereof, for efficacy and toxicity in animals; and
(iv) formulating a pharmaceutical preparation including one or more agents identified in step (iii) as having an acceptable therapeutic profile.
The method may include an additional step of establishing a distribution system for distributing the pharmaceutical preparation for sale, and may optionally include establishing a sales group for marketing the pharmaceutical preparation.

Another aspect of the present invention provides a method of conducting a target discovery business comprising:
(i) identifying, by the assay of claim 16, a target gene which provides a phenotypically desirable response when inhibited by RNAi;
(ii) (optionally) conducting therapeutic profiling of the target gene for efficacy and toxicity in animals; and
(iii). licensing, to a third party, the rights for further drug development of inhibitors of the target gene.

Another aspect of the invention provides a method for inhibiting RNAi by inhibiting the expression or activity of an RNAi enzyme. Thus, the subject method may include inhibiting the acitivity of Dicer and/or the 22-mer RNA.

Still another aspect relates to the a method for altering the specificity of an RNAi by modifying the sequence of the RNA component of the RNAi enzyme.

Another aspect of the invention relates to purified or semi-purified preparations of the RNAi enzyme or components thereof. In certain embodiments, the preparations are used for identifying compounds, especially small organic molecules, which inhibit or potentiate the RNAi activity. Small molecule inhibitors, for example, can be used to inhibit dsRNA responses in cells which are purposefully being transfected with a virus which produces double stranded RNA.

The dsRNA construct may comprise one or more strands of polymerized ribonucleotide. It may include modifications to either the phosphate-sugar backbone or the nucleoside. The double-stranded structure may be formed by a single self-complementary RNA strand or two complementary RNA strands. RNA duplex formation may be initiated either inside or outside the cell. The dsRNA construct may be introduced in an amount which allows delivery of at least one copy per cell. Higher doses of double-stranded material may yield more effective inhibition. Inhibition is sequence-specific in that nucleotide sequences corresponding to the duplex region of the RNA are targeted for genetic inhibition. dsRNA constructs containing a nucleotide sequences identical to a portion of the target gene is preferred for inhibition. RNA sequences with insertions, deletions, and single point mutations relative to the target sequence have also been found to be effective for inhibition. Thus, sequence identity may optimized by alignment algorithms known in the art and calculating the percent difference between the nucleotide sequences. Alternatively, the duplex region of the RNA may be defined functionally as a nucleotide sequence that is capable of hybridizing with a portion of the target gene transcript.

### Brief Description of the Drawings

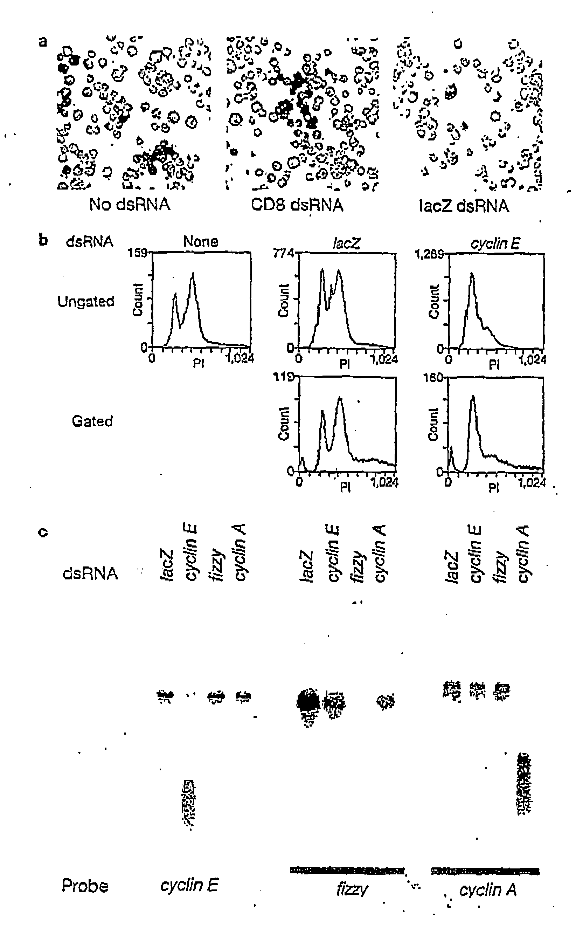

Figure 1; RNAi in S2 cells. a, *Drosophila* S2 cells were transfected with a plasmid that directs *lacZ* expression from the copia promoter in combination with dsRNAs corresponding to either human CD8 or lacZ, or with no dsRNA, as indicated. b, S2 cells were co-transfected with a plasmid that directs expression of a GFP-US9 fusion protein (12) and dsRNAs of either *lacZ* or *cyclin E,* as indicated. Upper panels show FACS profiles of the bulk population. Lower panels show FACS profiles from GFT-positive cells. c, Total RNA was extracted from cells transfected with *lacZ, cyclin E, fizzy or cyclin A* dsRNAs, as indicated. Northern blots were hybridized with sequences not present in the transfected dsRNAs.

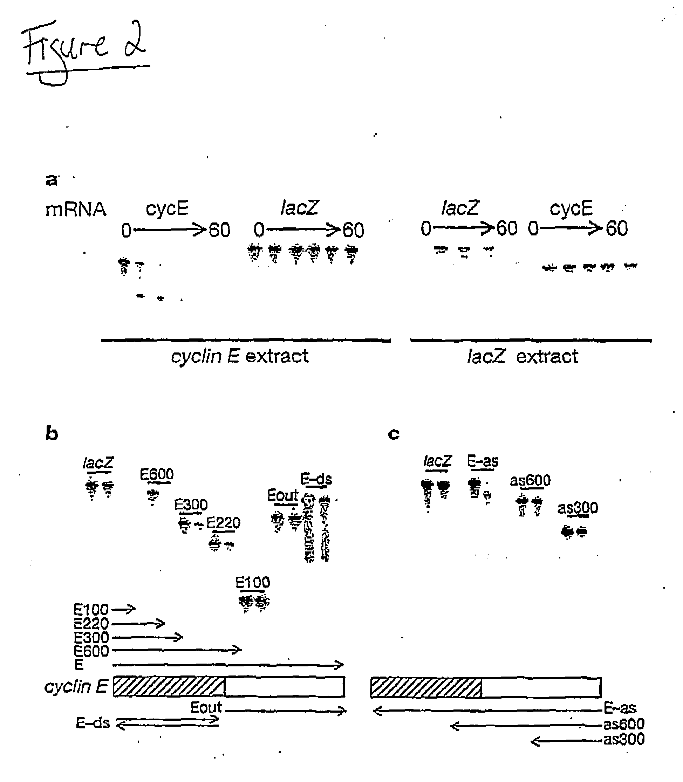

Figure 2: RNAi *in vitro,* a, Transcripts corresponding to either the first 600 nucleotides of *Drosophila cyclin E* (E600) or the first 800 nucleotides of *lacZ* (Z800) were incubated in lysates derived from cells that had been transfected with either *lacZ* or *cyclin E* (cycE) dsRNAs, as indicated. Time points were 0, 10, 20, 30, 40 and 60 min for *cyclin E* and 0, 10, 20, 30 and 60 min for *lacZ.* b, Transcripts were incubated in an extract of S2 cells that had been transfected with *cyclin E* dsRNA (cross-hatched box, below). Transcripts corresponded to the first 800 nucleotides of *lacZ* or the first 600, 300, 220 or 100 nucleotides of *cyclin E,* as indicated. Eout is a transcript derived from the portion of the *cyclin E* cDNA not contained within the transfected dsRNA. E-ds is identical to the dsRNA that had been transfected into S2 cells. Time points were 0 and 30 min. c, Synthetic transcripts complementary to the complete *cyclin E* cDNA (Eas) or the final 600 nucleotides (Eas600) or 300 nucleotides (Eas300) were incubated in extract for 0 or 30 min.

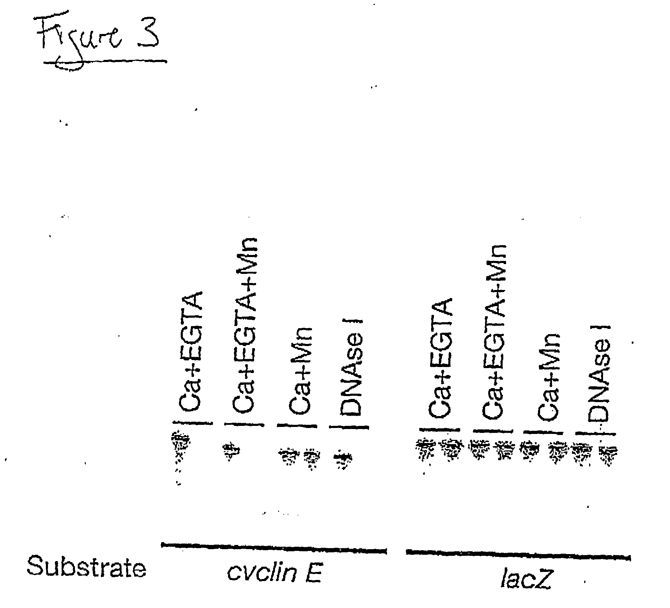

Figure 3: Substrate requirements of the RISC, Extracts were prepared from cells transfected with *cyclin E* dsRNA. Aliquots were incubated for 30 min at 30 °C before the addition of either the *cyclin E* (E600) or *lacZ* (Z800) substrate. Individual 20-µl aliquots, as indicated, were pre-incubated with 1 mM CaCl₂ and 5 mM EGTA, 1 mM CaCl₂, 5 mM EGTA and 60 U of micrococcal nuclease, 1 mM CaCl₂ and 60 U of micrococcal nuclease or 10 U of DNase I (promega) and 5 mM EGTA. After the 30-min pre-incubation, EGTA was added to those samples that lacked it. Yeast tRNA (1 µg) was added to all samples. Time points were at 0 and 30 min.

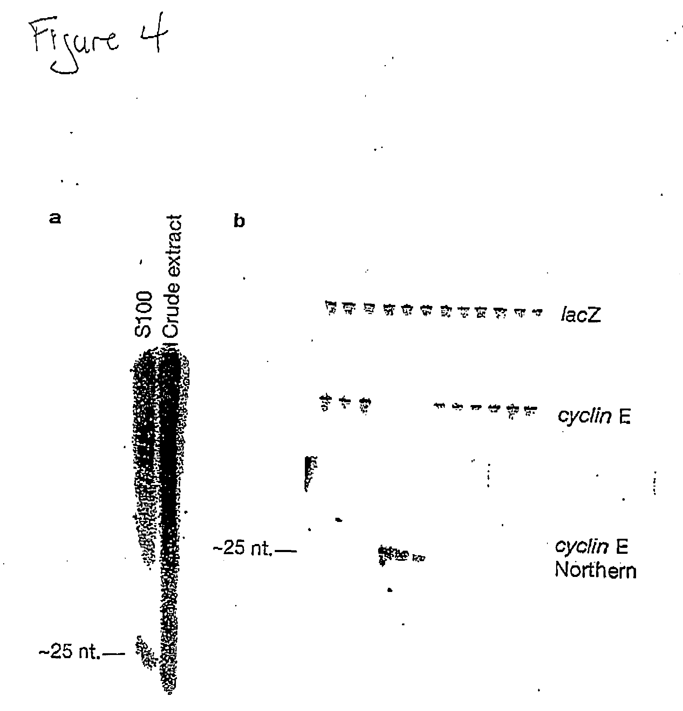

Figure 4: The RISC contains a potential guide RNA. a, Northern blots of RNA from either a crude lysate or the S100 fraction (containing the soluble nuclease activity, see Methods) were hybridized to a riboprobe derived from the sense strand of the *cyclin E* mRNA. b, Soluble *cyclin*-*E*-specific nuclease activity was fractionated as described in Methods. Fractions from the anion-exchange resin were incubated with the lacZ, control substrate (upper panel) or the cyclin E substrate (centre panel). Lower panel, RNA from each fraction was analysed by northern blotting with a uniformly labelled transcript derived from sense strand of the *cyclin E* cDNA. DNA oligonucleotides were used as size marlcers.

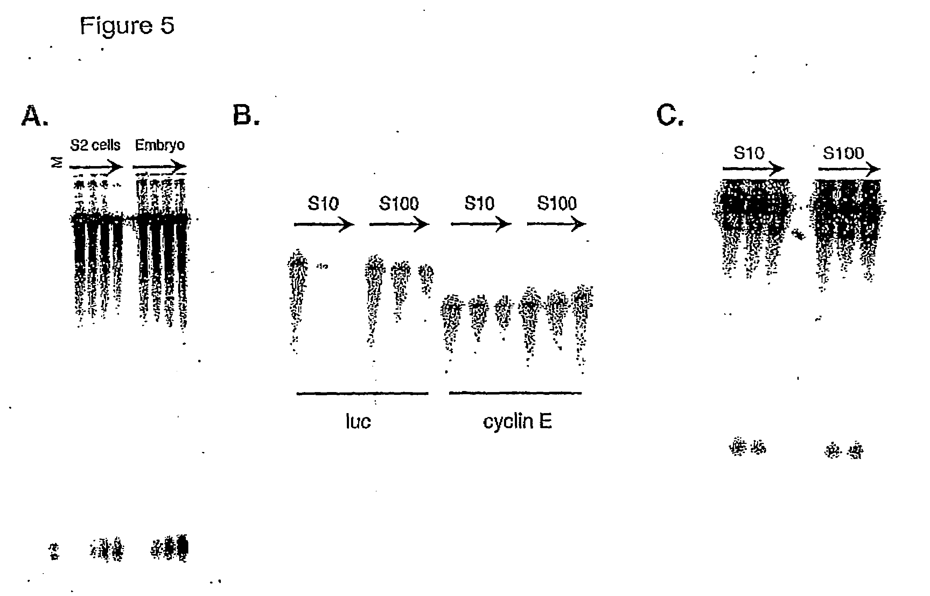

Figure 5: Generation of 22mers and degradation of mRNA are carried out by distinct enzymatic complexes. A. Extracts prepared either from 0-12 hour *Drosophila* embryos or *Drosophila* S2 cells (see Methods) were incubated 0, 15, 30, or 60 minutes (left to right) with a uniformly-labeled double-stranded RNA corresponding to the first 500 nucleotides of the *Drosophila cyclin E* coding region. M indicates a marker prepared by *in vitro* transcription of a synthetic template. The template was designed to yield a 22 nucleotide transcript. The doublet most probably results from improper initiation at the +1 position. B. Whole-cell extracts were prepared from S2 cells that had been transfected with a dsRNA corresponding to the first 500 nt. of the luciferase coding region. S10 extracts were spun at 30,000xg for 20 minutes which represents our standard RISC extract⁶. S100 extracts were prepared by further centrifugation of S10 extracts for 60 minutes at 100,000xg. Assays for mRNA degradation were carried out as described previously⁶ for 0,30 or 60 minutes (left to right in each set) with either a single-stranded luciferase mRNA or a single-stranded cyclin E mRNA, as indicated. C. S10 or S100 extracts were incubated with cyclin E dsRNAs for 0, 60 or 120 minutes (L to R).

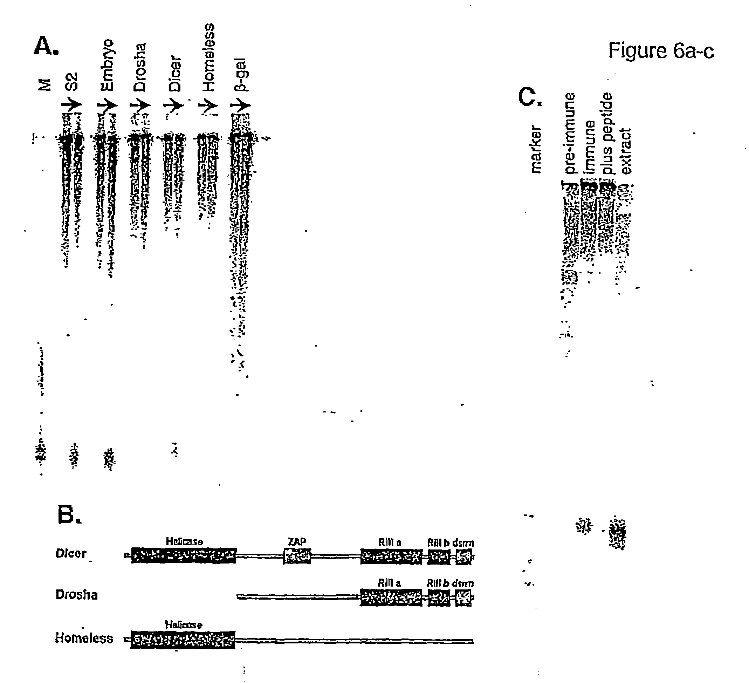

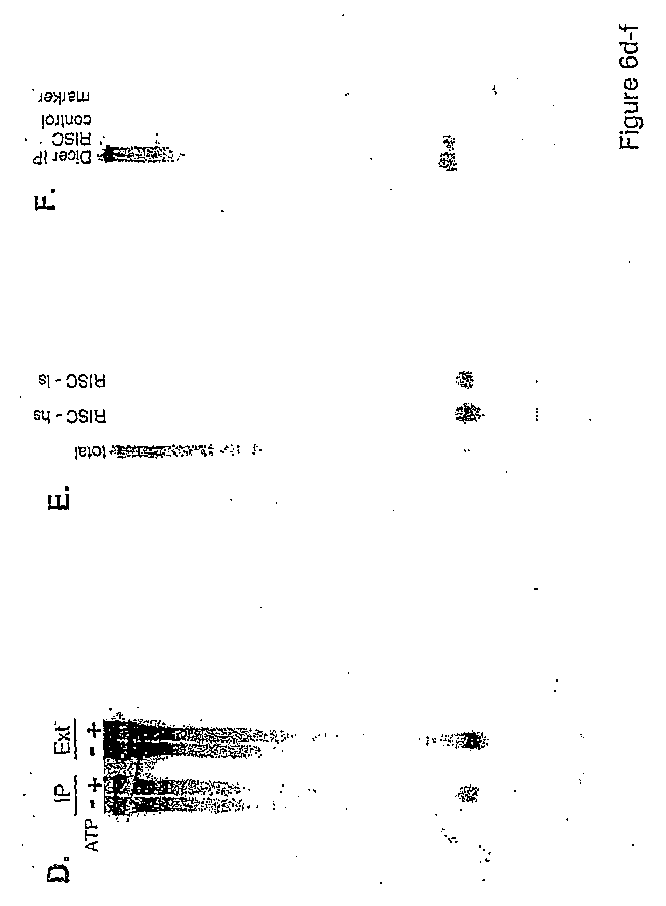

Figure 6: Production of 22mers by recombinant CG4792/Dicer. A. *Drosophila* S2 cells were transfected with plasmids that direct the expression of T7-epitope tagged versions of Drosha, CG4792/Dicer-1 and Homeless. Tagged proteins were purified from cell lysates by immunoprecipitation and were incubated with *cyclin E* dsRNA. For comparison, reactions were also performed in *Drosophila* embryo and S2 cell extracts. As a negative control, immunoprecipitates were prepared from cells transfected with a β-galactosidase expression vector. Pairs of lanes show reactions performed for 0 or 60 minutes. The synthetic marker (M) is as described in the legend to Figure 1. B. Diagrammatic representations of the domain structures of CG4792/Dicer-1, Drosha and Homeless are shown. C. Immunoprecipitates were prepared from detergent lysates of S2 cells using an antiserum raised against the C-terminal 8 amino acids of Drosophila Dicer-1 (CG4792). As controls, similar preparations were made with a pre-immune serum and with an immune serum that had been pre-incubated with an excess of antigenic peptide. Cleavage reactions in which each of these precipitates was incubated with an ~500 nt. fragment of Drosophila cyclin E are shown. For comparsion, an incubation of the substrate in Drosophila embryo extract was electrophoresed in parallel. D. Dicer immunoprecipitates were incubated with dsRNA substrates in the presence or absence of ATP. For comparison, the same substrate was incubated with S2 extracts that either contained added ATP or that were depleted of ATP using glucose and hexokinase (see methods). E. Drosophila S2 cells were transfected with uniformly, 32P-labelled dsRNA corresponding to the first 500 nt. of GFP. RISC complex was affinity purified using a histidine-tagged version of D.m. Ago-2, a recently identified component of the RISC complex (Hammond et aL, in prep). RISC was isolated either under conditions in which it remains ribosome associated (Is, low salt) or under conditions that extract it from the ribosome in a soluble form (hs, high salt)⁶. For comparison, the spectrum of labelled RNAs in the total lysate is shown. F. Guide RNAs produced by incubation of dsRNA with a Dicer immunoprecipitate are compared to guide RNAs present in a affinity-purified RISC complex. These precisely comigrate on a gel that has single-nucleotide resolution. The lane labelled control is an affinity selection for RISC from cell that had been transfected with labeled dsRNA but not with the epitope-tagged D.m. Ago-2.

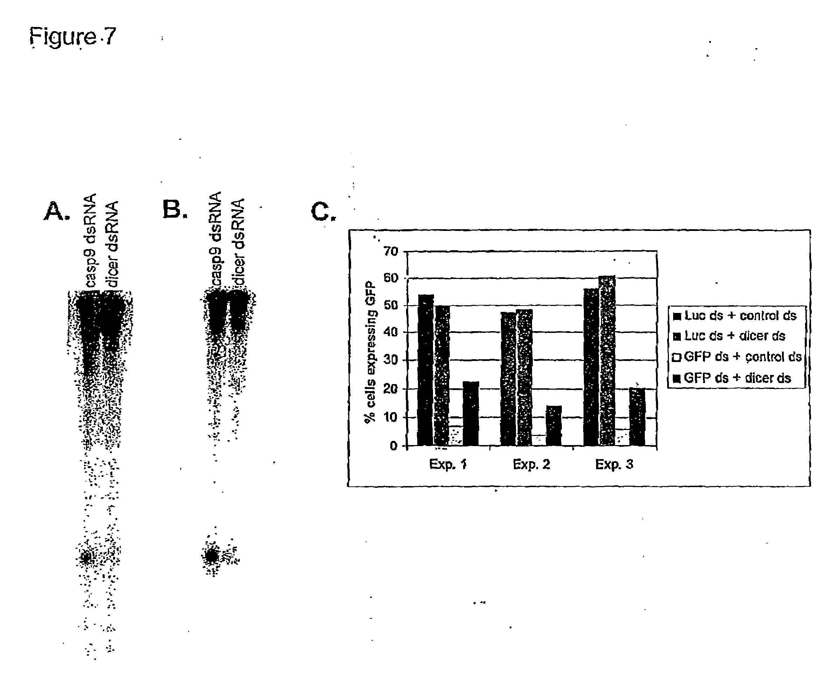

Figure 7: Dicer participates in RNAi. A- Drosophila S2 cells were transfected with dsRNAs corresponding to the two Drosophila Dicers (CG4792 and CG6493) or with a control dsRNA corresponding to murine easpase 9. Cytoplasmic extracts of these cells were tested for Dicer activity. Transfection with Dicer dsRNA reduced activity in lysates by 7.4-fold. B. The Dicer-1 antiserum (CG4792) was used to prepare immunoprecipitates from S2 cells that had been treated as described above. Dicer dsRNA reduced the activity of Dicer-1 in this assay by 6.2-fold. C. Cells that had been transfected two days previously with either mouse caspase 9 dsRNA or with Dicer dsRNA were cotransfected with a GFP expression plasmid and either control, luciferase dsRNA or GFP dsRNA. Three independent experiments were quantified by FACS. A comparison of the relative percentage of GFP-positive cells is shown for control (GFP plasmid plus luciferase dsRNA) or silenced (GFP plamsid plus GFP dsRNA) populations in cells that had previously been transfected with either control (caspase 9) or Dicer dsRNAs.

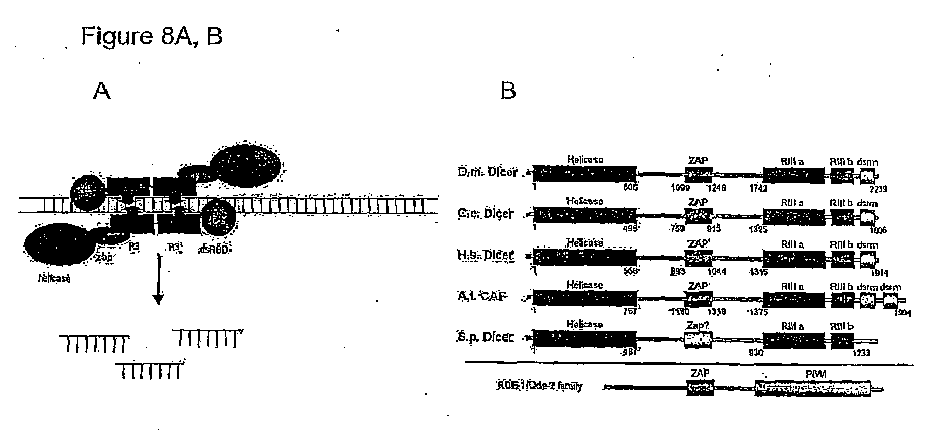

Figure 8: Dicer is an evolutionarily conserved ribonuclease. A. A model for production of 22mers by Dicer. Based upon the proposed mechanism of action of Ribonuclease III, we propose that Dicer acts on its substrate as a dimer. The positioning of the two ribonuclease domains (RIIIa and RIIIb) within the enzyme would thus determine the size of the cleavage product An equally plausible alternative model could be derived in which the RIIIa and RIIIb domains of each Dicer enzyme would cleave in concert at a single position. In this model, the size of the cleavage product would be determined by interaction between two neighboring Dicer enzymes. B. Comparison of the domain structures of potential Dicer homologs in various organisms (*Drosophila -* CG4792, CG6493, *C. elegans -* K12H4.8, *Arabidopsis -* CARPEL FACTORY²⁴, T25K16.4, AC012328_1, human Helicase-MOI²⁵ and *S. pombe -* YC9A_SCHPO). The ZAP domains were identified both by analysis of individual sequences with Pfam²⁷ and by Psi-blast²⁸ searches. The ZAP domain in the putative *S. pombe* Dicer is not detected by PFAM but is identified by Psi-Blast and is thus shown in a different color. For comparison, a domain structure of the RDE1/QDE2/ARGONAUTE family is shown. It should be noted that the ZAP domains are more similar within each of the Dicer and ARGONAUTE families than they are between the two groups. C. An alignment of the ZAP domains in selected Dicer and Argonaute family members is shown. The alignment was produced using ClustalW.

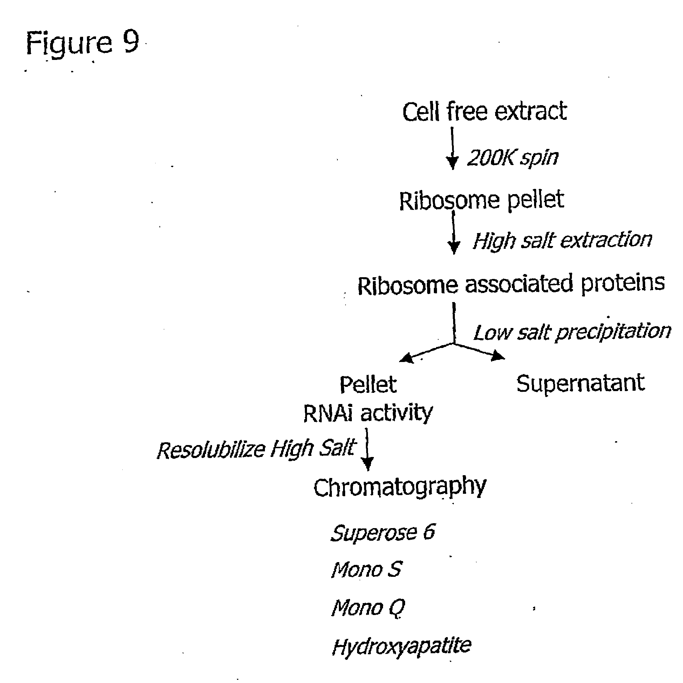

Figure 9: Purification strategy for RISC. (second step in RNAi model).

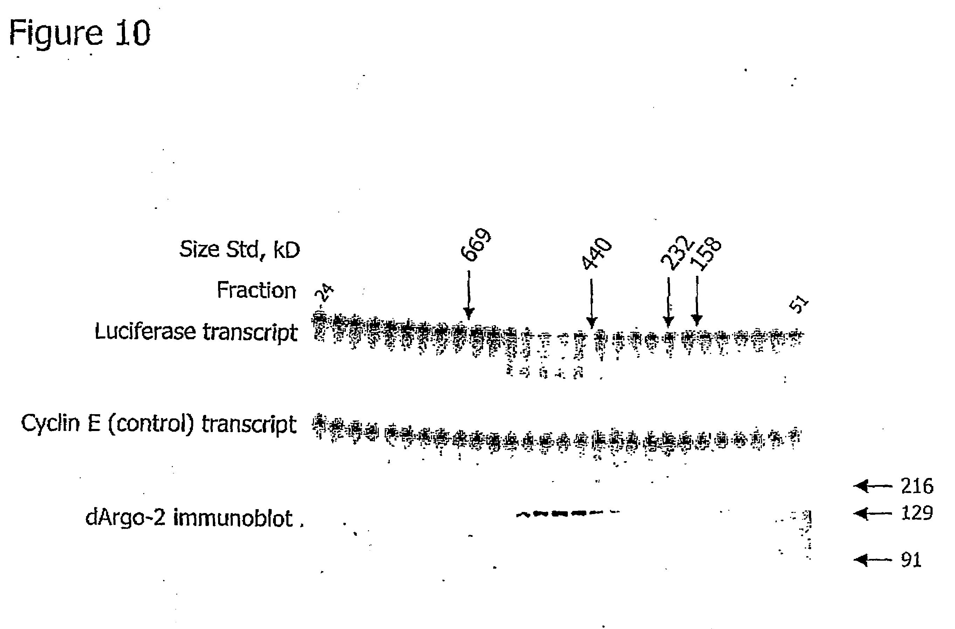

Figure 10: Fractionation of RISC activity over sizing column. Actvity fractionates as 500KD complex. Also, antibody to dm argonaute 2 cofractionates with activity.

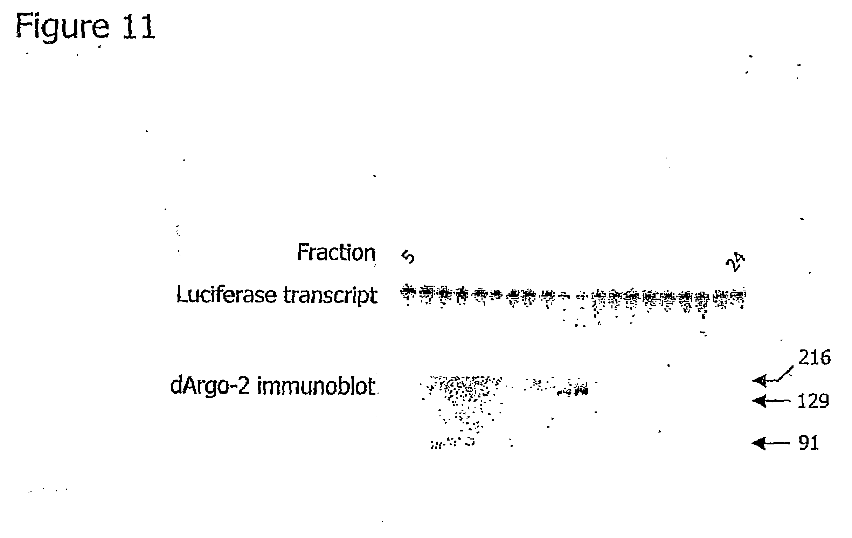

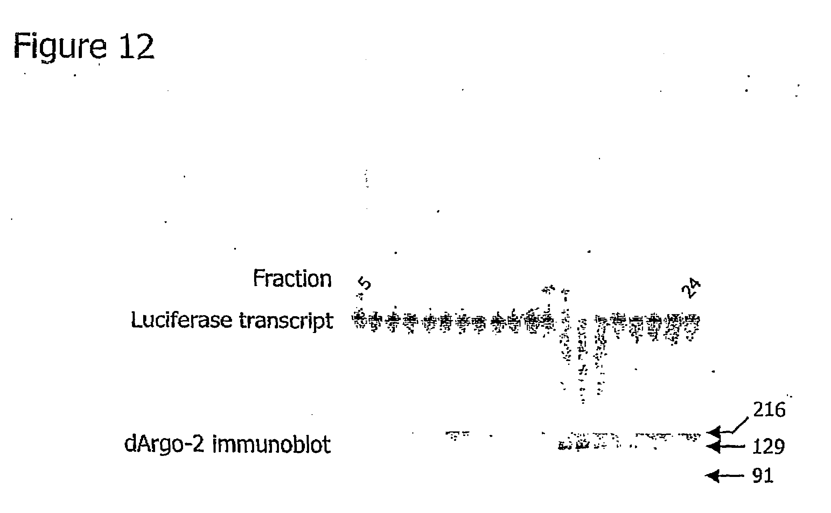

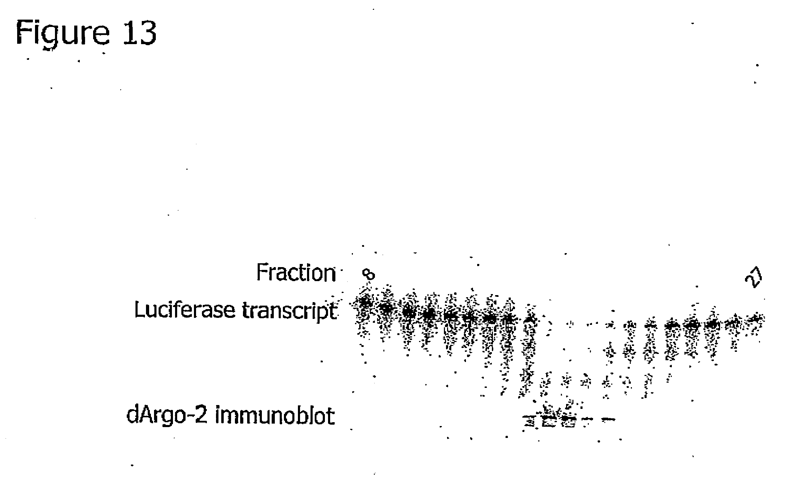

Figure 11-13: Fractionation of RISC over monoS, monoQ, Hydroxyapatite columns. Dm argoaaute 2 protein also cofactionates.

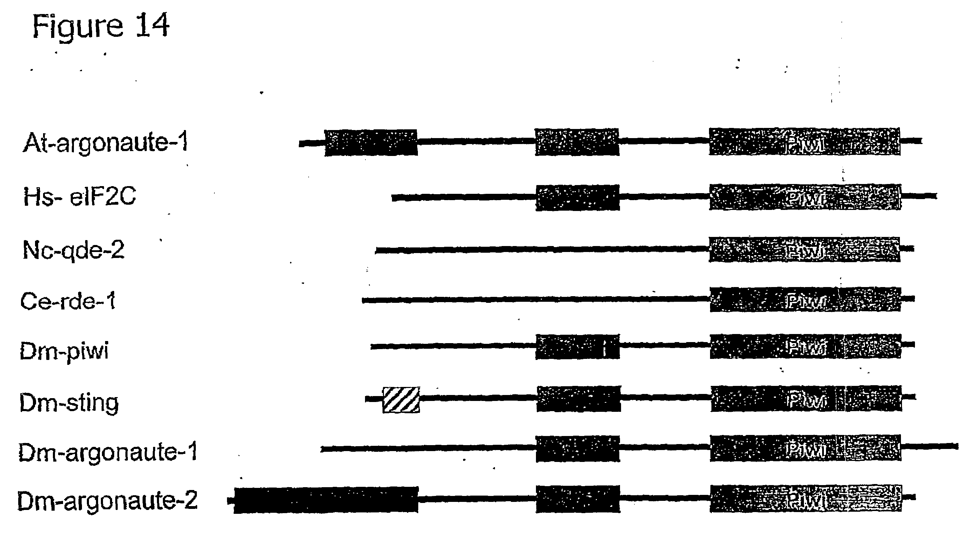

Figure 14: Alignment of dm argonaute 2 with other family members.

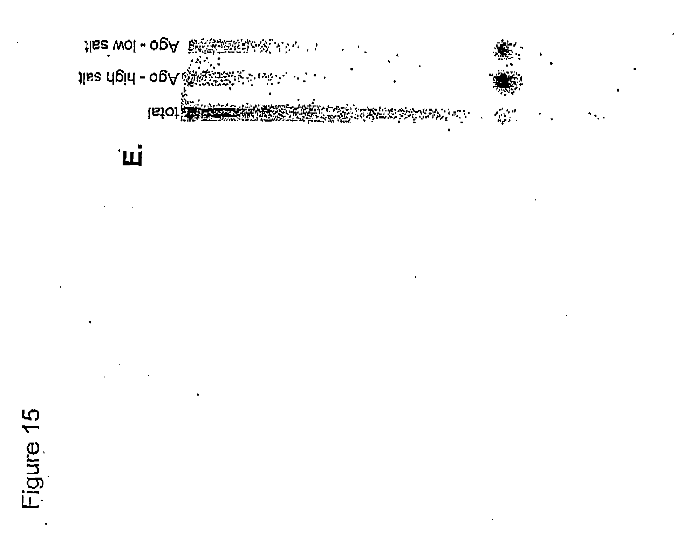

Figure 15: Confirmation of dm argonaute 2. S2 cells were transfected with labeled dsRNA and His tagged argonaute. Argonaute was isolated on nickel agarose and RNA component was identified on 15% acrylamide gel.

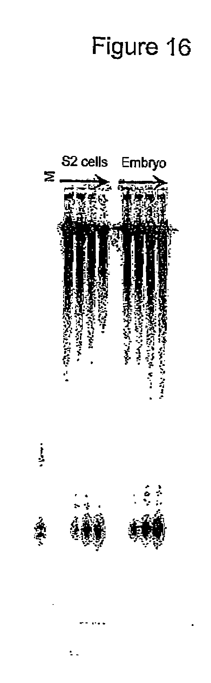

Figure 16: S2 cell and embryo extracts were assayed for 22mer generating activity.

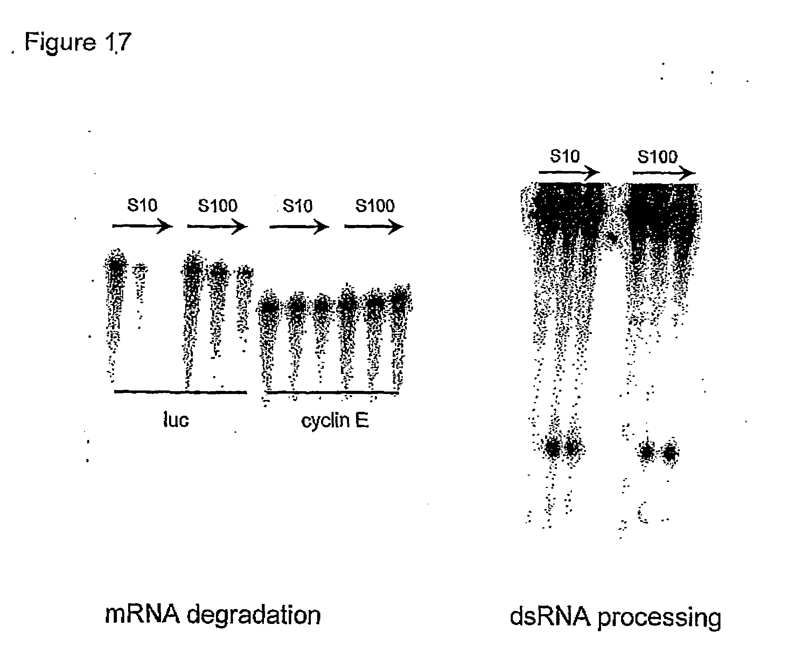

Figure 17: RISC can be separated from 22mer generating activity (dicer). Spinning extracts (S100) can clear RISC activity from supernatant (left panel) however, S100 spins still contain dicer activity (right panel).

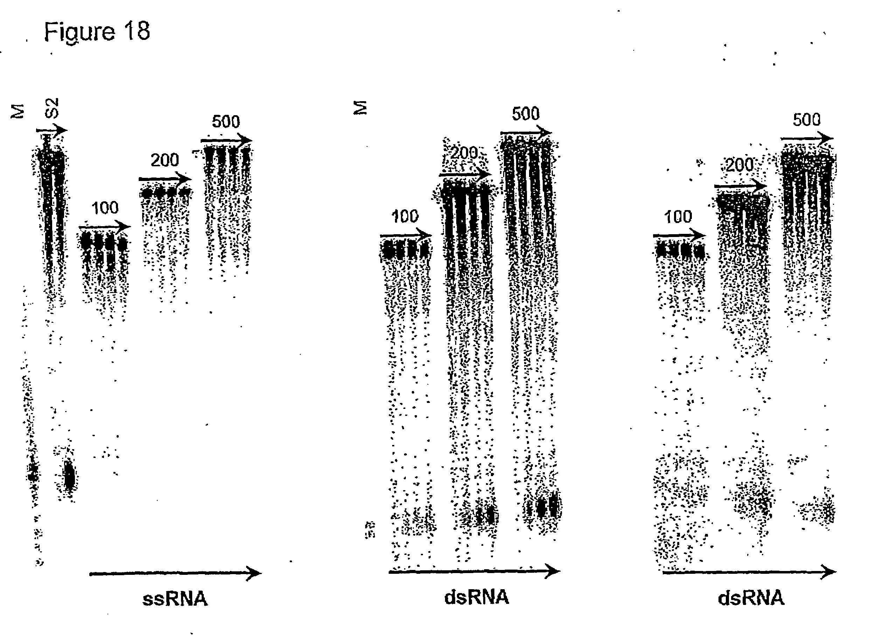

Figure 18: Dicer is specific for dsRNA and prefers longer substrates.

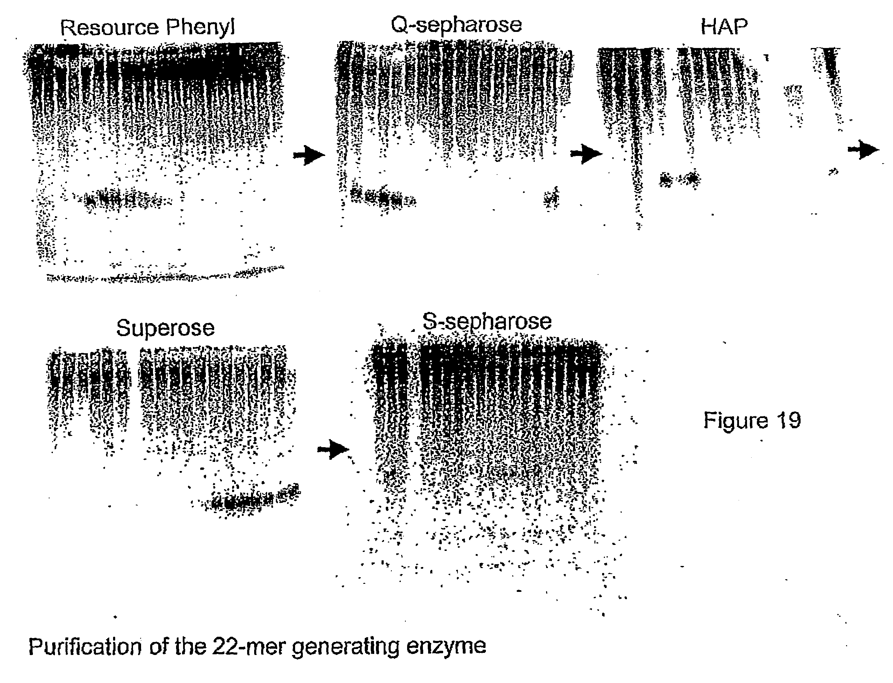

Figure 19: Dicer was fractionated over several columns.

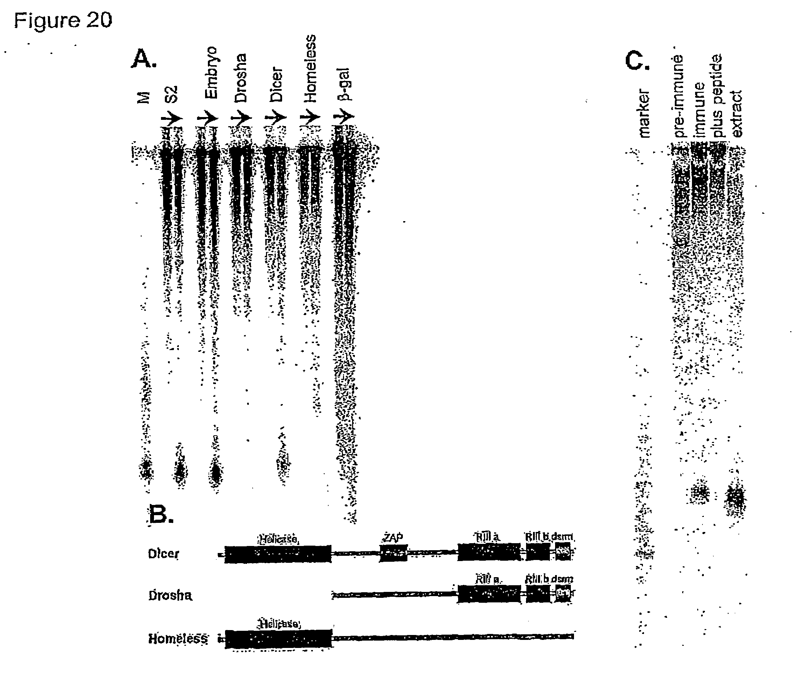

Figure 20: Identification of dicer as enzyme which can process dsRNA into 22mers. Various RNaseIII family members were expressed with n terminal tags, immunoprecipitated, and assayed for 22mer generating activity (left panel). In right panel, antibodies to dicer could also precipitate 22mer generating activity.

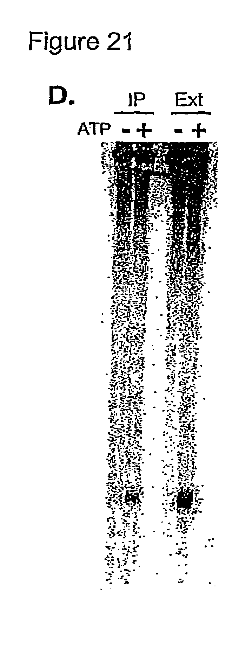

Figure 21: Dicer requires ATP.

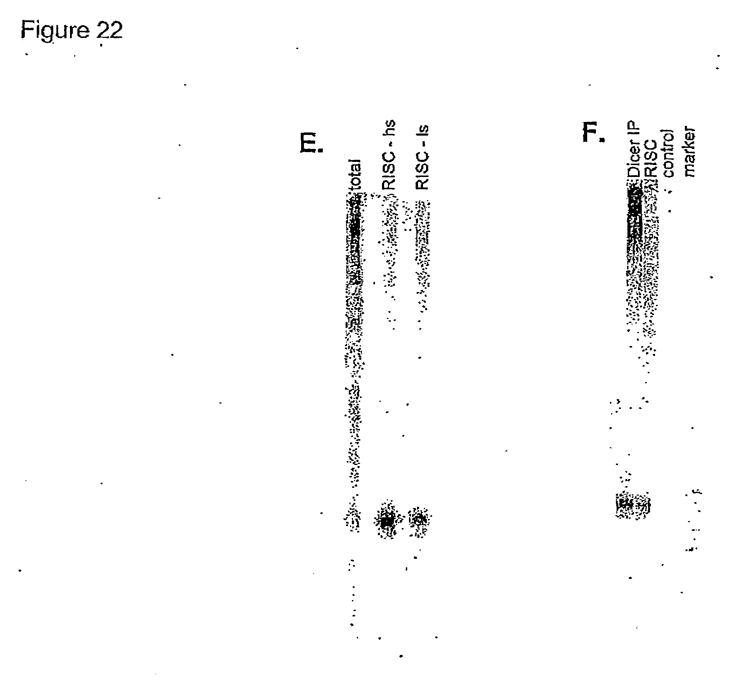

Figure 22: Dicer produces RNAs that are the same size as RNAs present in RISC.

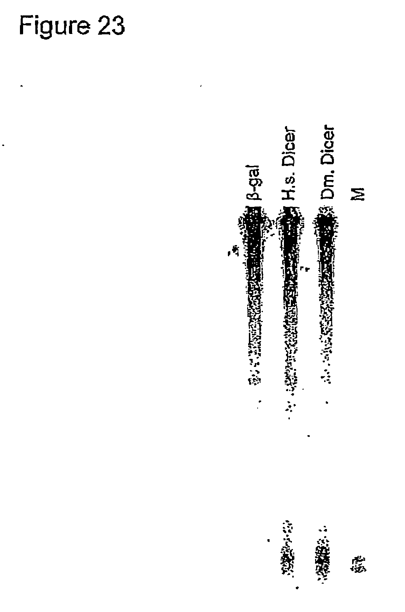

Figure 23: Human dicer homolog when expressed and immunoprecipitated has 22mer generating activity.
Figure 24: Sequence of dm argonaute 2. Peptides identified by microsequencing are shown in underline.

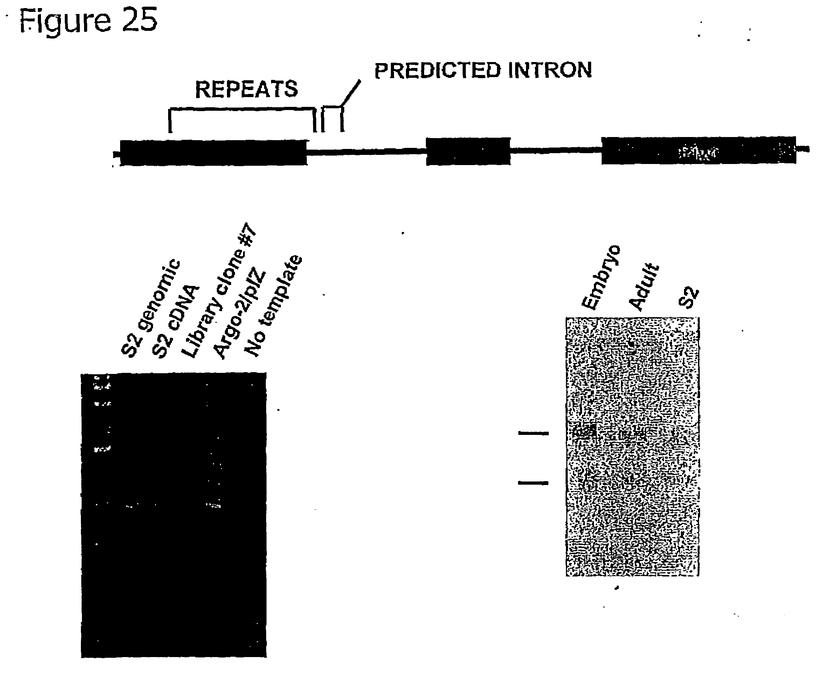

Figure 25: Molecular charaterization of dm argonaute 2. The presence of an intron in coding sequence was determined by northern blotting using intron probe. This results in a different 5' reading frame that that published genome seqeunce. Number of polyglutaine repeats was determined by genomic PCR.

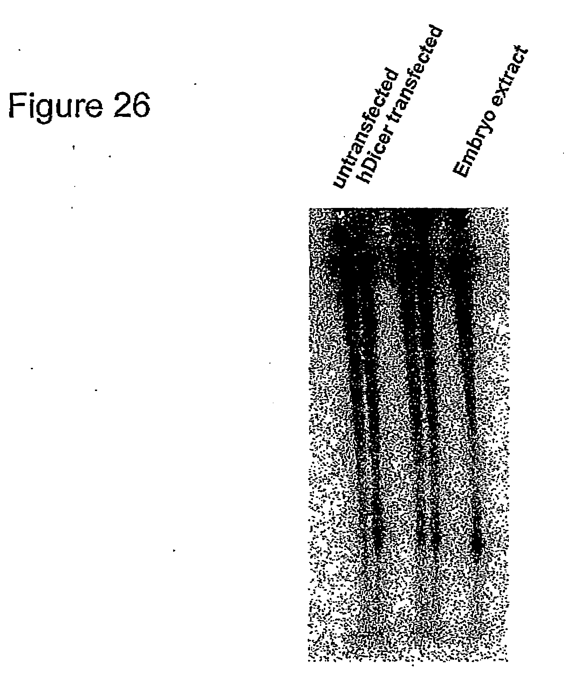

Figure 26: Dicer activity can be created in human cells by expression of human dicer gene. Host cell was 293, Crude extracts had dicer activity, while activity was absent from untransfected cells. Activity is not dissimilar to that seen in drosophila embryo extracts..

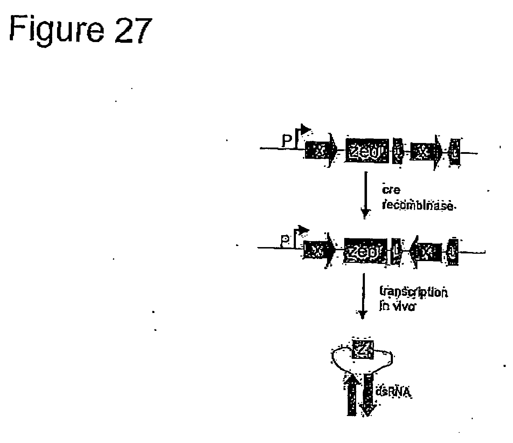

Figure 27: An ~500 nt. fragment of the gene that is to be silenced (X) is inserted into the modified vector as a stable direct repeat using standard cloning procedures. Treatment with commercially available ere recombinase reverses sequences within the loxP sites (L) to create an inverted repeat. This can be stably maintained and amplified in an sbc mutant bacterial strain (DL759). Transcription in vivo from the promoter of choice (P) yields a hairpin RNA that causes silencing. A zeocin resistance marker is included to insure maintenance of the direct and inverted repeat structures; however this is non-essential in vivo and could be removed by pre-mRNA splicing if desired. Smith, N. A. et al. Total silencing by intron-spliced hairpin RNAs. Nature 407, 319-20 (2000).

### Detailed Description of the Certain Preferred Embodiments

### I. Overview

The present invention provides methods for attenuating gene expression in a cell using gene-targeted double stranded RNA (dsRNA). The dsRNA contains a nucleotide sequence that hybridizes under. physiologic conditions of the cell to the nucleotide sequence of at least a portion of the gene to be inhibited (the "target" gene).

A significant aspect to certain embodiments of the present invention relates to the demonstration in the present application that RNAi can in fact be accomplished in cultured cells, rather than whole organisms as decribed in the art.

Another salient feature of the present invention concerns the ability to carry out RNAi in higher eukaryotes, particularly in non-oocytic cells of mammals, e.g., cells from adult mammals as an example.

As described in further detail below, the present invention(s) are based on the discovery that the RNAi phenomenum is mediated by a set of enzyme activities, including an essential RNA component, that are evolutionarily conserved in eukaryotes ranging from plants to mammals.

One enzyme contains an essential RNA component. After partial purification, a multi-component nuclease (herein "RISC nuclease") eo-fractionates with a discrete, 22-nucleotide RNA species which _nay confer specificity to the nuclease through homology to the substrate mRNAs. The short RNA molecules are generated by a processing reaction from the longer input dsRNA. Without wishing to be bound by any particular theory, these 22mer guide RNAs may serve as guide sequences that instruct the RISC nuclease to destroy specific mRNAs corresponding to the dsRNA sequences.

The appended examples also identify an enzyme, Dicer, that can produce the putative guide RNAs. Dicer is a member of the RNAse III family of nucleases that specifically cleave dsRNA and is evolutionarily conserved in worms, flies, plants, fungi and, as described herein, mammals. The enzyme has a distinctive structure which includes a helicase domain and dual RNAse III motifs. Dicer also contains a region of homology to the RDE1/QDE2/ARGONAUTE family, which have been genetically linked to RNAi in lower eukaryotes. Indeed, activation of, or overexpression of Dicer may be sufficient in many cases to permit RNA interference in otherwise non-receptive cells, such as cultured eukaryotic cells, or mammalian (non-oocytic) cells in culture or in whole organisms.

In certain embodiments, the cells can be treated with an agent(s) that inhibits the double-stranded RNA-dependent protein known as PKR (protein kinase RNA-activated), Double stranded RNAs in maminalian cells typically activate protein kinase PKR that phosphorylates and inactivates cIF2a (Fire (1999) Trends Genet 15;358). The ensuing inhibition of protein synthesis ultimately results in apoptosis. This sequence-independent response may reflect a form of primitive immune response, since the presence of dsRNA is a common feature of many viral lifecycles. However, as described herein, Applicants have demonstrated that the PKR. response can be overcome in favor of the sequence-specific RNAi response. However, in certain instances, it can be desirable to treat the cells with agents which inhibit expression of PKR, cause its destruction, and/or inhibit the kinase activity of PKF are specifically contemplated for use in the present method. Likewise, overexpression of or agents which ectopic activate IF2a can be used.

Thus, the present invention provides a process and compositions for inhibiting expression of a target gene in a cell, expecially a mammalian cell. In certain embodiments, the process comprises introduction of RNA (the "dsRNA construct") with partial or fully double-stranded character into the cell or into the extracellular environment. Inhibition is specific in that a nucleotide sequence from a portion of the target gene is chosen to produce the dsRNA construct. In preferred embodiments, the method utilizes a cell in which Dicer and/or Argonaute activities are recombinantly expressed or otherwise ectopically activated. This process can be (1) effective in attenuating gene expression, (2) specific to the targeted gene, and (3) general in allowing inhibition of many different types of target gene.

### II. Definitions

For convenience, certain terms employed in the specification, examples, and appended claims are collected here.

As used herein, the term "vector" refers to a nucleic acid molecule capable of transporting another nucleic acid to that it has been linked. One type of vector is a genomic integrated vector, or "integrated vector", which can become integrated into the chromsomal DNA of the host cell. Another type of vector is an episomal vector, i.e., a nucleic acid capable of extra-chromosomal replication. Vectors capable of directing the expression of genes to that they are operatively linked are referred to herein as "expression vectors". In the present specification, "plasmid" and "vector" are used interchangeably unless otherwise clear from the context.

As used herein, the term "nucleic acid" refers to polynucleotides such as deoxyribonucleic acid (DNA), and, where appropriate, ribonucleic acid (RNA). The term should also be understood to include, as applicable to the embodiment being described, single-stranded (such as sense or antisense) and double-stranded polynucleotides.

As used herein, the term "gene" or "recombinant gene" refers to a nucleic acid comprising an open reading frame encoding a polypeptide of the present invention, including both exon and (optionally) intron sequences. A "recombinant gene" refers to nucleic acid encoding such regulatory polypeptides, that may optionally include intron sequences that are derived from chromosomal DNA. The term "intron" refers to a DNA sequence present in a given gene that is not translated into protein and is generally found between exons. As used herein, the term "transfection" means the introduction of a nucleic acid, e.g., an expression vector, into a recipient cell by nucleic acid-mediated gene transfer.

A "protein coding sequence" or a sequence that "encodes" a particular polypeptide or peptide, is a nucleic acid sequence that is transcribed (in the case of DNA) and is translated (in the case of mRNA) into a polypeptide in vitro or in vivo when placed under the control of appropriate regulatory sequences. The boundaries of the coding sequence are determined by a start codon at the 5' (amino) terminus and a translation stop codon at the 3' (carboxy) terminus. A coding sequence can include, but is not limited to, cDNA from procaryotic or eukaryotic mRNA, genomic DNA sequences from procaryotic or eukaryotic DNA, and even synthetic DNA sequences. A transcription termination sequence will usually be located 3' to the coding sequence.

Likewise, "encodes", unless evident from its context, will be meant to include DNA sequences that encode a polypeptide, as the term is typically used, as well as DNA sequences that are transcribed into inhibitory antisense molecules.

The term "loss-of-function", as it refers to genes inhibited by the subject RNAi method, refers a diminishment in the level of expression of a gene when compared to the level in the absense of dsRNA constructs.

The term "expression" with respect to a gene sequence refers to transcription of the gene and, as appropriate, translation of the resulting mRNA transcript to a protein. Thus, as will be clear from the context, expression of a protein coding sequence results from transcription and translation of the coding sequence.

"Cells," "host cells" or "recombinant host cells" are terms used interchangeably herein. It is understood that such terms refer not only to the particular subject cell but to the progeny or potential progeny of such a cell. Because certain modifications may occur in succeeding generations due to either mutation or environmental influences, such progeny may not, in fact, be identical to the parent cell, but are still included within the scope of the term as used herein.

By "recombinant virus" is meant a virus that has been genetically altered, e.g., by the addition or insertion of a heterologous nucleic acid construct into the particle.

As used herein, the terms "transduction" and "transfection" are art recognized and mean the introduction of a nucleic acid, e.g., an expression vector, into a recipient cell by nucleic acid-mediated gene transfer. "Transformation", as used herein, refers to a process in which a cell's genotype is changed as a result of the cellular uptake of exogenous DNA or RNA, and, for example, the transformed cell expresses a dsRNA contruct.

"Transient transfection" refers to cases where exogenous DNA does not integrate into the genome ofatransfected cell, e.g., where episomal DNA is transcribed into mRNA and translated into protein.

A cell has been "stably transfected" with a nucleic acid construct when the nucleic acid construct is capable of being inherited by daughter cells.

As used herein, a "reporter gene construct" is a nucleic acid that includes a "reporter gene" operatively linked to at least one transcriptional regulatory sequence. Transcription of the reporter gene is controlled by these sequences to which they are linked. The activity of at least one or more of these control sequences can be directly or indirectly regulated by the target receptor protein. Exemplary transcriptional control sequences are promoter sequences. A reporter gene is meant to include a promoter-reporter gene construct that is heterologously expressed in a cell.

As used herein, "transformed cells" refers to cells that have spontaneously converted to a state of unrestrained growth, i.e., they have acquired the ability to grow through an indefinite number of divisions in culture. Transformed cells may be characterized by such terms as neoplastic, anaplastic and/or hyperplastic, with respect to their loss of growth control. For purposes of this invention, the terms "transformed phenotype of malignant mammalian cells" and "transformed phenotype " arc intended to encompass, but not be limited to, any of the following phenotypic traits associated with cellular transformation of mammalian cells: immortalization, morphological or growth transformation, and tumorigenicity, as detected by prolonged growth in cell culture, growth in semi-solid media, or tumorigenic growth in immune-incompetent or syngeneic animals.

As used herein, "proliferating" and "proliferation" refer to cells undergoing mitosis.

As used herein, "immortalized cells" refers to cells that have been altered via chemical, genetic, and/or recombinant means such that the cells have the ability to grow through an indefinite number of divisions in culture.

The "growth state" of a cell refers to the rate of proliferation of the cell and the state of differentiation of the cell.

### III. Exemplary embodiments of Isolation Method

One aspect of the invention provides a method for potentiating RNAi by induction or ectopic activation of an RNAi enzyme in a cell (in vivo or in vitro) or cell-free mixtures. In preferred embodiments, the RNAi activity is activated or added to a mammalian cell, e.g., a human cell; which cell may be provided in vitro or as part of a whole organism. In other embodiments, the subject method is carried out using eukaryotic cells generally (except for oocytes) in culture. For instance, the Dicer enzyme may be activated by virtue of being recombinantly expressed or it may be activated by use of an agent which (i) induces expression of the endogenous gene, (ii) stabilizes the protein from degradation, and/or (iii) allosterically modies the enzyme to increase its activity (by altering its Kcat, Km or both).

### A. Dicer and Argonaut Activities

In certain embodiment, at least one of the activated RNAi enzymes is Dicer, or a homolog thereof. In certain preferred embodiments, the present method provides for ectopic activation of Dicer. As used herein, the term "Dicer" refers to a protein which (a) mediates an RNAi response and (b) has an amino acid sequence at least 50 percent identical, and more preferablty at least 75, 85, 90 or 95 percent identical to SEQ ID No. 2 or 4, and/or which can be encoded by a nucleic acid which hybridizes under wash conditions of 2 x SSC at 22°C, and more preferably 0.2 x SSC at 65°C, to a nucleotide represented by SEQ ID No. 1 or 3. Accordingly, the method may comprise introducing a dsRNA contruct into a cell in which Dicer has been recombinantly expressed or otherwise ectopically activated.

In certain embodiment, at least one of the activated RNAi enzymes is Argonaut, or a homolog thereof. In certain preferred embodiments, the present method provides for ectopic activation of Argonaut. As used herein, the term "Argonaut" refers to a protein which (a) mediates an RNAi response and (b) has an amino acid sequence at least 50. percent identical, and more preferablty at least 75, 85, 90 or 95 percent identical to the amino acid sequence shown in Figure 24. Accordingly, the method may comprise. introducing a dsRNA contruct into a cell in which Argonaut has been recombinantly expressed or otherwise ectopically activated.

This invention also provides expression vectors containing a nucleic acid encoding a Dicer or Argonaut polypeptides, operably linked to at least one transcriptional regulatory sequence. Operably linked is intended to mean that the nucleotide sequence is linked to a regulatory sequence in a manner which allows expression of the nucleotide sequence. Regulatory sequences are art-recognized and are selected to direct expression of the subject Dicer or Argonaut proteins. Accordingly, the term transcriptional regulatory sequence includes promoters, enhancers and other expression Control elements. Such regulatory sequences are described in Gocddel; Gene Expression Technology: Methods in Enzymology 185, Academic Press, San Diego, CA (1990). For instance, any of a wide variety of expression control sequences, sequences that control the expression of a DNA sequence when operatively linked to it, may be used in these vectors to express DNA sequences encoding Dicer or Argonaut polypeptides of this invention. Such useful expression control sequences, include, for example, a viral LTR, such as the LTR of the Moloney murine leukemia virus, the early and late promoters of SV40, adenovirus or cytomegalovirus immediate early promoter, the lac system, the trp system, the TAC or TRC system, T7 promoter whose expression is directed by T7 RNA polymerase, the znajor operator and promoter regions of phage λ, the control regions for fd coat protein, the promoter for 3-phosphoglycerate kinase or other glycolytic enzymes, the promoters of acid phosphatase, e.g., Pho5, the promoters of the yeast α-mating factors, the polyhedron promoter of the baculovirus system and other sequences known to control the expression of genes of prokaryotic or eukaryotic cells or their viruses, and various combinations thereof. It should be understood that the design of the expression vector may depend on such factors as the choice of the host cell to be transformed and/or the type of protein desired to be expressed.

Moreover, the vector's copy number, the ability to control that copy number and the expression of any other proteins encoded by the vector, such as antibiotic markers, should also be considered.

The recombinant Dicer or Argonaut genes can be produced by ligating nucleic acid encoding a Dicer or Argonaut polypeptide into a vector suitable for expression in either prokaryotic cells, eukaryotic cells, or both. Expression vectors for production of recombinant forms of the subject Dicer or Argonaut polypeptides include plasmids and other vectors. For instance, suitable vectors for the expression of a Dicer or Argonaut polypeptide include plasmids of the types: pBR322-derived plasmids, pEMBL-derived plasmids, pEX-derived plasmids, pBTac-derived plasmids and pUC-derived plasmids for expression in prokaryotic cells, such as E. coli.

A number of vectors exist for the expression of recombinant proteins in yeast, For instance, YEP24, YIP5, YEP51, YEP52, pYES2, and YRP17 are cloning and expression vehicles useful in the introduction of genetic constructs into S. cerevisiae (see, for example, Broach et al. (1983) in Experimeatal Manipulation of Gene Expression, ed. M. Inouye Academic Press, p. 83, incorporated by reference herein). These vectors can replicate in E. coli due the presence of the pBR322 ori, and in S. cerevisiae due to the replication determinant of the yeast 2 micron plasmid. In addition, drug resistance markers such as ampicillin can be used. In an illustrative embodiment, a Dicer or Argonaut polypeptide is produced recombinantly utilizing an expression vector generated by sub-cloning the coding sequence of a Dicer or Argonaut gene.

The preferred mammalian expression vectors contain both prokaryotic sequences, to facilitate the propagation of the vector in bacteria, and one or more eukaryotic transcription units that are expressed in eukaryotic cells. The pcDNAI/amp, pcDNAI/neo, pRc/CMV, pSV2gpt, pSV2neo, pSV2-dhfr, pTk2, pRSVneo, pMSG, pSVT7, pko-neo and pHyg derived vectors are examples of mammalian expression vectors suitable for transfection of eukaryotic cells. Some of these vectors are modified with sequences from bacterial plasmids, such as pBR322, to facilitate replication and drug resistance selection in both prokaryotic and eukaryotic cells. Alternatively, derivatives of viruses such as the bovine papillomavirus (BPV-1), or Epstein-Barr virus (pHEBo, pREP-derived and p205) can be used for transient expression of proteins in eukaryotic cells. The various methods employed in the preparation of the plasmids and transformation of host organisms are well known in the art. For other suitable expression systems for both prokaryotic and eukaryotic cells, as well as general recombinant procedures, see Molecular Cloning A. Laboratory Manual, 2nd Ed., ed. by Sambrook, Fritsch and Maniatis (Cold Spring Harbor Laboratory Press: 1959) Chapters 16 and 17.

In yet another embodiment the subject invention provides a "gene activation" construct which, by homologous recombination with a genomic DNA, alters the transcriptional regulatory sequences of an endogenous Dicer or Argonaut gene. For instance, the gene activation construct can replace the endogenous promoter of a Dicer or Argonaut gene with a heterologous promoter, e.g., one which causes constitutive expression of the Dicer or Argonaut gene or which causes inducible expression of the gene under conditions different from the normal expression pattern of Dicer or Argonaut. A variety of different formats for the gene activation constructs are available. See, for example, the Transkaryotic Therapies, Inc PCT publications WO93/09222, WO95/31560, WO96/29411, WO95/31560 and WO94/12650.

In preferred embodiments, the nucleotide sequence used as the gene activation construct can be comprised of (1) DNA from some portion of the endogenous Dicer or Argonaut gene (exon sequence, intron sequence, promoter sequences, etc.) which direct recombination and (2) heterologous transcriptional regulatory sequence(s) which is to be operably linked to the coding sequence for the genomic Dicer or Argonaut gene upon recombination of the gene activation construct. For use in generating cultures of Dicer or Argonaut producing cells, the construct may further include a reporter gene to detect the presence of the knockout construct in the cell.

The gene activation construct is inserted into a cell, and integrates with the genomic DNA of the cell in such a position so as to provide the heterologous regulatory sequences in operative association with the native Dicer or Argonaut gene. Such insertion occurs by homologous recombination, i.e., recombination regions of the activation construct that are homologous to the endogenous Dicer or Argonaut gene sequence hybridize to the genomic DNA and recombine with the genomic sequences so that the construct is incorporated into the corresponding position of the genomic DNA.

The terms "recombination region" or "targeting sequence" refer to a segment (i.e., a portion) of a gene activation construct having a sequence that is substantially identical to or substantially complementary to a genomic gene sequence, e.g., including 5' flanking sequences of the genomic gene, and can facilitate homologous recombination between the genomic sequence and the targeting transgene construct.

As used herein, the term "replacement region" refers to a portion of a activation construct which becomes integrated into an endogenous chromosomal location following homologous recombination between a recombination region and a genomic sequence.

The heterologous regulatory sequences, e.g., which are provided in the replacement region, can include one or more of a variety elements, including: promoters (such as constitutive or inducible promoters), enhancers, negative regulatory elements, locus control regions, transcription factor binding sites, or combinations thereof.

Promoters/enhancers which may be used to control the expression of the targeted gene *in vivo* include, but are not limited to, the cytomegalovirus (CMV) promoter/enhancer (Karasuyama et al., 1989, J. Exp. Med., 169:13), the human β-actin promoter (Gunning et al. (1987) PNAS 84:4831-4835), the glucocorticoid-inducible promoter present in the mouse mammary tumor virus long terminal repeat (MMTV LTR) (Klessig et al. (1984) Mol, Cell Biol. 4:1354-1362), the long terminal repeat sequences of Moloney murine leukemia virus (MuLV LTR) (Weiss et al. (1985) RNA Tumor Viruses, Cold Spring Harbor Laboratory, Cold Spring Harbor, New York), the SV40 early or late region promoter (Bemoist et at. (1981) Nature 290:304-310; Templeton et al. (1984) Mol. cell Biol., 4:817; and Sprague et al. (1983) J. Virol., 45:773), the promoter contained in the 3' long terminal repeat of Rous sarcoma virus (RSV) (Yamanioto et al., 1980, Cell, 22:787-797), the herpes simplex virus (HSV) thymidine kinase promoter/enhancer (Wagner et al. (1981) PNAS 82:3567-71), and the herpes simplex virus LAT promoter (Wolfe et al. (1992) Nature Genetics, 1:379-384).

In still other embodiments, the replacement region merely deletes a negative transcriptional control element of the native gene, e.g., to activate expression, or ablates a positive control element, e.g., to inhibit expression of the targeted gene.

### B. Cell/Organism

The cell with the target gene may be derived from or contained in any organism (e.g., plant, animal, protozoan, virus, bacterium, or fungus). The dsRNA construct may be synthesized either in vivo or in vitro. Endogenous RNA polymerase of the cell may mediate transcription in vivo, or cloned RNA polymerase can be used for transcription in vivo or in vitro. For generating double stranded transcripts from a transgene in vivo, a regulatory region may be used to transcribe the RNA strand (or strands):

Furthermore, genetic manipulation becomes possible in organisms that are not classical genetic models. Breeding and screening programs may be accelerated by the ability to rapidly assay me consequences of a specific, targeted gene disruption. Gene disruptions may be used to discover the function of the target gene, to produce disease models in which the target gene are involved in causing or preventing a pathological condition, and to produce organisms with improved economic properties.

The cell with the target gene may be derived from or contained in any organism. The organism may a plant, animal, protozoan, bacterium, virus, or fungus. The plant may be a monocot, dicot or gymnosperm; the animal may be a vertebrate or invertebrate. Preferred microbes are those used in agriculture or by industry, and those that are pathogenic for plants or animals. Fungi include organisms in both the mold and yeast morphologies.

Plants include arabidopsis; field crops (e.g., alfalfa, barley, bean, com, cotton, flax, pea, rape, rice, rye, safflower, sorghum, soybean, sunflower, tobacco, and wheat); vegetable crops (e.g., asparagus, beet, broccoli, cabbage, carrot, cauliflower, celery, cucumber, eggplant, lettuce, onion, pepper, potato, pumpkin, radish, spinach, squash, taro, tomato, and zucchini); fruit and nut crops (e.g., almond, apple, apricot, banana, blackberry, blueberry, cacao, cherry, coconut, cranberry, date, faJoa, filbert, grape, grapefruit, guava, kiwi, lemon, lime, mango, melon, nectarine, orange, papaya, passion fruit, peach, peanut, pear, pineapple, pistachio, plum, raspberry, strawberry, tangerine, walnut, and watermelon); and ornamentals (e.g., alder, ash, aspen, azalea, birch, boxwood, camellia, camation, chrysanthemum, elm, fir, ivy, jasmine, juniper, oak, palm, poplar, pine, redwood, rhododendron, rose, and rubber).

Examples of vertebrate animals include fish, mammal, cattle, goat, pig, sheep, rodent, hamster, mouse, rat, primate, and human.

Invertebrate animals include nematodes, other worms, drosophila, and other insects. Representative generae of nematodes include those that infect animals (e.g., Ancylostoma, Ascaridia, Ascaris, Bunostomum, Caenorhabditis, Capillaria, Chabertia, Cooperia, Dictyocaulus, Haemonchus, Heterakis, Nematodirus, Oesophagostomum, Ostertagia, Oxyuris, Parascaris, Strongylus, Toxascaris, Trichuris, Trichostrongylus, Tflichonema, Toxocara, Uncinaria) and those that infect plants (e.g., B ursaphalenchus, Criconerriella, Diiylenchus, Ditylenchus, Globodera, Helicotylenchus, Heterodera, Longidorus, Melodoigyne, Nacobbus, Paratylenchus, Pratylenchus, Radopholus, Rotelynchus, Tylenchus, and Xiphinema). Representative orders of insects include Colcoptera, Diptera, Lepidoptera, and Homoptera.

The cell having the target gene may be from the germ *line* or somatic, totipotent or pluripotent, dividing or non-dividing, parenchyma or epithelium, immortalized or transformed, or the like. The cell may be a stem cell or a differentiated cell. Cell types that are differentiated include adipocytes, fibroblasts, myocytes, cardiomyocytes, endothelium, neurons, glia, blood cells, megakaryocytes, lymphocytes, macrophages, neutrophils, eosinophils, basophils, mast cells, leukocytes, granulocytes, keratinocytes, chondrocytes, osteoblasts, osteoclasts, hepatocytes, and cells of the endocrine or exocrine glands.

### C. Targeted Genes

The target gene may be a gene derived from the cell, an endogenous gene, a transgene, or a gene of a pathogen which is present in the cell after infection thereof Depending on the particular target gene and the dose of double stranded RNA material delivered, the procedure may provide partial or complete loss of function for the target gene. Lower doses of injected material and longer times after administration of dsRNA may result in Inhibition in a smaller faction of cells. Quantitation of gene expression in a cell may show similar amounts of inhibition at the level of accumulation of target mRNA or translation oftarget protein.

"Inhibition of gene expression" refers to the absence (or observable decrease) in the level of protein and/or mRNA product from a target gene. "Specificity" refers to the ability to inhibit the target gene without manifest effects on other genes of the cell. The consequences of inhibition can be confirmed by examination of the outward properties of the cell or organism (as presented below in the examples) or by biochemical techniques such as RNA solution hybridization, nuclease protection, Northern hybridization, reverse transcription, gene expression monitoring with a microarray, antibody binding, enzyme linked immunosorbent assay (ELISA), Western blotting, radiolmmunoassay (RIA), other immunoassays, and fluorescence activated cell analysis (FACS). For RNA-mediated inhibition in a cell line or whole organism, gene expression is conveniently assayed by use of a reporter or drug resistance gene whose protein product is easily assayed. Such reporter genes include acetohydroxyacid syathase (AHAS), alkaline phosphatase (AP), beta galactosidase (LacZ), beta glucoronidase (GUS), chloramphenicol acetyltransferase (CAT), green fluorescent protein (GFP), horseradish peroxidase (HRP), luciferase (Luc), nopaline synthase (NOS), octopine synthase (OCS), and derivatives thereof multiple selectable markers are available that confer resistance to ampicillin, bleomycin, chloramphenicol, gentamycin, hygromycin, kanamycin, lincomycin, methotrexate, phosphinothhcin, puromycin, and tatracyclin.

Depending on the assay, quantitation of the amount of gene expression allows one to determine a degree of inhibition which is greater than 10%, 33%, 50%, 90%, 95% or 99% as compared to a cell not treated according to the present invention. Lower doses of injected material and longer times after administration of dsRNA may result in inhibition in a smaller fraction of cells (e.g., at least 14%, 20%, 50%, 75%,90%, or 95% of targeted cells). Quantitation of gene expression in a cell may show similar amounts of inhibition at the level of accumulation of target mRNA or translation of target protein. As an example, the efficiency of inhibition may be determined by assessing the amount of gene product in *the cell:* mRNA may be detected with a hybridization probe having a nucleotide sequence outside the region used for the inhibitory double-stranded RNA, or translated polypeptide may be detected with an antibody raised against the polypeptide sequence of that region.

As disclosed herein, the present invention may is not limited to any type of target gene or nucleodde sequence, But the following classes of possible target genes are listed for illustrative purposes: developmental genes (e.g., adhesion molecules, cyclin kinase inhibitors, Writ family members, Pax family members, Winged helix family members, Hox family members, cytokines/lymphokines and their receptors, growth/differentiation factors and their receptors, neurotransmitters and their receptors); oncogenes (e.g., ABLI,. BCLI, BCL2, BCL6, CBFA2, CBL, CSFIR, ERBA, ERBB, EBRB2, ETSI, ETS1, ETV6, FGR, FOS, FYN, HCR, HRAS, JUN, KRAS, LCK, LYN, MDM2, MLL, MYB, MYC, MYCLI, MYCN, NRAS, PIM 1, PML, RET, SRC, TALI, TCL3, and YES); tumor suppressor genes (e.g., APC, BRCA 1, BRCA2, MADH4. MCC, NF 1, NF2, RB I, TP53, and WTT); and enzymes (e.g., ACC synthases and oxidases, ACP desaturases and hydroxylases, ADP-glucose pyrophorylases, ATPases, alcohol dehydrogenases, amylases, amyloglucosidases, catalases, cellulases, chalcone synthases, chitinases, cyclooxygenases, decarboxylases, dextrinases, DNA and RNA polymerases, galactosidases, glucanases, glucose oxidases, granule-bound starch synthases, GTPases, helicases, hemicellulases, integrases, inulinases, invertases, isomerases, kinases, lactases, lipases, lipoxygenases, lysozymes, nopaline synthases, octopine synthases, pectinesterases, peroxidases; phosphatases, phospholipases, phosphorylases, phytases, plant growth regulator synthases, polygalacturonases, proteinases and peptidases, pullanases, recombinases, reverse transcriptases, RUBISCOs, topoisomerases, and xylanases).

### D. dsRNA constructs

The dsRNA construct may comprise one or more strands of polymerized ribonucleotide. It may include modifications to either the phosphate-sugar backbone or the nucleoside. For example, the phosphodiester linkages of natural RNA may be modified to include at least one of a nitrogen or sulfur heteroatom. Modifications in RNA structure may be tailored to allow specific genetic inhibition while avoiding a general panic response in some organisms which is generated by dsRNA. Likewise, bases may be modified to block the activity of adenosine deaminase. The dsRNA construct may be produced enzymatically or by partial/total organic synthesis, any modified ribonucieotide can be introduced by in vitro enzymatic or organic synthesis.

The dsRNA construct may be directly introduced into the cell (i.e., intracellularly); or introduced' extracellularly into a cavity, interstitial space, into the circulation of an organism, introduced orally, or may be introduced by bathing an organism in a solution containing RNA. Methods for oral introduction include direct mixing of RNA with food of the organism, as well as engineered approaches in which a species that is used as food is engineered to express an RNA, then fed to the organism to be affected. Physical methods of introducing nucleic, acids include injection directly into the cell or extracellular injection into the organism of an RNA solution.

The double-stranded structure may be formed by a single self-complementary RNA strand or two complementary RNA strands. RNA duplex formation may be initiated either inside or outside the cell. The RNA may be introduced in an amount which allows delivery of at least one copy per cell. Higher doses (e.g., at least 5, 10, 100, 500 or 1000 copies per cell) of double-stranded material may yield more elective inhibition; lower doses may also be useful for specific applications. Inhibition is sequence-specific in that nucleotide sequences corresponding to the duplex region of the RNA are targeted for genetic inhibition.

dsRNA constructs containing a nucleotide sequences identical to a portion of the target gene are preferred for inhibition. RNA sequences with insertions, deletions, and single point mutations relative to the target sequence have also been found to be effective for inhibition. Thus, sequence identity may optimized by sequence comparison and alignment algorithms known in the art (see Gribskov and Devereux, Sequence Analysis Primer, Stockton Press, 199 1, and references cited therein) and calculating the percent difference between the nucleotide sequences by, for example, the Smith-Waterman algorithm as implemented in the BESTFIT software program using default parameters (e.g., University of Wisconsin Genetic Computing Group). Greater than 90% sequence identity, or even 100% sequence identity, between the inhibitory RNA and the portion of the target gene is preferred. Alternatively, the duplex region of the RNA may be defined functionally as a nucleotide sequence that is capable of hybridizing with a portion of the target gene transcript (e.g., 400 mM NaCl, 40 mM PIPES pH 6.4, 1 mM EDTA, 50'C or 70'C hybridization for 12-16 hours; followed by washing). The length of the identical nucleotide sequences may be, for example, at least 25, 50, 100, 200, 300 or 400 bases. In certain embodiments, the dsRNA construct is 400-800 bases in length.

100% sequence identity between the RNA and the target gene is not required to practice the present invention. Thus the invention has the advantage of being able to toierate sequence variations that might be expected due to genetic mutation, strain polymorphism, or evolutionary divergence.

The dsRNA construct may be synthesized either in vivo or in vitro. Endogenous RNA polyrnerase of the cell may mediate transcription in vivo, or cloned RNA polymerase can be used for transcription in vivo or in vitro. For transcription from a transgene in vivo or an expression construct, a regulatory region (e.g., promoter, enhancer, silencer, splice donor and acceptor, polyadenylation) may be used to transcribe the dsRNA strand (or strands). Inhibition may be targeted by specific transcription in an organ, tissue, or cell type; stimulation of an environmental condition (e.g., infection, stress, temperature, chemical, inducers); and/or engineering transcription at a developmental stage or age. The RNA, strands may or may not be polyadenylated; the RNA strands may or may not be capable of being translated into a polypeptide by a cell's translational apparatus. The dsRNA construct may be chemically or enzymatically synthesized by manual or automated reactions. The dsRNA construct may be synthesized by a cellular RNA-polymerase or a bacteriophage RNA polymerase (e.g., T3, T7, SP6). The use and production of an expression construct are known in the art32,33,34 (see also WO 97/32016; U.S. Pat. Nos. 5,593,874, 5,698,425, 5,712,135, 5,789,214, and 5,804,693; and the references cited therein). If synthesized chemically or by in vitro enzymatic synthesis, the RNA may be purified prior to introduction into the cell. For example, RNA can be punified from a mixture by extraction with a solvent or resin, precipitation, electrophoresis, chromatography or a combination thereof. Alternatively, the dsRNA construct may be used with no or a minimum of purification to avoid losses due to sample processing.. The dsRNA construct may be dried for storage or dissolved in an aqueous solution. The solution may contain buffers or salts to promote annealing, and/or stabilization of the duplex strands.

Physical niethods of introducing nucleic acids include injection of a solution containing the dsRNA construct, bombardment by particles covered by the dsRNA construct, soaking the cell or organism in a solution of the RNA, or electroporation of cell membranes in the presence of the dsRNA construct. A viral construct packaged into a viral particle would accomplish both efficient introduction of an expression construct into the cell and transcription of dsRNA construct encoded by the expression construct. Other methods known in the art for introducing nucleic acids to cells may be used, such as lipid-mediated carrier transport, chemicalmediated transport, such as calcium phosphate, and the like. Thus the dsRNA construct may be introduced along with components that perform one or more of the following activities: enhance RNA uptake by the cell, promote annealing of the duplex strands, stabilize the annealed strands, or other-wise increase inhibition of the target gene.

### E. Illustrative Uses

One utility of the present invention is as a method of identifying gene function in an organism, especially higher eukaryotes comprising the use of double-stranded RNA to inhibit the activity of a target gene of previously unknown function. Instead of the time consuming and laborious isolation of mutants by traditional genetic screening, functional genomics would envision determining the function of uncharacterized genes by employing the invention to reduce the amount and/or alter the timing of target gene activity. The invention could be used in determining potential targets for pharmaceutics, understanding normal and pathological events associated with development, determining signaling pathways responsible for postnatal development/aging, and the like. The increasing speed of acquiring nucleotide sequence information from genomic and expressed gene sources, including total sequences for mammalian genomes, can be coupled with the invention to determine gene function in a cell or in a whole organism. The preference of different organisms to use particular codons, searching sequence databases for related gene products, correlating the linkage map of genetic traits with the physical map from which the nucleotide sequences are derived, and artificial intelligence methods may be used to define putative open reading frames from the nucleotide sequences acquired in such sequencing projects.

A simple assay would be to inhibit gene expression according to the partial sequence available from an expressed sequence tag (EST). Functional alterations in growth, development, metabolism, disease resistance, or other biological processes would be indicative of the normal role of the ESTs gene product.

The ease with which the dsRNA construct can be introduced into an intact cell/organism containing the target gene allows the present invention to be used in high throughput screening (HTS). For example, duplex RNA can be produced by an amplification reaction using primers flanking the inserts of any gene library derived from the target calUorganism. Inserts may be derived from genomic DNA or mRNA (e.g., cDNA and cRNA). Individual clones from the library can be replicated and then isolated in separate reactions, but preferably the library is maintained in individual reaction vessels (e.g., a 96 well microtiter plate) to minimize the number of steps required to practice the invention and to allow automation of the process. Solutions containing duplex RNAs that are capable of inhibiting the different expressed genes can be placed into individual wells positioned on a microtiter plate as an ordered array, and intact cells/organisms in each well can be assayed for any changes or modifications in behavior or development due to inhibition of target gene activity. The amplified RNA can be fed directly to, injected into, the cell/organism containing the target gene. Alternatively, the duplex RNA can be produced by in vivo or in vitro transcription from an expression construct used to produce the library. The construct can be replicated as individual clones of the library and transcribed to produce the RNA; each clone can then be fed to, or injected into, the cell/organism containing the target gene. The function of the target gene can be assayed from the effects it has on the cell/organism when gene activity is inhibited. This screening could be amenable to small subjects that can be processed in large number, for example, tissue culture cells derived from mammals, especially primates, and most preferably humans.

If a characteristic of an organism is determined to be genetically linked to a polymorphism through RFLP or QTL analysis, the present invention can be used to gain insight regarding whether that genetic polymorphism might be directly responsible for the characteristic. For example, a fragment defining the genetic polymorphism or sequences in the vicinity of such a genetic polymorphism can be amplified to produce an RNA, the duplex RNA can be introduced to the organism or cell, and whether an alteration in the charactenstic is correlated with inhibition can be determined. Of course, there may be trivial explanations for negative results with this type of assay, for example: inhibition of the target gene causes lethality, inhibition of the target gene may not result in any observable alteration, the fragment contains nucleotide sequences that are not capable of inhibiting the target gene, or the target gene's activity is redundant.

The present invention may be useful in allowing the inhibition of essential genes. Such genes may be required for cell or organism viability at only particular stages of development or cellular compartments. The functional equivalent of conditional mutations may be produced by inhibiting activity of the target gene when or where it is not required for viability. The invention allows addition of RNA at specific times of development and locations in the organism without introducing permanent mutations into the target genome.

If alternative splicing produced a family of transcripts that were distinguished by usage of characteristic exons, the present invention can target inhibition through the appropriate exons to specifically inhibit or to distinguish among the functions of family members. For example, a hormone that contained an alternatively spliced transmembrane domain may be expressed in both membrane bound and secreted forms. Instead of isolating a nonsense mutation that terminates translation before the transmembrane domain, the functional consequences of having only secreted hormone can be determined according to the invention by targeting the exon containing the transmembrane domain and thereby inhibiting exp-ession of membrane-bound hormone.

The present invention may be used alone or as a component of a kit having at least one of the reagents necessary to carry out the in vitro or in vivo introduction of RNA to test samples or subjects. Preferred components are the dsRNA and a vehicle that promotes introduction of the dsRNA. Such a kit may also include instructions to allow a user of the kit to practice the invention.

Alternatively, an organism may be engineered to produce dsRNA which produces commercially or medically beneficial results, for example, resistance to a pathogen or its pathogenic effects, improved growth, or novel developmental patterns.

### IV. Exemplification

The invention, now being generally described, will be more readily understood by reference to the following examples, which are included merely for purposes of illustration of certain aspects and embodiments of the present invention and are not intended to limit the invention.

### Example 1: An RNA-directed nuclease mediates RNAi gene silencing

In a diverse group of organisms that includes *Caenorhabditis elegans, Drosophila,* planaria, hydra, trypanosomes, fungi and plants, the introduction of double-stranded RNAs inhibits gene expression in a sequence-specific manner¹⁻⁷. These responses, called RNA interference or post-transcriptional gene silencing, may provide anti-viral defence, modulate transposition or regulate gene expression^{1. 6 8-10}. We have taken a biochemical approach towards elucidating the mechanisms underlying this genetic phenomenon. Here we show that 'loss-of-function' phenotypes can be created in cultured *Drosophila* cells by transfection with specific double-stranded RNAs. This coincides with a marked reduction in the level of cognate cellular messenger RNAs. Extracts of transfected cells contain a nuclease activity that specifically degrades exogenous transcripts homologous to transfected double-stranded RNA. This enzyme contains an essential RNA component, After partial purification, the sequence-specific nuclease co-fractionates with a discrete, ~25-nuelcotide RNA species which may confer specificity to the enzyme through homology to the substrate mRNAs.

Although double-stranded RNAs (dsRNAs) can provoke gene silencing in numerous biological contexts including D*rosophila*^{11, 12}, the mechanisms underlying this phenomenon have remained mostly unknown. We therefore wanted to establish a biochemically tractable model in which such mechanisms could be investigated.

Transient transfection of cultured, *Drosophila* S2 cells with a *lacZ* expression vector resulted in β-galactosidase activity that was easily detectable by an *in situ* assay (Fig. 1a). This activity was greatly reduced by co-transfectioa with a dsRNA corresponding to the first 300 nucleotides of the *lacZ* sequence, whereas co-transfection with a control dsRNA (*CD8*) (Fig. 1a) or with single-stranded RNAs of either sense or antisense orientation (data not shown) had little or no effect. This indicated that dsRNAs could interfere, in a sequence-specifie fashion, with gene expression in cultured cells.

To determine whether RNA interference (RNAi) could be used to target endogenous genes, we transfected S2 cells with a dsRNA corresponding to the first 540 nucleotides of *Drosophila cyclin E,* a gene that is essential for progression into S phase of the cell cycle. During log-phase growth, untreated S2 cells reside primarily in G2/M (Fig. 1b). Transfection with *lacZ* dsRNA had no effect on cell-cycle distribution, but transfection with the *cyclin E* dsRNA caused a G1-phase cell-cycle arrest (Fig. 1b). The ability of *cyclin E* dsRNA to provoke this response was length-dependent. Double-stranded RNAs of 540 and 400 nucleotides were quite effective, whereas dsRNAs of 200 and 300 nucleotides were less potent. Double-stranded *cyclin E* RNAs of 50 or 100 nucleotides were inert in our assay, and transfection with a single-stranded, antisense *cyclin E* RNA had virtually no effect.

One hallmark of RNAi is a reduction in the level of mRNAs that are homologous to the dsRNA. Cells transfected with the *cyclin E* dsRNA (bulk population) showed diminished endogenous *cyclin E* mRNA as compared with control cells (Fig. 1c). Similarly, transfection of cells with dsRNAs homologous to *fizzy,* a component of the anaphase-promoting complex (APC) or *cyclin A,* a cyclin that acts in S, G2 and M, also caused reduction of their cognate mRNAs (Fig. 1c). The modest reduction in *fizzy* mRNA levels in cells transfected with *cyclin A* dsRNA probably resulted from arrest at a point in the division cycle at which *fizzy* transcription is low^{14, 15}. These results indicate that RNAi may be a generally applicable method for probing gene function in cultured *Drosophila* cells.

The decrease in mRNA levels observed upon transfection of specific dsRNAs into *Drosophila* cells could be explained by effects at transcriptional or post-transcriptional levels. Data from other systems have indicated that some elements of the dsRNA, response may affect mRNA directly (reviewed in refs 1 and 6). We therefore sought to develop a cell-free assay that reflected, at least in part, RNAi.

S2 cells were transfected with dsRNAs corresponding to either *cyclin E* or *lacZ.* Cellular extracts were incubated with synthetic mRNAs of *lacZ* or *cyclin E.* Extracts prepared from cells transfected with the 340-nucleotide *cyclin E* dsRNA efficiently degraded the *cyclin E* transcript; however, the *locZ* transcript was stable in there lysates (Fig. 2a). Conversely, lysates from cells transfected with the *lacZ* dsRNA degraded the *lacZ* transcript but left the *cyclin E* mRNA intact. These results indicate that RNAi ablates target mRNAs through the generation of a sequence-specific nuclease activity. We have termed this enzyme RISC (RNA-induced silencing complex). Although we occasionally observed possible intermediates in the degradation process (see Fig. 2), the absence of stable cleavage end-products indicates an exonuclease (perhaps coupled to an endonuclease). However, it is possible that the RNAi nuclease makes an initial endonucleolytic cut and that non-specific exonucleases in the extract complete the degradation process¹⁶. In addition, our ability to create an extract that targets *lacZ in vitro* indicates that the presence of an endogenous gene is not required for the RNAi response.

To examine the substrate requirements for the dsRNA-induced, sequence-specific nuclease activity, we incubated a variety of *cyclin-E-*derived transcripts with an extract derived from cells that had been transfected with the 540-nucleotide *cyclin E* dsRNA (Fig. 2b, c). Just as a length requirement was observed for the transfected dsRNA, the RNAi nuclease activity showed a dependence on the size of the RNA substrate. Both a 600-nucleotide transcript that extends slightly beyond the targeted region (Fig. 2b) and an ―1-kilobase (kb) transcript that contains the entire coding sequence (data not shown) were completely destroyed by the extract. Surprisingly, shorter substrates were not degraded as efficiently. Reduced activity was observed against either a 300- or a 220-nucleotide transcript, and a 100-nucleotide transcript was resistant to nuclease in our assay. This was not due solely to position effects because -100-nucleotide transcripts derived from other portions of the transfected dsRNA behaved similarly (data not shown). As expected, the nuclease activity (or activities) present in the extract could also recognize the antisense strand of the *cyclin E* mRNA. Again, substrates that contained a substantial portion of the targeted region were degraded efficiently whereas those that contained a shorter stretch of homologous sequence (~130 nucleotides) were recognized inefficiently (Fig. 2c, as600). For both the sense and antisense strands, transcripts that had no homology with the transfected dsRNA (Fig. 2b, Eout; Fig. 2c, as300) were not degraded. Although we cannot exclude the possibility that nuclease specificity could have migrated beyond the targeted region, the resistance of transcripts that do not contain homology to the dsRNA is consistent with data from *C. elegans.* Double-stranded RNAs homologous to an upstream cistron have little or no effect on a linked downstream cistron, despite the fact that unprocessed, polycistronic mRNAs can be readily detected^{17, 18}. Furthermore, the nuclease was inactive against a dsRNA identical to that used to provoke the RNAi response *in vivo* (Fig. 2b). In the *in vitro* system, neither a 5' cap nor a poly(A) tail was required, as such transcripts were degraded as efficiently as uncapped and non-polyadenylated RNAs.

Gene silencing provoked by dsRNA is sequence specific. A plausible mechanism for determining specificity would be incorporation of nucleic-acid guide sequences into the complexes that accomplish silencing¹⁹. In accord with this idea, pre-treatment of extracts with a Ca²⁺-dependent nuclease (micrococcal nuclease) abolished the ability of these extracts to degrade cognate mRNAs (Fig. 3). Activity could not be rescued by addition of non-specific RNAs such as yeast transfer RNA. Although micrococcal nuclease can degrade both DNA and RNA, treatment of the extract with DNAse I had no effect (Fig.3). Sequence-specific nuclease activity, however, did require protein (data not shown). Together, our results support the possibility that the RNAi nuclease is a ribonucleoprotein, requiring both RNA and protein components. Biochemical fractionation (see below) is consistent with these components being associated in extract rather than being assembled on the target mRNA after its addition.

In plants, the phenomenon of co-suppression has been associated with the existence of small (∼25-nucleotide) RNAs that correspond to the gene that is being. silenced¹⁹. To address the possibility that a similar RNA might exist in *Drosophila* and guide the sequence-specific nuclease in the choice of substrate, we partially purified our. activity through several fractionation steps. Crude extracts contained both sequence-specific nuclease activity and abundant, heterogeneous RNAs homologous to the transfected dsRNA (Figs 2 and 4a). The RNAi nuclease fractionated with ribosomes in a high-speed centrifugation step. Activity could be extracted by treatment with high salt, and ribosomes could be removed by an additional centrifugation step. Chromatography of soluble nuclease over an anion-exchange column resulted in a discrete peak of activity (Fig. 4b, *cyclin E*). This retained specificity as it was inactive against a heterologous mRNA (Fig. 4b, *lacZ*). Active fractions also contained an RNA species of 25 nucleotides that is homologous to the *cyclin E* target (Fig. 4b, northern). The band observed on northern blots may represent a family of discrete RNAs because it could be detected with *probes specific for both the sense and antisense cyclin E sequences and with probes derived from distinct segments of the* dsRNA (data not shown). At present, we cannot determine whether the 25-nucleotide RNA is present in the nuclease complex in a double-stranded or single-stranded form.

RNA interference allows an adaptive defence against both exogenous and endogenous dsRNAs, providing something akin to a dsRNA immune response. Our data, and that of others¹⁹, is consistent with a model in which dsRNAs present in a cell are converted, either through processing or replication, into small specificity determinants of discrete size in a manner analogous to antigen processing. Our results suggest that the post-transcriptional component of dsRNA-dependent gene silencing is accomplished by a sequence-specific nuclease that incorporates these small RNAs as guides that target specific messages based upon sequence recognition. The identical size of putative specificity determinants in plants¹⁹ and animals predicts a conservation of both the mechanisms and the components of dsRNA-induced, post-transcriptional gene silencing in diverse organisms. In plants, dsRNAs provoke not only post-transcriptional gene silencing but also chromatin remodelling and transcriptional repression^{20, 21}. It is now critical to determine whether conservation of gene-silencing mechanisms also exists at the transcriptional level and whether chromatin remodelling can be directed in a sequence-specific fashion by these same dsRNA-derived guide sequences.

### Methods

Cell culture and RNA methods S2 (ref. 22) cells were cultured at 27°C in 90% Schneider's insect media (Sigma), 10% heat inactivated fetal bovine serum (FBS), Cells were transfected with dsRNA and plasmid DNA by calcium phosphate co-precipitation²³. Identical results were observed when cells were transfected using lipid reagents (for example, Superfect, Qiagen). For FACS analysis, cells were additionally transfected with a vector that directs expression of a green fluorescent protein (GrFP) US9 fusion protein¹³. These cells were fixed in 90% ice-cold ethanol and stained with propidium iodide at 25 µg ml⁻¹. FACS was performed on an Elite flow cytometer (Coulter). For northern blotting, equal loading was ensured by over-probing blots with a control complementary DNA (RP49). For the production of dsRNA, transcription templates were generated by polymerase chain reaction such that they contained T7 promoter sequences on each end of the template. RNA was prepared using the RiboMax kit (Promega). Confirmation that RNAs were double stranded came from their complete sensitivity to RNAse III (a gift from A. Nicholson). Target mRNA transcripts were synthesized using the Riboprobe kit (Promega) and were gel purified before use.

Extract preparation Log-phase S2 cells were plated on 15-cm tissue culture dishes and transfected with 30 µg dsRNA and 30 µg carrier plasmid DNA. Seventy-two hours after transfection, cells were harvested in PBS containing 5 mM EGTA washed twice in PBS and once in hypotonic buffer (10 mM HEPES pH 7.3, 6 mM β-mercaptoethanol). Cells were suspended in 0.7 packed-cell volumes of hypotonic buffer containing *Complete* protease inhibitors (Boehringer) and 0.5 units ml⁻¹ of RNasin (Promega), Cells were disrupted in a dounce homogenizer with a type B pestle, and lysates were centrifuged at 30,000g for 20 min. Supernatants were used in an *in vitro* assay containing 20 mM HEPES pH 7.3, 110 mM KOAc, 1 mM Mg(OAc)₂, 3 mM EGTA, 2 mM CaCl₂, 1 mM DTT. Typically, 5 µl extract was used in a 10 µl assay that contained also 10,000 c.p.m. synthetic mRNA substrate.

Extract fractionation Extracts were centrifuged at 200,000g for 3h and the resulting pellet (containing ribosomes) was extracted in hypotonic buffer containing also 1 mM MgCl₂ and 300 mM KOAc. The extracted material was spun at 100,000g for 1 h and the resulting supernatant was fractionated on Source 15Q column (Pharmacia) using a KCl gradient in buffer A (20 mM HBPES pH 7.0, 1 mM dithiothreitol, 1 mM: MgCl₂). Fractions were assayed for nuclease activity as described above. For northern blotting, fractions were proteinase K/SDS treated, phenol extracted, and resolved on 15% acrylamide 8M urea gels. RNA was electroblotted onto Hybond N+ and probed with strand-specific riboprobes derived from cyclin E mRNA. Hybridization was carried out in 500 mM NaPO₄ pH 7.0, 15% formamide, 7% SDS, 1% BSA. Blots were washed in 1 SSC at 37-45 °C.

### References cited in Example I

1. Sharp, P. A. RNAi and double-strand RNA. Genes Dev. 13,139-141 (1999).
2. Sanchez-Alvarado, A. & Newmark, P. A. Double-stranded RNA specifically disrupts gene expression during planarian regeneration. Proc. Natl Acad. Sci. USA 96, 5049-5054 (1999).
3. Lohmann, J. U., Endl, I. & Bosch, T. C. Silencing of developmental genes in Hydra. Dev. Biol. 214, 211-214 (1999).
4. Cogoni, C. & Macino, G. Gene silencing in Neurospora crassa requires a protein homologous to RNA-dependent RNA polymerase. Nature 399,166-169 (1999).
5. Waterhouse, P. M., Graham, M. W. & Wang, M. B. Virus resistance and gene silencing in plants can be induced by simultaneous expression of sense and antisense RNA. Proc. Natl Acad. Sci. USA 95, 13959-13964 (1998).
6. Montgomery, M. K. & fire, A. Double-stranded RNA as a mediator in sequence-specific genetic silencing and co-suppression. Trends Genet. 14, 225-228 (1998).
7. Ngo, H., Tschudi, C., Gull, K. & Ullu, E. Double-stranded RNA induces mRNA degradation in Trypanosoma brucei. Proc. Natl Acad Sci. USA 95, 14687-14692 (1993).
8. Tabara, H. et al. The rde-1 gene, RNA interference, and transposon silencing in C. elegans. Cell 99, 123-132 (1999).
9. Ketting, R. P., Haverkamp, T. H. A., van Luenen, H. G. A. M. & Piasterk, R. H. A. mut-7 of C. elegans, required for transposon silencing and RNA interference, is a homolog of Werner Syndrome helicase and RnaseD. Cell 99, 133-141 (1999).
10. Ratcliff, F., Harrison, B. D. & Baulcombe, D. C. A similarity between viral defense and gene silencing in plants. Science 276, 1558-1560 (1997).
11. Kennerdell, J. R. & Carthew, R. W. Use of dsRNA-mediated genetic interference to demonstrate that frizzled and frizzled 2 act in the wingless pathway. Cell 95, 1017-1026 (1998).
12. Misquitta, L. & Paterson, B. M. Targeted disruption of gene function in Drosophila by RNA interference: a role for nautilus in embryonic somatic muscle formation. Proc. Natl Acad Sci. USA 96, 1451-1456 (1999).
13. Kalejta, R. F., Brideau, A. D., Banfield, B. W. & Beavis, A. J. An integral membrane green fluorescent protein marker, Us9-GFP, is quantitatively retained in cells during propidium iodine-based cell cycle analysis by flow cytometry. Exp. Cell. Res. 248, 322-328 (1999).
14. Wolf, D. A. & Jackson, P. K. Cell cycle: oiling the gears of anaphase. Curr. Biol. 8, R637-R639 (1998).
15. Kramer, E. R., Gieffers, C., Holz, G., Hengstschlager, M. & Peters, J. M. Activation of the human anaphase-promoting complex by proteins of the CDC20/fizzy family. Curr. Biol. 8, 1207-1210 (1998).
16. Shuttleworth, J. & Colman, A. Antisense oligonucleotide-directed cleavage of mRNA in Xenopus oocytes and eggs. EMBO J. 7, 427-434 (1988).
17. Tabara, H., Grishok, A. & Mello, C., C. RNAi in C. elegans: soaking in the genome sequence. Science 282, 430-432 (1998).
18. Bosher, J. M., Dufourcq, P., Sookhareea, S. & Labouesse, M. RNA interference can target pre-mRNA. Consequences for gene expression in a Caenorhabditis clegans operon. Genetics 153, 1245-1256 (1999).
19. Hamilton, J. A. & Baulcombe, D. C. A species of small antisense RNA in posttranscriptional gene silencing in plants. Science 286, 950-952 (1999).
20. Jones, L. A., Thomas, C. L. & Maule, A. J. De novo methylation and co-suppression induced by a cytoplasmically replicating plant RNA virus. EMBO J. 17, 6385-6393 (1998).
21. Jones, L. A. et al. RNA-DNA interactions and DNA methylation in post-transcriptional gene silencing. Plant Cell 11, 2291-2301 (1999).
22. Schneider, I. Cell lines derived from late embryonic stages of Drosophila melanogaster. J. Embryol, Exp. Morpho. 27, 353-365 (1972).
23. Di Nocera, P. P. & Dawid, I. B. Transient expression of genes introduced into cultured cells of Drosophila. Proc. Natl Acad. Set. USA 80, 7095-7098 (1983).

### Example 2: Role for a bidentate ribonuclease in the initiation step of RNA interference

Genetic approaches in worms, fungi and plants have identified a group of proteins that are essential for double-stranded RNA-induced gene silencing. Among these are ARGONAUTE family members (e.g. RDE1 QDE2)^{9,10,30}, recQ-family helicases (MUT-7, QDE3)^{11,12}, and RNA-dependent RNA polymerases (e.g. EGO-1, QDE1, SGS2/SDE1)¹³⁻¹⁶. While potential roles have been proposed, none of these genes has been assigned a definitive function in the silencing process. Biochemical studies have suggested that PTGS is accomplished by a multicomponent nuclease that targets mRNAs for degradation^{6,8,17}. We have shown that the specificity of this complex may derive from the incorporation of a small guide sequence that is homologous to the mRNA subsirate⁶. Originally identified in plants that were actively silencing transgenes², these ~22 nt RNAs. have been produced during RNAi *in vitro* using an extract prepared from *Drosophila* embryos^{3.} Putative guide RNAs can also be produced in extracts from *Drosophila* S2 cells (Fig. 5a). With the goal of understanding the mechanism of post-transcriptional gene silencing, we have undertaken both biochemical fractionation and candidate gene approaches to identify the enzymes that execute each step of RNAi.

Our previous studies resulted in the partial purification of a nurlease, RISC, that is an effector of RNA interference. See Example 1. This enzyme was isolated from *Drosophila* S2 cells in which RNAi had been initiated *in vivo* by transfection with dsRNA. We first sought to determine whether the RISC enzyme and the enzyme that initiates RNAi via processing of dsRNA into 22mers are distinct activities. RISC activity could be largely cleared from extracts by high-speed centrifugation (100,000xg for 60 min.) while the activity that produces 22mers remained in the supernatant (Fig. 5b,c). This simple fractionation indicated that RISC and the 22mer-generatirig activity are separable and thus distinct enzymes. However, it seems likely that they might interact at some point during the silencing process.

RNAse III family members are among the few nucleases that show specificity for double-stranded RNA¹⁸. Analysis of the *Drosophila* and *C, elegans* genomes reveals several types of RNAse III enzymes. First is the canonical RNAse III which contains a single RNAse III signature motif and a double-stranded RNA binding domain (dsRBD; e.g. RNC_CAEEL). Second is a class represented by Drosha¹⁹, a *Drosophila* enzyme that contains two RNAse III motifs and a dsRBD (CeDrosha in C. *elegans*). A third class contains two RNAse III signatures and an amino terminal helicase domain (e.g. *Drosophila* CG4792, CG6493, *C*. *elegans* K12H4.8), and these had previously been proposed by Bass as candidate RNAi nucleases²⁰. Representatives of all three classes were tested for the ability to produce discrete, ~22 nt. RNAs from dsRNA substrates.

Partial digestion of a 500 nt. cyclin E dsRNA with purified, bacterial RNAse III produced a smear of products while nearly complete digestion produced a heterogeneous group of ~11-17 nucleotide RNAs (not shown). In order to test the dual-RNAse III enzymes, we prepared T7 epitope-tagged versions of Drosha and CG4792. These were expressed in transfected S2 cells and isolated by immunoprecipitation using antibody-agarose conjugates. Treatment of the dsRNA with the CG4792 immunoprecipitate yielded ~22 nt. fragments similar to those produced in either S2 or embryo extracts (Fig. 6a). Neither activity in extract nor activity in immunoprecipitates depended on the sequence of the RNA substrate since dsRNAs derived from several genes were processed equivalently (see Supplement 1). Negative results were obtained with Drosha and with immunoprecipitates of a DExH box helicase (Homeless²¹; see Fig 6a,b). Western blotting confirmed that each of the tagged proteins was expressed and immunoprecipitated similarly (see Supplement 2). Thus, we conclude that CG4792 may carry out the initiation step of RNA interference by producing ~22 nt. guide sequences from dsRNAs. Because of its ability to digest dsRNA into uniformly sized, small RNAs, we have named this enzyme Dicer (*Dcr*). *Dicer* mRNA is expressed in embryos, in S2 cells; and in adult flies, consistent with the presence of functional RNAi machinery in all of these contexts (see Supplement 3).

The possibility that Dicer might be the nuclease responsible for the production of guide RNAs from dsRNAs prompted us to raise an antiserum directed against the caxboxy-terminus of the Dicer protein (Dicer-1, CG4792). This antiserum could immunoprecipitate a nuclease activity from either Drosophila embryo extracts or from S2 cell lysates that produced ~22 nt. RNAs from dsRNA substrates (FAg. 6C). The putative guide RNAs that are produced by the Dicer-1 enzyme precisely comigrate with 22mers that are produced in extract and with 22mers that are associated with the RISC enzyme (Fig. 6 D,F). It had previously been shown that the enzyme that produced guide RNAs in Drosophila embryo extracts was ATP-dependent⁸. Depletion of this cofactor resulted in an ~6-fold lower rate of dsRNA cleavage and in the production of RNAs with a slightly lower mobility. Of interest was the fact that both Dicer-I immunoprecipitates and extracts from S2 cells require ATP for the production of ~22mers (Fig. 6D). We do not observe the accumulation of lower mobility products in these cases, although we do routinely observe these in ATP-depleted embryo extracts. The requirement of this nuclease for ATP is a quite unusual property. We hypothesize that this requirement could indicate that the enzyme may act processively on the dsRNA, with the helicase domain harnessing the energy of ATP hydrolysis both for unwinding guide RNAs and for translocation along the substrate.

Efficient induction of RNA interference in C. *elegans* and in *Drosophila* has several requirements. For example, the initiating RNA must be double-stranded, and it must be several hundred nucleotides in length. To determine whether these requirements are dictated by Dicer, we characterized the ability of extracts and of immunoprecipitated enzyme to digest various RNA substrates. Dicer was inactive against single stranded RNAs regardless of length (see Supplement 4). The enyzme could digest both 200 and 500 nucleotide dsRNAs but was significantly less active with shorter substrates (see Supplement 4). Double-stranded RNAs as short as 35 nucleotides could be cut by the enzyme, albeit very inefficiently (data not shown). In contrast, E. coli RNAse III could digest to completion dsRNAs of 35 or 22 nucleotides (not shown). This suggests that the substrate preferences of the Dicer enzyme may contribute to but not wholly determine the size dependence of RNAi.

To determine whether the Dicer enzyme indeed played a role in RNAi *in vivo,* we sought to deplete Dicer activity from S2 cells and test the effect on dsRNA-induced gene silencing. Transfection of S2 cells with a mixture of dsRNAs homologous to the two Drosophila Dicer genes (CG4792 and CG6493) resulted in an ~6.7 fold reduction of Dicer activity either in whole cell lysates or in Dicer-1 immunoprecipitates (Fig. 7A,B). Transfection with a control dsRNA (murine caspase 9) had no effect. Qualitatively similar results were seen if Dicer was examined by Northern blotting (not shown). Depletion of Dicer in this manner substantially compromised the ability of cells to silence subsequently an exogenous, GFP transgene by RNAi (Fig. 7C). These results indicate that Dicer is involved in RNAi *in vivo.* The lack of complete inhibition of silencing could result from an incomplete suppression of Dicer (which is itself required for RNAi) or could indicate that *in vivo*, guide RNAs can be produced by more than one mechanism (e.g. through the action of RNA-dependent RNA polymerases).

Our results indicate that the process of RNA interference can be divided into at least two distinct steps. According to this model, initiation of PTGS would occur upon processing of a double-stranded RNA by Dicer into ~22 nucleotide guide sequences, although we cannot formally exclude the possibility that another, Dicer-associated nuclease may participate in this process. These guide RNAs would be incorporated into a distinct nuclease complex (RISC) that targets single-stranded mRNAs for degradation. An implication of this model is that guide sequences are themselves derived directly from the dsRNA that triggers the response. In accord with this model, we have demonstrated that ³²P-labeled, exogenous dsRNAs that have been introduced into S2 cells by transfection are incorporated into the RISC enzyme as 22 mers (Fig. 7E). However, we cannot exclude the possibility that RNA-dependent RNA polymerases might amplify 22mers once they have been generated or provide an alternative method for producing guide RNAs.

The structure of the Dicer enzyme provokes speculation on the mechanism by which the enzyme might produce discretely sized fragments irrespective of the sequence of the dsRNA (see Supplement 1, Fig. 8a), It has been established that bacterial RNAse III acts on its substrate as a dimer^{18,22,23}. Similarly, a dimer of Dicer enzymes may be required for cleavage of dsRNAs into -22 nt, pieces. According to one model, the cleavage interval would be determined by the physical arrangement of the two RNAse III domains within Dicer enzyme (Fig. 8a). A plausible alternative model would dictate that cleavage was directed at a single position by the two RIII domains in a single Dicer protein. The 22 nucleotide interval could be dictated by interaction of neighboring Dicer enzymes or by translocation along the mRNA substrate. The presence of an integral helicase domain suggests that the products of Dicer cleavage might be single-stranded 22 mers that are incorporated into the RISC enzyme as such.

A notable feature of the Dicer family is its evolutionary conservation. Homologs are found in C. *elegans* (K12H4.8), *Arabidopsis* (e.g., CARPEL FACTORY²⁴, T25K16.4, AC012328_1), mammals (Helicase-MOI²⁵) and *S. pombe* (YC9A_SCHPO) (Fig 8b, see Supplements 6,7 for sequence comparisons). In fact, the human Dicer family member is capable of generating ~22 nt. RNAs from dsRNA substrates (Supplement 5) suggesting that these structurally similar proteins may all share similar biochemical functions. It has been demonstrated that exogenous dsRNAs can affect gene function in early mouse embryos²⁹, and our results suggest that this regulation may be accomplished by an evolutionarily conserved RNAi machinery.

In addition to RNAseIII and helicase motifs, searches of the PFAM database indicate that each Dicer family member also contains a ZAP domain (Fig 8c)²⁷. This sequence was defined based solely upon its conservation in the Zwille/ARGONAUTE/Piwi family that has been implicated in RNAi by mutations *in C*. *elegans* (Rde-1)⁹ and *Neurospora* (Qde-2)¹⁰. Although the function of this domain is unknown, it is intriguing that this region of homology is restricted to two gene families that participate in dsRNA-dependent silencing. Both the ARGONAUTE and Dicer families have also been implicated in common biological processes, namely the determination of stem-cell fates. A hypomorphic allele of *carpel factory,* a member of the Dicer family in *Arabidopsis,* is characterized by increased proliferation in floral meristems²⁴. This phenotype and a number of other characteristic features are also shared by *Arabidopsis ARGONAUTE* (*agol-1*) mutants²⁶ (C. Kidner and R. Martiennsen, pers. comm.). These genetic analyses begin to provide evidence that RNAi may be more than a defensive response to unusual RNAs but may also play important roles in the regulation of endogenous genes.

With the identification of Dicer as a catalyst of the initiation step of RNAi, we have begun to unravel the biochemical basis of this unusual mechanism of gene regulation. It will be of critical importance to determine whether the conserved family members from other organisms, particularly mammals, also play a role in dsRNA-mediated gene regulation.

### Methods

Plasmid constructs. A full-length cDNA encoding Drosha was obtained by PCR from an EST sequenced by the Berkeley Drosophila genome project. The *Homeless* clone was a gift from Gillespie and Berg (Univ. Washington). The T7 epitope-tag was added to the amino terminus of each by PCR, and the tagged cDNAs were cloned into pRIP, a retroviral vector designed specifically for expression in insect cells (E. Bernstein, unpublished). In this vector, *expression* is driven by the *Orgyia pseudotsugata* IE2 promoter (Invitrogen). Since no cDNA was available for CG4792/Dicer, a genomic clone was amplified from a bacmid (BACR23F10; obtained from the BACPAC Resource Center in the Dept. of Human Genetics at the Roswell Park Cancer Institute). Again, during amplification, a T7 epitope tag was added at the amino terminus of the coding sequence. The human Dicer gene was isolated from a cDNA library prepared from HaCaT cells (GJH, unpublished). A T7-tagged version of the complete coding sequence was cloned into pCDNA3 (Invitrogen) for expression in human cells (LinX-A).

Cell culture and extract preparation. *S2 and embryo culture,* S2 cells were cultured at 27°C in 5% CO₂ in Schneider's insect media supplemented with 10% heat inactivated fetal bovine serum (Gemini) and 1% antibiotic-antimycotic solution (Gibco BRL). Cells were harvested for extract preparation at 10x10⁶ cells/ml. The cells were washed 1X in PBS and were resuspended in a hypotonic buffer (10 mM Hepes pH 7.0, 2mM MgCl2, 6 mM βME) and dounced. Cell lysates were spun 20,000xg for 20 minutes. Extracts were stored at -80°C. *Drosophila* embryos were reared in fly cages by standard methodologies and were collected every 12 hours. The embryos were dechorionated in 50% chlorox bleach and washed thoroughly with distilled water. Lysis buffer (10mM Hepes, 10mM KCl, 1.5 mM MgCl₂, 0.5mM EGTA, 10mM β-glycerophosphate, 1mM DTT, 0.2 mM PMSF) was added to the embryos, and extracts were prepared by homogenization in a tissue grinder. Lysates were spun for two hours at 200,000xg and were frozen at -80°C. LinX-A cells, a highly-transfectable derivative of human 293 cells, (Lin Xie and GJH, unpublished) were maintained in DMEM/10%FCS.

Transfections and immunoprecipitations. S2 cells were transfected using a calcium phosphate procedure essentially as previously described⁶. Transfection rates were ~90% as monitored in controls using an *in situ* β-galactosidase assay. LinX-A cells were also transfected by calcium phosphate co-precipitation. For immunoprecipitations, cells (~ 5x10⁶ per IP) were transfected with various clones and lysed three days later in IP buffer (125mM KOAc, 1mM MgOAc, 1mM CaCl₂, 5mM EGTA, 20mM Hepes pH 7.0, 1mM DTT, 1% NP-40 plus Complete protease inhibitors (Roche)). Lysates were spun for 10 minutes at 14,000xg and supernatants were added to T7 antibody-agarose beads (Novagen). Antibody binding proceeded for 4 hours at 4°C. Beads were centrifuged and washed in lysis buffer three times, and once in reaction buffer. The Dicer antiserum was raised in rabbits using a KLH-conjugated peptide corresponding to the C-terminal 8 amino acids of Drosophila Dicer-1 (CG4792).

Cleavage reactions. *RNA preparation.* Templates to be transcribed into dsRNA were generated by PCR with forward and reverse primers, each containing a T7 promoter sequence. RNAs were produced using Riboprobe (Promega) kits and were uniformly labeling during the transcription reaction with ³²P-UTP. Single-stranded RNAs were purified from 1% agarose gels. *dsRNA cleavage.* Five microliters of embryo or S2 extracts were incubated for one hour at 30°C with dsRNA in a reaction containing 20mM Hepes pH 7.0, 2mM MgOAc, 2mM DTT, 1mM ATP and 5% Superasin (Ambion). Immunoprecipitates were treated similarly except that a minimal volume of reaction buffer (including ATP and Superasin) and dsRNA were added to beads that had been washed in reaction buffer (see above). For ATP depletion, *Drosophila* embryo extracts were incubated for 20 minutes at 30°C with 2mM glucose and 0.375 U of hexokinase (Roche) prior to the addition of dsRNA.

Northern and Western analysis. Total RNA was prepared from *Drosophila* embryos (0-12 hour), from adult flies, and from S2 cells using Trizol (Liferech). Messenger RNA was isolated by affinity selection using magnetic oligo-dT beads (Dynal). RNAs were electrophoresed on denaturing formaldehyde/agarose gels, blotted and probed with randomly primed DNAs corresponding to Dicer. For Western analysis, T7-tagged proteins were immunoprecipitated from whole cell lysates in IP buffer using anti-T7-antibody-agarose conjugates. Proteins were released from the beads by boiling in Laemmli buffer and were separated by electrophoresis on 8% SDS PAGE. Following transfer to nitroeellulose, proteins were visualized using an HRP-conjugated anti-T7 antibody (Novagen) and chemiluminescent detection (Supersignal, Pierce).

RNAi of Dicer. Drosophila S2 cells were transfected either with a dsPNA corresponding to mouse caspase 9 or with a mixture of two dsRNAs corresponding to Drosophila Dicer-1 and Dicer-2 (CG4792 and CG6493). Two days after the initial transfection, cells were again transfected with a mixture containing a GFP expression plasmid and either luciferase dsRNA or GFP dsRNA as previously described⁶. Cells were assayed for Dicer activity or fluorescence three days after the second transfection. Quantification of fluorescent cells was done on a Coulter EPICS cell sorter after fixation. Control transfections indicated that Dicer activity was not affected by the introduction of caspase 9 dsRNA.

### References cited Example 2

1. Baulcombe, D. C. RNA as a target and an initiator of post-transcriptional gene silencing in transgenic plants. Plant Mol Biol 32, 79-88 (1996).
2. Wassenegger, M. & Pelissier, T. A model for BNA-mediated gene silencing in higher plants. Plant Mol Biol 37, 349-62 (1998).
3. Montgomery, M. K. & Fire, A. Double-stranded RNA as a mediator in sequence-specific genetic silencing and co-suppression [see comments]. Trends Genet 14, 255-8 (1998).
4. Sharp, P. A. RNAI and double-strand RNA. Genes Dev 13, 139-41 (1999).
5. Sijen, T. & Kooter, J. M. Post-transcriptional gene-silencing: RNAs on the attack or on the defense? [In Process Citation]. Bioessays 22, 520-31 (2000).
6. Hammond, S. M., Bernstein, E., Beach, D. & Hannon, G. J. An RNA-directed nuclease mediates post-transcriptional gene silencing in Drosophila cells. Nature 404,293-6 (2000).
7. Hamilton, A. J. & Baulcombe, D. C. A species of small antisense RNA in posttranscriptional gene silencing in plants [see comments]. Science 286, 950-2 (1999).
8. Zamore, P. D., Tuschl, T., Sharp, P. A. & Bartel, D. P. RNAi: double-stranded RNA directs the ATP-dependent cleavage of mRNA at 21 to 23 nucleotide intervals. Cell 101, 25-33 (2000).
9. Tabara, H, et al. The rde-1 gene, RNA interference, and transposon silencing in C. elcgans. Cell 99, 123-32 (1999).
10. Catalanoto, C., Azzalin, G., Macino, G. & Cogoni, C. Gene silencing in wornis and fungi. Nature 404, 245 (2000).
11. Ketting, R. F., Haverkamp, T. H., van Luenen, H. G. & Plasterk, R. H. Mut-7 of C. elegans, required for transposon silencing and RNA interference, is a homolog of Werner syndrome helicase and RNaseD. Cell 99, 133-41 (1999).
12. Cogoni, C. & Macino, G. Posttranscriptional gene silencing in Neurospora by a RecQ DNA helicase. Science 286, 2342-4 (1999).
13. Cogoni, C. & Macino, G. Gene silencing in Neurospora crassa requires a protein homologous to RNA-dependent RNA polymerase. Nature 399, 166-9 (1999).
14. Smardon, A. et al. EGO-1 is related to RNA-directed RNA polymerase and functions in germ-line development and RNA interference in C. elegans [published enatum appears in Curr Biol 2000 May 18;10(10):R393-4]. Curr Biol 10, 169-78 (2000).
15. Mourrain, P. et al. Arabidopsis SGS2 and SGS3 genes are required for posttranscriptional gene silencing and natural virus resistance. Cell 101, 533-42 (2000).
16. Dalmay, T., Hamilton, A., Rudd, S., Angell, S. & Baulcombe, D. C. An RNA-dependent RNA polymerase gene in Arabidopsis is required for posttranscriptional gene silencing mediated by a transgene but not by a virus. Cell I01, 543-53 (2000).
17. Tuschl, T., Zamore, P. D., Lehmann, R., Bartel, D. P. & Sharp, P. A. Targeted mRNA degradation by double-stranded RNA in vitro. Genes Dev 13, 3191-7 (1999).
18. Nicholson, A. W. Function, mechanism and regulation of bacterial ribonucleases. FEMS Microbiol Rev 23, 371-90 (1999).
19. Filippov, V., Solovyev, V., Filippova, M. & Gill, S. S. A novel type of RNase III family proteins in eukaryotes. Gene 245, 213-21 (2000).
20. Bass, B. L. Double-stranded RNA as a template for gene silencing. Cell 101, 235-8 (2000).
21. Gillespie, D. E. & Berg, C. A. Homeless is required for RNA localization in Drosophila oogenesis and encodes a new member of the DE-H family of RNA-dependent ATPases. Genes Dev 9, 2495-508 (1995).
22. Robertson, H. D., Webster, R. E. & Zinder, N. D. Purification and properties of ribonuclease III from Escherichia coli. J Biol Chem 243, 82-91 (1968).
23. Dunn, J. J. RNase III cleavage of single-stranded RNA. Effect of ionic strength on the fideltiy of cleavage. J Biol Chem 251, 3807-14 (1976).
24. Jacobsen, S. E., Running, M. P. & Meyerowitz, E. M. Disruption of an RNA helicase/RNAse III gene in Arabidopsis causes unregulated cell division in floral meristems. Development 126, 5231-43 (1999).
25. Matsuda, S. et al. Molecular cloning and characterization of a novel human gene (HERNA) which encodes a putative RNA-helicase. Blochim Biophys Acta 1490, 163-9 (2000).
26. Bohmert, K. et al. AGOI defines a novel locus of Arabidopsis controlling leaf development. Embo J 17,170-80 (1998).
27. Sonnhammer, E. L., Eddy, S. R. & Durbin, R. Pfam: a comprehensive database of protein domain families based on seed alignments. Proteins 28, 405-20 (1997).
28. Altschul, S. F. et al. Gapped BLAST and PSI-BLAST: a new generation of protein database search programs. Nucleic Acids Res 25, 3389-402 (1997).
29. Wianny, F. and Zemicka-Goetz, M. Specific interference with gene function by double-stranded RNA in early mouse development. Nature Cell Biol. 2, 70-75 (2000).
30. Fagard, M., Boutet, S., Morel, J. B., Bellini, C. and Vaucheret, H. Ago-1, Qde-2 and Rde-1 are related proteins required for post-transcriptional gene silencing in plants, quelling in fungi, and RNA interference in animals. Proc. Natl. Acad Sci. USA 97, 11650-11654 (2000).

### Example 3: A simplified method for the creation of hairpin constructs for RNA interference.

In numerous model organisms, double stranded RNAs have been shown to cause effective and specific suppression of gene function (ref. 1). This response, termed RNA interference or post-transcriptional gene silencing, has evolved into a highly effective reverse genetic tool in *C. elegans, Drosophila,* plants and numerous other systems. In these cases, double-stranded RNAs can be introduced by injection, transfection or feeding; however, in all cases, the response is both transient and systemic. Recently, stable interference with gene expression has been achieved by expression of RNAs that form snap-back or hairpin structures (refs 2-7). This has the potential not only to allow stable silencing of gene expression but also inducible silencing as has been observed in trypanosomes and adult *Drosophila* (refs 2,4,5). The utility of this approach is somewhat hampered by the difficulties that arise in the construction of bacterial plasmids containing the long inverted repeats that are necessary to provoke silencing. In a recent report, it was stated that more than 1,000 putative clones were screed to identify the desired construct (ref 7).

The presence of hairpin structures often induces plasmid rearrangement, in part due to the E. coli sbc proteins that recognize and cleave cruciform DNA structures (ref 8). We have developed a method for the construction of hairpins that does not require cloning of inverted repeats, per se. Instead, the fragment of the gene that is to be silenced is cloned as a direct repeat, and the inversion is accomplished by treatment with a site-specific recombinase, either *in vitro* (or potentially *in vivo)* (see Fig 29). Following recombination, the inverted repeat structure is stable in a bacterial strain that lacks an intact SBC system (DL759). We have successfully used this strategy to construct numerous hairpin expression constructs that have been successfully used to provoke gene silencing in Drosophila cells.

### Literature Cited in Example 3

1. Bosher, J. M. & Labouesse, M. RNA interference; genetic wand and genetic watchdog. Nat Cell Biol 2, E31-6 (2000).
2. Fortier, E. & Belote, J. M. Temperature-dependent gene silencing by an expressed inverted repeat in Drosophila [published erratum appears in Genesis;2000 May;27(1):47], Genesis 26, 240-4 (2000).
3. Kennerdell, J. R. & Carthew, R. W. Heritable gene silencing in Drosophila using double-stranded RNA. Nat Bioteclmol 18, 896-8 (2000).
4. Lam, G. & Thummel, C. S, Inducible expression of double-stranded RNA directs specific genetic interference in Drosophila [In Process Citation]. Curr Biol 10, 957-63 (2000).
5. Shi, H. et al. Genetic interference in Trypanosoma brucei by heritable and inducible double-stranded RNA. Rna 6, 1069-76 (2000).
6. Smith, N. A. et al. Total silencing by intron-spliced hairpin RNAs. Nature 407; 319-20(2000).
7. Tavernarakis, N., Wang, S. L., Dorovkov, M., Ryazanov, A. & Driscoll, M. Heritable and inducible genetic interference by double-stranded RNA encoded by transgenes. Nat Genet 24, 180-3 (2000).
8. Connelly, J. C. & Leach, D. R. The sbcC and sbcD genes of Escherichia coli encode a nuclease involved in palindrome inviability and genetic recombination. Genes Cells 1, 285-91 (1996).

### V. Equivalents

Those skilled in the art will recognize, or be able to ascertain using no more than routine experimentation, many equivalents to the specific embodiments of the invention described herein. Such equivalents are intended to be encompassed by the following claims.

All of the above-cited references and publications are hereby incorporated by reference.

## Claims

1. An *in vitro* method for attenuating expression of a target gene in a non-oocytic cell suspended in culture, comprising transfecting the cell with a double stranded RNA (dsRNA) in an amount sufficient to attenuate expression of the target gene, wherein the dsRNA is a single self-complementary RNA strand and comprises a nucleotide sequence that is capable of hybridizing to a nucleotide sequence of the target gene in the presence of 400 mM NaCl, 40 mM PIPES pH 6.4, 1 mM EDTA, 50°C or 70°C for 12-16 hours, followed by washing, and (i) is cleaved by dicer into nucleotides of about 22 mers in the cell; (ii) attenuates expression of the target gene in a sequence-specific manner; and (iii) does not cause sequence-independent apoptosis.

2. The method of claim 1, wherein the target gene is an endogenous gene of the cell.

3. The method of claim 1, wherein the target gene is an oncogene.

4. The method of claim 1, wherein the target gene is a pathogen gene which is present in the cell after infection thereof.

5. The method of claim 1, wherein the cell is a mammalian cell.

6. The method of claim 1, wherein the cell is a primate cell.

7. The method of claim 6, wherein the primate cell is a human cell.

8. The method of claim 1, wherein the cell is a somatic cell.

9. The method of claim 1, wherein expression of the target gene is attenuated by at least 6 fold.

10. The method of claim 1, wherein the dsRNA is produced by a vector.

11. The method of claim 1, wherein the dsRNA is chemically synthesized.

12. The method of claim 1, wherein the transfection is conducted using a chemical mediated transport.

13. The method of claim 1, wherein the transfection is conducted using a lipid-mediated carrier transport.

14. A non-human transgenic mammal having germline and/or somatic cells comprising a transgene encoding a double stranded RNA (dsRNA), wherein the dsRNA comprises a nucleotide sequence that hybridizes in cells of the transgenic mammal to a target gene, and (i) is cleaved by dicer into nucleotides of about 22 mers in the cell; (ii) attenuates expression of the target gene in a sequence-specific manner; and (iii) does not cause sequence-independent apoptosis.

15. The transgenic mammal of claim 14, which is chimeric for said transgene.

16. The transgenic mammal of claim 14, wherein said transgene is chromosomally incorporated.


SEQUENCE LISTING

<110> Genetica, Inc.

<120> Methods and Compositions for RNA Interference

<130> GCNA-pWO-007

<140> PCT/US01/—,—
<141> 2001-03-16

<150> US 60/189,739
<151> 2000-03-16

<150> US 60/243,097
<151> 2000-10-24

<160> 4

<170> PatentIn version 3.0

<210> 1
<211> 5775
<212> DNA
<213> Homo sapiens

<220>
<221> CDS
<222> (1)..(5775)

<400> 1
atg aaa agc cct gct ttg caa ccc ctc agc atg gca ggc ctg cag ctc 48

1               5                   10                  15

atg acc cct gct tcc tca cca atg ggt cct ttc ttt gga ctg cca tgg 96


            20                  25                  30

caa caa gaa gca att cat gat aac att tat acg cca aga aaa tat cag 144

        35                  40                  45

gtt gaa ctg ctt gaa gca gct ctg gat cat aat acc atc gtc tgt tta 192

    50                  55                  60

aac act ggc tca ggg aag aca ttt att gct agt act act cta cta aag 240

65                  70                  75                  80

agc tgt ctc tat cta gat cta ggg gag act tca gct aga aat gga aaa 288

                85                  90                  95

agg.acg gtg ttc ttg gtc aac tct gca aac cag gtt gct caa caa gtg 336

            100                 105                 110

tca gct gtc aga act cat tca gat ctc aag gtt ggg gaa tac tca aac 384

        115                 120                 125

cta gaa gta aat gca tct tgg aca aaa gag aga tgg aac caa gag ttt 432

    130                 135                 140

act aag cac cag gtt ctc att atg act tgc tat gtc gcc ttg aat gtt 480

145                 150                 155                 160

ttg aaa aat ggt tac tta tca ctg tca gac att aac ctt ttg gtg ttt 528

                165                 170                 175

gat gag tgt cat ctt gca atc cta gac cac ccc tat cga gaa ttt atg 576

            180                 185                 190

aag ctc tgt gaa att tgt cca tca tgt cct cgc att ttg gga cta act 624

        195                 200                 205

gct tcc att tta aat ggg aaa tgg gat cca gag gat ttg gaa gaa aag 672

    210                 215                 220

ttt cag aaa cta gag aaa att ctt aag agt aat gct gaa act gca act 720

225                 230                 235                 240

gac ctg gtg gtc tta gac agg tat act tct cag cca tgt gag att gtg 768

                245                 250                 255

gtg gat tgt gga cca ttt act gac aga ag[?] ggg ctt tat gaa aga ctg 816

            260                 265                 270

ctg atg gaa tta gaa gaa gca ctt aat ttt atc aat gat tgt aat ata 864

        275                 280                 285

tct gta cat tca aaa gaa aga gat tct act tta att tcg aaa cag ata 912

    290                 295                 300

cta tca gac tgt cgt gcc gta ttg gta gtt ctg gga ccc tgg tgt gca 960

305                 310                 315                 320

gat aaa gta gct gga atg atg gta aga gaa cta cag aaa tac atc aaa 1008

                325                 330                 335

cat gag caa gag gag ctg cac agg aaa ttt tta ttg ttt aca gac act 1056

            340                 345                 350

ttc cta agg aaa ata cat gca cta tgt gaa gag cac ttc tca cct gcc 1104

        355                 360                 365

tca ctt gac ctg aaa ttt gta act cct aaa gta atc aaa ctg ctc gaa 1152

    370                 375                 380

atc tta cgc aaa tat aaa cca tat gag cga cac agt ttt gaa agc gtt 1200

385                 390                 395                 400

gag tgg tat aat aat aga aat cag gat aat tat gtg tca tgg agt gat 1248

                405                 410                 415

tct gag gat gat gat gag gat gaa gaa att gaa gaa aaa gag aag cca 1296

            420                 425                 430

gag aca aat ttt cct tct cct ttt acc aac att ttg tgc gga att att 1344

        435                 440                 445

ttt gtg gaa aga aga tac aca gca gtt gtc tta aac aga ttg ata aag 1392

    450                 455                 460

gaa gct ggc aaa caa gat cca gag ctg gct tat atc agt agc aat ttc 1440


465                 470                 475                 480

ata act gga cat ggc att ggg aag aat cag cct cgc aac aac acg atg 1488

                485                 490                 495

gaa gca gaa ttc aga aaa cag gaa gag gta ctt agg aaa ttt cga gca 1536

            500                 505                 510

cat gag acc aac ctg ctt att gca aca agt att gta gaa gag ggt gtt 1584

        515                 520                 525

gat ata cca aaa tgc aac ttg gtg gtt cgt ttt gat ttg ccc aca gaa 1632

    530                 535                 540

tat cga tcc tat gtt caa tct aaa gga aga gca agg gca ccc atc tct 1680

545                 550                 555                 560

aat tat ata atg tta gcg gat aca gac aaa ata aaa agt ttt gaa gaa 1728

                565                 570                 575

gac ctt aaa acc tac aaa gct att gaa aag atc ttg aga aac aag tgt 1776

            580                 585                 590

tcc aag tcg gtt gat act ggt gag act gac att gat cct gtc atg gat 1824

        595                 600                 605

gat gat cac gtt ttc cca cca tat gtg ttg agg cct gac gat ggt ggt 1872


    610                 615                 620

cca cga gtc aca atc aac acg gcc att gga cac atc aat aga tac tgt 1920

625                 630                 635                 640

gct aga tta cca agt gat ccg ttt act cat cta gct cct aaa tgc aga 1968

                645                 650                 655

acc cga gag ttg cct gat ggt aca ttt tat tca act ctt tat ctg cca 2016

            660                 665                 670

att aac tca cct ctt cga gcc tcc att gtt ggt cca cca atg agc tgt 2064

        675                 680                 685

gta cga ttg gct gaa aga gtt gtc gct ctc att tgc tgt gag aaa ctg 2112

    690                 695                 700

cac aaa att ggc gaa ctg gat gac cat ttg atg cca gtt ggg aaa gag 2160

705                 710                 715                 720

act gtt aaa tat gaa gag gag ctt gat ttg cat gat gaa gaa gag acc 2208

                725                 730                 735

agt gtt cca gga aga cca ggt tcc acg aaa cga agg cag tgc tac cca 2256

            740                 745                 750

aaa gca att cca gag tgt ttg agg gat agt tat ccc aga cct gat cag 2304

        755                 760                 765

ccc tgt tac ctg tat gtg ata gga atg gtt tta act aca cct tta cct     2352

    770                 775                 780

gat gaa ctc aac ttt aga agg cgg aag ctc tat cct cct gaa gat acc     2400

785                 790                 795                 800

aca aga tgc ttt gga ata ctg acg gcc aaa ccc ata cct cag att cca     2448

                805                 810                 815

cac ttt cct gtg tac aca cgc tct gga gag gtt acc ata tcc att gag     2496

            820                 825                 830

ttg aag aag tct ggt ttc atg ttg tct cta caa atg ctt gag ttg att     2544

        835                 840                 845

aca aga ctt cac cag tat ata ttc tca cat att ctt cgg ctt gaa aaa     2592

    850                 855                 860

cct gca cta gaa ttt aaa cct aca gac gct gat tca gca tac tgt gtt     2640

865                 870                 875                 880

cta cct ctt aat gtt gtt aat gac tcc agc act ttg gat att gac ttt     2688

                885                 890                 895

aaa ttc atg gaa gat att gag aag tct gaa gct cgc ata ggc att ccc     2736

            900                 905                 910

agt aca aag tat aca aaa gaa aca ccc ttt gtt ttt aaa tta gaa gat 2784

        915                 920                 925

tac caa gat gcc gtt atc att cca aga tat cgc aat ttt gat cag cct 2832

    930                 935                 940

cat cga ttt tat gta gct gat gtg tac act gat ctt acc cca ctc agt 2880

945                 950                 955                 960

aaa ttt cct tcc cct gag tat gaa act ttt gca gaa tat tat aaa aca 2928

                965                 970                 975

aag tac aac ctt gac cta acc aat ctc aac cag cca ctg ctg gat gtg 2976

            980                 985                 990

gac cac aca tct tca aga ctt aat ctt ttg aca cct cga cat ttg aat 3024

        995                 1000                1005

cag aag ggg aaa gcg ctt cct tta agc agt gct gag aag agg aaa 3069

    1010                1015                1020

gcc aaa tgg gaa agt ctg cag aat aaa cag ata ctg gtt cca gaa 3114

    1025                1030                1035

ctc tgt gct ata cat cca att cca gca tca ctg tgg aga aaa gct 3159

    1040                1045                1050

gtt tgt ctc ccc agc ata ctt tat cgc ctt cac tgc ctt ttg act 3204


    1055                1060                1065

gca gag gag cta aga gcc cag act gcc agc gat gct ggc gtg gga 3249

    1070                1075                1080

gtc aga tca ctt cct gcg gat ttt aga tac cct aac tta gac ttc 3294

    1085                1090                1095

ggg tgg aaa aaa tct att gac agc aaa tct ttc atc tca att tct 3339

    1100                1105                1110

aac tcc tct tca gct gaa aat gat aat tac tgt aag cac agc aca 3384

    1115                1120                1125

att gtc cct gaa aat gct gca cat caa ggt gct aat aga acc tcc 3429

    1130                1135                1140

tct cta gaa aat cat gac caa atg tct gtg aac tgc aga acg ttg 3474

    1145                1150                1155

ctc agc gag tcc cct ggt aag ctc cac gtt gaa gtt tca gca gat 3519

    1160                1165                1170

ctt aca gca att aat ggt ctt tct tac aat caa aat ctc gcc aat 3564

    1175                1180                1185

ggc agt tat gat tta gct aac aga gac ttt tgc caa gga aat cag 3609


    1190                1195                1200

cta aat tac tac aag cag gaa ata ccc gtg caa cca act acc tca 3654

    1205                1210                1215

tat tcc att cag aat tta tac agt tac gag aac cag ccc cag ccc 3699

    1220                1225                1230

agc gat gaa tgt act ctc ctg agt aat aaa tac ctt gat gga aat 3744

    1235                1240                1245

gct aac aaa tct acc tca gat gga agt cct gtg atg gcc gta atg 3789

    1250                1255                1260

cct ggt acg aca gac act att caa gtg ctc aag ggc agg atg gat 3834

    1265                1270                1275

tct gag cag agc cct tct att ggg tac tcc tca agg act ctt ggc 3879

    1280                1285                1290

ccc aat cct gga ctt att ctt cag gct ttg act ctg tca aac gct 3924

    1295                1300                1305

agt gat gga ttt aac ctg gag cgg ctt gaa atg ctt ggc gac tcc 3969

    1310                1315                1320

ttt tta aag cat gcc atc acc aca tat cta ttt tgc act tac cct 4014

    1325                1330                1335

gat gcg cat gag ggc cgc ctt tca tat atg aga agc aaa aag gtc 4059

    1340                1345                1350

agc aac tgt aat ctg tat cgc ctt gga aaa aag aag gga cta ccc 4104

    1355                1360                1365

agc cgc atg gtg gtg tca ata ttt gat ccc cct gtg aat tgg ctt 4149

    1370                1375                1380

cct cct ggt tat gta gta aat caa gac aaa agc aac aca gat aaa 4194

    1385                1390                1395

tgg gaa aaa gat gaa atg aca aaa gac tgc atg ctg gcg aat ggc 4239

    1400                1405                1410

aaa ctg gat gag gat tac gag gag gag gat gag gag gag gag agc 4284

    1415                1420                1425

ctg atg tgg agg gct ccg aag gaa gag gct gac tat gaa gat gat 4329

    1430                1435                1440

ttc ctg gag tat gat cag gaa cat atc aga ttt ata gat aat atg 4374

    1445                1450                1455

tta atg ggg tca gga gct ttt gta aag aaa atc tct ctt tct cct 4419

    1460                1465                1470

ttt tca acc act gat tct gca tat gaa tgg aaa atg ccc aaa aaa 4464

    1475                1480                1485

tcc tcc tta ggt agt atg cca ttt tca tca gat ttt gag gat ttt 4509

    1490                1495                1500

gac tac agc tct tgg gat gca atg tgc tat ctg gat cct agc aaa 4554

    1505                1510                1515

gct gtt gaa gaa gat gac ttt gtg gtg ggg ttc tgg aat cca tca 4599

    1520                1525                1530

gaa gaa aac tgt ggt gtt gac acg gga aag cag tcc att tct tac 4644

    1535                1540                1545

gac ttg cac act gag cag tgt att gct gac aaa agc ata gcg gac 4689

    1550                1555                1560

tgt gtg gaa gcc ctg ctg ggc tgc tat tta acc agc tgt ggg gag 4734

    1565                1570                1575

agg gct gct cag ctt ttc ctc tgt tca ctg ggg ctg aag gtg ctc 4779

    1580                1585                1590

ccg gta att aaa agg act gat cgg gaa aag gcc ctg tgc cct act 4824

    1595                1600                1605

cgg gag aat ttc aac agc caa caa aag aac ctt tca gtg agc tgt 4869


    1610                1615                1620

gct gct gct tct gtg gcc agt tca cgc tct tct gta ttg aaa gac 4914

    1625                1630                1635

tcg gaa tat ggt tgt ttg aag att cca cca aga tgt atg ttt gat 4959

    1640                1645                1650

cat cca gat gca gat aaa aca ctg aat cac ctt ata tcg ggg ttt 5004

    1655                1660                1665

gaa aat ttt gaa aag aaa atc aac tac aga ttc aag aat aag gct 5049

    1670                1675                1680

tac ctt ctc cag gct ttt aca cat gcc tcc tac cac tac aat act 5094

    1685                1690                1695

atc act gat tgt tac cag cgc tta gaa ttc ctg gga gat gcg att 5139

    1700                1705                1710

ttg gac tac ctc ata acc aag cac ctt tat gaa gac ccg cgg cag 5184

    1715                1720                1725

cac tcc ccg ggg gtc ctg aca gac ctg cgg tct gcc ctg gtc aac 5229

    1730                1735                1740

aac acc atc ttt gca tcg ctg gct gta aag tac gac tac cac aag 5274


    1745                1750                1755

tac ttc aaa gct gtc tct cct gag ctc ttc cat gtc att gat gac 5319

    1760                1765                1770

ttt gtg cag ttt cag ctt gag aag aat gaa atg caa gga atg gat 5364

    1775                1780                1785

tct gag ctt agg aga tct gag gag gat gaa gag aaa gaa gag gat 5409

    1790                1795                1800

att gaa gtt cca aag gcc atg ggg gat att ttt gag tcg ctt gct 5454

    1805                1810                1815

ggt gcc att tac atg gat agt ggg atg tca ctg gag aca gtc tgg 5499

    1820                1825                1830

cag gtg tac tat ccc atg atg cgg cca cta ata gaa aag ttt tct 5544

    1835                1840                1845

gca aat gta ccc cgt tcc cct gtg cga gaa ttg ctt gaa atg gaa 5589

    1850                1855                1860

cca gaa act gcc aaa ttt agc ccg gct gag aga act tac gac ggg 5634

    1865                1870                1875

aag gtc aga gtc act gtg gaa gta gta gga aag ggg aaa ttt aaa 5679

    1880                1885                1890

ggt gtt ggt cga agt tac agg att gcc aaa tct gca gca gca aga 5724

    1895                1900                1905

aga gcc ctc cga agc ctc aaa gct aat caa cct cag gtt ccc aat 5769

    1910                1915                1920

agc tga 5775


<210> 2
<211> 1924
<212> PRT
<213> Homo sapiens

<400> 2

<210> 3
<211> 6750
<212> DNA
<213> Drosophila melanogaster

<220>
<221> CDS
<222> (1)..(6750)

<400> 3
atg gcg ttc cac tgg tgc gac aac aat ctg cac acc acc gtg ttc acg 48
Met Ala Phe His Trp Cys Asp Asn Asn Leu His Thr Thr Val Phe Thr
1               5                   10                  15

ccg cgc gac ttt cag gtg gag cta ctg gcc acc gcc tac gag cgg aac 96
Pro Arg Asp Phe Gln Val Glu Leu Leu Ala Thr Ala Tyr Glu Arg Asn
            20                  25                  30

acg at[?] att tgc ctg ggc cat cga agt tcc aag gag ttt ata gcc ctc 144
Thr Ile Ile Cys Leu Gly His Arg Ser Ser Lys Glu Phe Ile Ala Leu
        35                  40                  45

aag ctg ctc cag gag ctg tcg cgt cga gca cgc cga cat ggt cgt gtc 192
Lys Leu Leu Gln Glu Leu Ser Arg Arg Ala Arg Arg His Gly Arg Val
    50                  55                  60

agt gtc tat ctc agt tgc gag gtt ggc acc agc acg gaa cca tgc tcc 240
Ser Val Tyr Leu Ser Cys Glu Val Gly Thr Ser Thr Glu Pro Cys Ser
65                  70                  75                  80

atc tac acg atg ctc acc cac ttg act gac ctg cgg gtg tgg cag gag 288
Ile Tyr Thr Met Leu Thr His Leu Thr Asp Leu Arg Val Trp Gln Glu
                85                  90                  95

cag ccg gat atg caa att ccc ttt gat cat tgc tgg acg gac tat cac 336
Gln Pro Asp Met Gln Ile Pro Phe Asp His Cys Trp Thr Asp Tyr His
            100                 105                 110

gtt tcc atc cta cgg cca gag gga ttt ctt tat ctg ctc gaa act cgc 384
Val Ser Ile Leu Arg Pro Glu Gly Phe Leu Tyr Leu Leu Glu Thr Arg
        115                 120                 125

gag ctg ctg ctg agc agc gtc gaa ctg atc gtg ctg gaa gat tgt cat 432
Glu Leu Leu Leu Ser Ser Val Glu Leu Ile Val Leu Glu Asp Cys His
    130                 135                 140

gac agc gcc gtt tat cag agg ata agg cct ctg ttc gag aat cac att 480
Asp Ser Ala Val Tyr Gln Arg Ile Arg Pro Leu Phe Glu Asn His Ile
145                 150                 155                 160

atg cca gcg cca ccg gcg gac agg cca cgg att ctc gga ctc gct gga 52[?]
Met Pro Ala Pro Pro Ala Asp Arg Pro Arg Ile Leu Gly Leu Ala Gly
                165                 170                 175

ccg ctg cac agc gcc gga tgt gag ctg cag caa ctg agc gcc atg ctg 57[?]
Pro Leu His Ser Ala Gly Cys Glu Leu Gln Gln Leu Ser Ala Met Leu
            180                 185                 190

gcc acc ctg gag cag agt gtg ctt tgc cag atc gag acg gcc agt gat 624
Ala Thr Leu Glu Gln Ser Val Leu Cys Gln Ile Glu Thr Ala Ser Asp
        195                 200                 205

att gtc acc gtg ttg cgt tac tgt tcc cga ccg cac gaa tac atc gta 672
Ile Val Thr Val Leu Arg Tyr Cys Ser Arg Pro His Glu Tyr Ile Val
    210                 215                 220

cag tgc gcc ccc ttc gag atg gac gaa ctg tcc ctg gtg ctt gcc gat 720
Gln Cys Ala Pro Phe Glu Met Asp Glu Leu Ser Leu Val Leu Ala Asp
225                 230                 235                 240

gtg ctc aac aca cac aag tcc ttt tta ttg gac cac cgc tac gat ccc 768

Val Leu Asn Thr His Lys Ser Phe Leu Leu Asp His Arg Tyr Asp Pro
                245                 250                 255

tac gaa atc tac ggc aca gac cag ttt atg gac gaa ctg aaa gac ata 816
Tyr Glu Ile Tyr Gly Thr Asp Gln Phe Met Asp Glu Leu Lys Asp Ile
            260                 265                 270

ccc gat ccc aag gtg gac ccc ctg aac gtc atc aac tca cta ctg gtc 864
Pro Asp Pro Lys Val Asp Pro Leu Asn Val Ile Asn Ser Leu Leu Val
        275                 280                 285

gtg ctg cac gag atg ggt cct tgg tgc acg cag cgg gct gca cat cac 912
Val Leu His Glu Met Gly Pro Trp Cys Thr Gln Arg Ala Ala His His
    290                 295                 300

ttt tac caa tgc aat gag aag tta aag gtg aag acg ccg cac gaa cgt 960
Phe Tyr Gln Cys Asn Glu Lys Leu Lys Val Lys Thr Pro His Glu Arg
305                 310                 315                 320

cac tac ttg ctg tac tgc cta gtg agc acg gcc ctt atc caa ctg tac 1008
His Tyr Leu Leu Tyr Cys Leu Val Ser Thr Ala Leu Ile Gln Leu Tyr
                325                 330                 335

tcc ctc tgc gaa cac gca ttc cat cga cat tta gga agt ggc agc gat 1056
Ser Leu Cys Glu His Ala Phe His Arg His Leu Gly Ser Gly Ser Asp
            340                 345                 350

tca cgc cag acc atc gaa cgc tat tcc agc ccc aag gtg cga cgt ctg 1104
Ser Arg Gln Thr Ile Glu Arg Tyr Ser Ser Pro Lys Val Arg Arg Leu
        355                 360                 365

ttg cag aca ctg agg tgc ttc aag ccg gaa gag gtg cac acc caa gcg 1152
Leu Gln Thr Leu Arg Cys Phe Lys Pro Glu Glu Val His Thr Gln Ala
    370                 375                 380

gac gga ctg cgc aga atg cgg cat cag gtg gat cag gcg gac ttc aat 1200
Asp Gly Leu Arg Arg Met Arg His Gln Val Asp Gln Ala Asp Phe Asn

385                390                395                400

cgg tta tct cat acg ctg gaa agc aag tgc cga atg gtg gat caa atg      1248
Arg Leu Ser His Thr Leu Glu Ser Lys Cys Arg Met Val Asp Gln Met
            405                410                415

gac caa ccg ccg acg gag aca cga gcc ctg gtg gcc act ctt gag cag      1296
Asp Gln Pro Pro Thr Glu Thr Arg Ala Leu Val Ala Thr Leu Glu Gln
        420                425                430

att ctg cac acg aca gag gac agg cag acg aac aga agc gcc gct cgg      1344
Ile Leu His Thr Thr Glu Asp Arg Gln Thr Asn Arg Ser Ala Ala Arg
    435                440                445

gtg act cct act cct act ccc gct cat gcg aag ccg aaa cct agc tct      1392
Val Thr Pro Thr Pro Thr Pro Ala His Ala Lys Pro Lys Pro Ser Ser
    450                455                460

ggt gcc aac act gca caa cca cga act cgt aga cgt gtg tac acc agg      1440
Gly Ala Asn Thr Ala Gln Pro Arg Thr Arg Arg Arg Val Tyr Thr Arg
465                470                475                480

cgc cac cac cgg gat cac aat gat ggc agc gac acg ctc tgc gca ctg      1488
Arg His His Arg Asp His Asn Asp Gly Ser Asp Thr Leu Cys Ala Leu
            485                490                495

att tac tgc aac cag aac cac acg gct cgc gtg ctc ttt gag ctt cta      1536
Ile Tyr Cys Asn Gln Asn His Thr Ala Arg Val Leu Phe Glu Leu Leu
        500                505                510

gcg gag att agc aga cgt gat ccc gat ctc aag ttc cta cgc tgc cag      1584
Ala Glu Ile Ser Arg Arg Asp Pro Asp Leu Lys Phe Leu Arg Cys Gln
    515                520                525

tac acc acg gac cgg gtg gca gat ccc acc acg gag ccc aaa gag gct      1632
Tyr Thr Thr Asp Arg Val Ala Asp Pro Thr Thr Glu Pro Lys Glu Ala
    530                535                540

gag ttg gag cac cgg cgg cag gaa gag gtg cta aag cgc ttc cgc atg 1680
Glu Leu Glu His Arg Arg Gln Glu Glu Val Leu Lys Arg Phe Arg Met
545                 550                 555                 560

cat gac tgc aat gtc ctg atc ggt act tcg gtg ctg gaa gag ggc atc 1728
His Asp Cys Asn Val Leu Ile Gly Thr Ser Val Leu Glu Glu Gly Ile
                565                 570                 575

gat gtg ccc aag tgc aat ttg gtt gtg cgc tgg gat ccg cca acc aca 1776
Asp Val Pro Lys Cys Asn Leu Val Val Arg Trp Asp Pro Pro Thr Thr
            580                 585                 590

tat cgc agt tac gtt cag tgc aaa ggt cga gcc cgt gct gct cca gcc 1824
Tyr Arg Ser Tyr Val Gln Cys Lys Gly Arg Ala Arg Ala Ala Pro Ala
        595                 600                 605

tat cat gtc att ctc gtc gct ccg agt tat aaa agc cca act gtg ggg 1872
Tyr His Val Ile Leu Val Ala Pro Ser Tyr Lys Ser Pro Thr Val Gly
    610                 615                 620

tca gtg cag ctg acc gat cgg agt cat cgg tat att tgc gcg act ggt 1920
Ser Val Gln Leu Thr Asp Arg Ser His Arg Tyr Ile Cys Ala Thr Gly
625                 630                 635                 640

gat act aca gag gcg gac agc gac tct gat gat tca gcg atg cca aac 1968
Asp Thr Thr Glu Ala Asp Ser Asp Ser Asp Asp Ser Ala Met Pro Asn
                645                 650                 655

tcg tcc ggc tcg gat ccc tat act ttt ggc acg gca cgc gga acc gtg 2016
Ser Ser Gly Ser Asp Pro Tyr Thr Phe Gly Thr Ala Arg Gly Thr Val
            660                 665                 670

aag atc ctc aac ccc gaa gtg ttc agt aaa caa cca ccg aca gcg tgc 2064
Lys Ile Leu Asn Pro Glu Val Phe Ser Lys Gln Pro Pro Thr Ala Cys
        675                 680                 685

gac att aag ctg cag gag atc cag gac gaa ttg cca gcc gca gcg cag 2112
Asp Ile Lys Leu Gln Glu Ile Gln Asp Glu Leu Pro Ala Ala Ala Gln
    690                 695                 700

ctg gat acg agc aac tcc agc gac gaa gcc gtc agc atg agt aac acg 2160
Leu Asp Thr Ser Asn Ser Ser Asp Glu Ala Val Ser Met Ser Asn Thr
705                 710                 715                 720

tct cca agc gag agc agt aca gaa caa aaa tcc aga cgc ttc cag tgc 2208
Ser Pro Ser Glu Ser Ser Thr Glu Gln Lys Ser Arg Arg Phe Gln Cys
                725                 730                 735

gag ctg agc tct tta acg gag cca gaa gac aca agt gat act aca gcc 2256
Glu Leu Ser Ser Leu Thr Glu Pro Glu Asp Thr Ser Asp Thr Thr Ala
            740                 745                 750

gaa atc gat act gct cat agt tta gcc agc acc acg aag gac ttg gtg 2304
Glu Ile Asp Thr Ala His Ser Leu Ala Ser Thr Thr Lys Asp Leu Val
        755                 760                 765

cat caa atg gca cag tat cgc gaa atc gag cag atg ctg cta tcc aag 2352
His Gln Met Ala Gln Tyr Arg Glu Ile Glu Gln Met Leu Leu Ser Lys
    770                 775                 780

tgc gcc aac aca gag ccg ccg gag cag gag cag agt gag gcg gaa cgt 2400
Cys Ala Asn Thr Glu Pro Pro Glu Gln Glu Gln Ser Glu Ala Glu Arg
785                 790                 795                 800

ttt agt gcc tgc ctg gcc gca tac cga ccc aag ccg cac ctg cta aca 2448
Phe Ser Ala Cys Leu Ala Ala Tyr Arg Pro Lys Pro His Leu Leu Thr
                805                 810                 815

ggc gcc tcc gtg gat ctg ggt tct gct ata gct ttg gtc aac aag tac 2496
Gly Ala Ser Val Asp Leu Gly Ser Ala Ile Ala Leu Val Asn Lys Tyr
            820                 825                 830

tgc gcc cga ctg cca agc gac acg ttc acc aag ttg acg gcg ttg tgg 2544

Cys Ala Arg Leu Pro Ser Asp Thr Phe Thr Lys Leu Thr Ala Leu Trp
        835                 840                 845

cgc tgc acc cga aac gaa agg gct gga gtg acc ctg ttt cag tac aca 2592
Arg Cys Thr Arg Asn Glu Arg Ala Gly Val Thr Leu Phe Gln Tyr Thr
    850                 855                 860

ctc cgt ctg ccc atc aac tcg cca ttg aag cat gac att gtg ggt ctt 2640
Leu Arg Leu Pro Ile Asn Ser Pro Leu Lys His Asp Ile Val Gly Leu
865                 870                 875                 880

ccg atg cca act caa aca ttg gcc cgc cga ctg gct gcc ttg cag gct 2688
Pro Met Pro Thr Gln Thr Leu Ala Arg Arg Leu Ala Ala Leu Gln Ala
                885                 890                 895

tgc gtg gaa ctg cac agg atc ggt gag tta gac gat cag ttg cag cct 2736
Cys Val Glu Leu His Arg Ile Gly Glu Leu Asp Asp Gln Leu Gln Pro
            900                 905                 910

atc ggc aag gag gga ttt cgt gcc ctg gag ccg gac tgg gag tgc ttt 2784
Ile Gly Lys Glu Gly Phe Arg Ala Leu Glu Pro Asp Trp Glu Cys Phe
        915                 920                 925

gaa ctg gag cca gag gac gaa cag att gtg cag cta agc gat gaa cca 2832
Glu Leu Glu Pro Glu Asp Glu Gln Ile Val Gln Leu Ser Asp Glu Pro
    930                 935                 940

cgt ccg gga aca acg aag cgt cgt cag tac tat tac aaa cgc att gca 2880
Arg Pro Gly Thr Thr Lys Arg Arg Gln Tyr Tyr Tyr Lys Arg Ile Ala
945                 950                 955                 960

tcc gaa ttt tgc gat tgc cgt ccc gtt gcc gga gcg cca tgc tat ttg 2928
Ser Glu Phe Cys Asp Cys Arg Pro Val Ala Gly Ala Pro Cys Tyr Leu
                965                 970                 975

tac ttt atc caa ctg acg ctc caa tgt ccg att ccc gaa gag caa aac 2976
Tyr Phe Ile Gln Leu Thr Leu Gln Cys Pro Ile Pro Glu Glu Gln Asn

            980                 985                 990

acg cgg gga cgc aag att tat ccg ccc gaa gat gcg cag cag gga ttc 3024
Thr Arg Gly Arg Lys Ile Tyr Pro Pro Glu Asp Ala Gln Gln Gly Phe
        995                 1000                1005

ggc att cta acc acc aaa cgg ata ccc aag ctg agt gct ttc tcg 3069
Gly Ile Leu Thr Thr Lys Arg Ile Pro Lys Leu Ser Ala Phe Ser
    1010                1015                1020

ata ttc acg cgt tcc ggt gag gtg aag gtt tcc ctg gag tta gct 3114
Ile Phe Thr Arg Ser Gly Glu Val Lys Val Ser Leu Glu Leu Ala
    1025                1030                1035

aag gaa cgc gtg att cta act agc gaa caa ata gtc tgc atc aac 3159
Lys Glu Arg Val Ile Leu Thr Ser Glu Gln Ile Val Cys Ile Asn
    1040                1045                1050

gga ttt tta aac tac acg ttc acc aat gta ctg cgt ttg caa aag 3204
Gly Phe Leu Asn Tyr Thr Phe Thr Asn Val Leu Arg Leu Gln Lys
    1055                1060                1065

ttt ctg atg ctc ttc gat ccg gac tcc acg gaa aat tgt gta ttc 3249
Phe Leu Met Leu Phe Asp Pro Asp Ser Thr Glu Asn Cys Val Phe
    1070                1075                1080

att gtg ccc acc gtg aag gca cca gct ggc ggc aag cac atc gac 3294
Ile Val Pro Thr Val Lys Ala Pro Ala Gly Gly Lys His Ile Asp
    1085                1090                1095

tgg cag ttt ctg gag ctg atc caa gcg aat gga aat aca atg cca 3339
Trp Gln Phe Leu Glu Leu Ile Gln Ala Asn Gly Asn Thr Met Pro
    1100                1105                1110

cgg gca gtg ccc gat gag gag cgc cag gcg cag ccg ttt gat ccg 3384
Arg Ala Val Pro Asp Glu Glu Arg Gln Ala Gln Pro Phe Asp Pro
    1115                1120                1125

caa cgc ttc cag gat gcc gtc gtt atg ccg tgg tat cgc aac cag 3429
Gln Arg Phe Gln Asp Ala Val Val Met Pro Trp Tyr Arg Asn Gln
    1130                1135                1140

gat caa ccg cag tat ttc tat gtg gcg gag ata tgt cca cat cta 3474
Asp Gln Pro Gln Tyr Phe Tyr Val Ala Glu Ile Cys Pro His Leu
    1145                1150                1155

tcc cca ctc agc tgc ttc cct ggt gac aac tac cgc acg ttc aag 3519
Ser Pro Leu Ser Cys Phe Pro Gly Asp Asn Tyr Arg Thr Phe Lys
    1160                1165                1170

cac tac tac ctc gtc aag tat ggt ctg acc ata cag aat acc tcg 3564
His Tyr Tyr Leu Val Lys Tyr Gly Leu Thr Ile Gln Asn Thr Ser
    1175                1180                1185

cag ccg cta ttg gac gtg gat cac acc agt gcg cgg tta aac ttc 3609
Gln Pro Leu Leu Asp Val Asp His Thr Ser Ala Arg Leu Asn Phe
    1190                1195                1200

ctc acg cca cga tac gtt aat cgc aag ggc gtt gct ctg ccc act 3654
Leu Thr Pro Arg Tyr Val Asn Arg Lys Gly Val Ala Leu Pro Thr
    1205                1210                1215

agt tcg gag gag aca aag cgg gca aag cgc gag aat ctc gaa cag 3699
Ser Ser Glu Glu Thr Lys Arg Ala Lys Arg Glu Asn Leu Glu Gln
    1220                1225                1230

aag cag atc ctt gtg cca gag ctc tgc act gtg cat cca ttc ccc 3744
Lys Gln Ile Leu Val Pro Glu Leu Cys Thr Val His Pro Phe Pro
    1235                1240                1245

gcc tcc ttg tgg cga act gcc gtg tgc ctg ccc tgc atc ctg tac 3789
Ala Ser Leu Trp Arg Thr Ala Val Cys Leu Pro Cys Ile Leu Tyr
    1250                1255                1260

cgc ata aat ggt ctt cta ttg gcc gac gat att cgg aaa cag gtt 3834
Arg Ile Asn Gly Leu Leu Leu Ala Asp Asp Ile Arg Lys Gln Val
    1265                1270                1275

tct gcg gat ctg ggg ctg gga agg caa cag atc gaa gat gag gat 3879
Ser Ala Asp Leu Gly Leu Gly Arg Gln Gln Ile Glu Asp Glu Asp
    1280                1285                1290

ttc gag tgg ccc atg ctg gac ttt ggg tgg agt cta tcg gag gtg 3924
Phe Glu Trp Pro Met Leu Asp Phe Gly Trp Ser Leu Ser Glu Val
    1295                1300                1305

ctc aag aaa tcg cgg gag tcc aaa caa aag gag tcc ctt aag gat 3969
Leu Lys Lys Ser Arg Glu Ser Lys Gln Lys Glu Ser Leu Lys Asp
    1310                1315                1320

gat act att aat ggc aaa gac tta gct gat gtt gaa aag aaa ccg 4014
Asp Thr Ile Asn Gly Lys Asp Leu Ala Asp Val Glu Lys Lys Pro
    1325                1330                1335

act agc gag gag acc caa cta gat aag gat tca aaa gac gat aag 4059
Thr Ser Glu Glu Thr Gln Leu Asp Lys Asp Ser Lys Asp Asp Lys
    1340                1345                1350

gtt gag aaa agt gct att gaa cta atc att gag gga gag gag aag 4104
Val Glu Lys Ser Ala Ile Glu Leu Ile Ile Glu Gly Glu Glu Lys
    1355                1360                1365

ctg caa gag gct gat gac ttc att gag ata ggc act tgg tca aac 4149
Leu Gln Glu Ala Asp Asp Phe Ile Glu Ile Gly Thr Trp Ser Asn
    1370                1375                1380

gat atg gcc gac gat ata gct agt ttt aac caa gaa gac gac gac 4194
Asp Met Ala Asp Asp Ile Ala Ser Phe Asn Gln Glu Asp Asp Asp
    1385                1390                1395

gag gat gac gcc ttc cat ctc cca gtt tta ccg gca aac gtt aag 4239

Glu Asp Asp Ala Phe His Leu Pro Val Leu Pro Ala Asn Val Lys
    1400                1405                1410

ttc tgt gat cag caa acg cgc tac ggt tcg ccc aca ttt tgg gat 4284
Phe Cys Asp Gln Gln Thr Arg Tyr Gly Ser Pro Thr Phe Trp Asp
    1415                1420                1425

gtg agc aat ggc gaa agc ggc ttc aag ggt cca aag agc agt cag 4329
Val Ser Asn Gly Glu Ser Gly Phe Lys Gly Pro Lys Ser Ser Gln
    1430                1435                1440

aat aag cag ggt ggc aag ggc aaa gca aag ggt ccg gca aag ccc 4374
Asn Lys Gln Gly Gly Lys Gly Lys Ala Lys Gly Pro Ala Lys Pro
    1445                1450                1455

aca ttt aac tat tat gac tcg gac aat tcg ctg ggt tcc agc tac 4419
Thr Phe Asn Tyr Tyr Asp Ser Asp Asn Ser Leu Gly Ser Ser Tyr
    1460                1465                1470

gat gac gac gat aac gca ggt ccg ctc aat tac atg cat cac aac 4464
Asp Asp Asp Asp Asn Ala Gly Pro Leu Asn Tyr Met His His Asn
    1475                1480                1485

tac agt tcg gat gac gac gat gtg gca gat gat atc gat gcg gga 4509
Tyr Ser Ser Asp Asp Asp Asp Val Ala Asp Asp Ile Asp Ala Gly
    1490                1495                1500

cgc att gcg ttc acc tcc aag aat gaa gcg gag act att gaa acc 4554
Arg Ile Ala Phe Thr Ser Lys Asn Glu Ala Glu Thr Ile Glu Thr
    1505                1510                1515

gca cag gaa gtg gaa aag cgc cag aag cag ctg tcc atc atc cag 4599
Ala Gln Glu Val Glu Lys Arg Gln Lys Gln Leu Ser Ile Ile Gln
    1520                1525                1530

gcg acc aat gct aac gag cgg cag tat cag cag aca aag aac ctg 4644
Ala Thr Asn Ala Asn Glu Arg Gln Tyr Gln Gln Thr Lys Asn Leu

1535                1540                1545

ctc att  gga ttc aat ttt aag  cat gag gac cag aag  gaa cct gcc      4689
Leu Ile  Gly Phe Asn Phe Lys  His Glu Asp Gln Lys  Glu Pro Ala
    1550                1555                1560

act ata  aga tat gaa gaa tcc  ata gct aag ctc aaa  acg gaa ata      4734
Thr Ile  Arg Tyr Glu Glu Ser  Ile Ala Lys Leu Lys  Thr Glu Ile
    1565                1570                1575

gaa tcc  ggc ggc atg ttg gtg  ccg cac gac cag cag  ttg gtt cta      4779
Glu Ser  Gly Gly Met Leu Val  Pro His Asp Gln Gln  Leu Val Leu
    1580                1585                1590

aaa aga  agt gat gcc gct gag  gct cag gtt gca aag  gta tcg atg      4824
Lys Arg  Ser Asp Ala Ala Glu  Ala Gln Val Ala Lys  Val Ser Met
    1595                1600                1605

atg gag  cta ttg aag cag ctg  ctg ccg tat gta aat  gaa gat gtg      4869
Met Glu  Leu Leu Lys Gln Leu  Leu Pro Tyr Val Asn  Glu Asp Val
    1610                1615                1620

ctg gcc  aaa aag ctg ggt gat  agg cgc gag ctt ctg  ctg tcg gat      4914
Leu Ala  Lys Lys Leu Gly Asp  Arg Arg Glu Leu Leu  Leu Ser Asp
    1625                1630                1635

ttg gta  gag cta aat gca gat  tgg gta gcg cga cat  gag cag gag      4959
Leu Val  Glu Leu Asn Ala Asp  Trp Val Ala Arg His  Glu Gln Glu
    1640                1645                1650

acc tac  aat gta atg gga tgc  gga gat agt ttt gac  aac tat aac      5004
Thr Tyr  Asn Val Met Gly Cys  Gly Asp Ser Phe Asp  Asn Tyr Asn
    1655                1660                1665

gat cat  cat cgg ctg aac ttg  gat gaa aag caa ctg  aaa ctg caa      5049
Asp His  His Arg Leu Asn Leu  Asp Glu Lys Gln Leu  Lys Leu Gln
    1670                1675                1680

tac gaa cga att gaa att gag cca cct act tcc acg aag gcc ata 5094
Tyr Glu Arg Ile Glu Ile Glu Pro Pro Thr Ser Thr Lys Ala Ile
    1685                1690                1695

acc tca gcc ata tta cca gct ggc ttc agt ttc gat cga caa ccg 5139
Thr Ser Ala Ile Leu Pro Ala Gly Phe Ser Phe Asp Arg Gln Pro
    1700                1705                1710

gat cta gtg ggc cat cca gga ccc agt ccc agc atc att ttg caa 5184
Asp Leu Val Gly His Pro Gly Pro Ser Pro Ser Ile Ile Leu Gln
    1715                1720                1725

gcc ctc aca atg tcc aat gct aac gat ggc atc aat ctg gag cga 5229
Ala Leu Thr Met Ser Asn Ala Asn Asp Gly Ile Asn Leu Glu Arg
    1730                1735                1740

ctg gag aca att gga gat tcc ttt cta aag tat gcc att acc acc 5274
Leu Glu Thr Ile Gly Asp Ser Phe Leu Lys Tyr Ala Ile Thr Thr
    1745                1750                1755

tac ttg tac atc acc tac gag aat gtg cac gag gga aaa cta agt 5319
Tyr Leu Tyr Ile Thr Tyr Glu Asn Val His Glu Gly Lys Leu Ser
    1760                1765                1770

cac ctg cgc tcc aag cag gtt gcc aat ctc aat ctc tat cgt ctg 5364
His Leu Arg Ser Lys Gln Val Ala Asn Leu Asn Leu Tyr Arg Leu
    1775                1780                1785

ggc aga cgt aag aga ctg ggt gaa tat atg ata gcc act aaa ttc 5409
Gly Arg Arg Lys Arg Leu Gly Glu Tyr Met Ile Ala Thr Lys Phe
    1790                1795                1800

gag ccg cac gac aat tgg ctg cca ccc tgc tac tac gtg cca aag 5454
Glu Pro His Asp Asn Trp Leu Pro Pro Cys Tyr Tyr Val Pro Lys
    1805                1810                1815

gag cta gag aag gcg ctc atc gag gcg aag atc ccc act cac cat 5499
Glu Leu Glu Lys Ala Leu Ile Glu Ala Lys Ile Pro Thr His His
    1820                1825                1830

tgg aag ctg gcc gat ctg cta gac att aag aac cta agc agt gtg 5544
Trp Lys Leu Ala Asp Leu Leu Asp Ile Lys Asn Leu Ser Ser Val
    1835                1840                1845

caa atc tgc gag atg gtt cgc gaa aaa gcc gat gcc ctg ggc ttg 5589
Gln Ile Cys Glu Met Val Arg Glu Lys Ala Asp Ala Leu Gly Leu
    1850                1855                1860

gag cag a[?]t ggg ggt gcc caa aat gga caa ctt gac gac tcc aat 5634
Glu Gln Asn Gly Gly Ala Gln Asn Gly Gln Leu Asp Asp Ser Asn
    1865                1870                1875

gat agc tgc aat gat ttt agc tgt ttt att ccc tac aac ctt gtt 5679
Asp Ser Cys Asn Asp Phe Ser Cys Phe Ile Pro Tyr Asn Leu Val
    1880                1885                1890

tcg caa cac agc att ccg gat aag tct att gcc gat tgc gtc gaa 5724
Ser Gln His Ser Ile Pro Asp Lys Ser Ile Ala Asp Cys Val Glu
    1895                1900                1905

gcc ctc att gga gcc tat ctc att gag tgc gga ccc cga ggg gct 5769
Ala Leu Ile Gly Ala Tyr Leu Ile Glu Cys Gly Pro Arg Gly Ala
    1910                1915                1920

tta ctc ttt atg gcc tgg ctg ggc gtg aga gtg ctc cct atc aca 5814
Leu Leu Phe Met Ala Trp Leu Gly Val Arg Val Leu Pro Ile Thr
    1925                1930                1935

agg cag ttg gac ggg ggt aac cag gag caa cga ata ccc ggt agc 5859
Arg Gln Leu Asp Gly Gly Asn Gln Glu Gln Arg Ile Pro Gly Ser
    1940                1945                1950

aca aaa ccg aat gcc gaa aat gtg gtc acc gtt tac ggt gca tgg 5904

Thr Lys Pro Asn Ala Glu Asn Val Val Thr Val Tyr Gly Ala Trp
    1955                1960                1965

ccc acg ccg cgt agt cca ctg ctg cac ttt gct cca aat gct acg 5949
Pro Thr Pro Arg Ser Pro Leu Leu His Phe Ala Pro Asn Ala Thr
    1970                1975                1980

gag gag ctg gac cag tta cta agc ggc ttt gag gag ttt gag gag 5994
Glu Glu Leu Asp Gln Leu Leu Ser Gly Phe Glu Glu Phe Glu Glu
    1985                1990                1995

agt ttg gga tac aag ttc cgg gat cgg tcg tac ctg ttg caa gcc 6039
Ser Leu Gly Tyr Lys Phe Arg Asp Arg Ser Tyr Leu Leu Gln Ala
    2000                2005                2010

atg aca cat gcc agt tac acg ccc aat cga ttg acg gat tgc tat 6084
Met Thr His Ala Ser Tyr Thr Pro Asn Arg Leu Thr Asp Cys Tyr
    2015                2020                2025

cag cgt ctg gag ttc ctg ggc gat gct gtt cta gat tac ctc att 6129
Gln Arg Leu Glu Phe Leu Gly Asp Ala Val Leu Asp Tyr Leu Ile
    2030                2035                2040

acg cgg cat tta tac gaa gat ccc cgc cag cat tct cca ggc gca 6174
Thr Arg His Leu Tyr Glu Asp Pro Arg Gln His Ser Pro Gly Ala
    2045                2050                2055

tta acg gat ttg cgg tca gca ctg gtg aat aat aca ata ttc gcc 6219
Leu Thr Asp Leu Arg Ser Ala Leu Val Asn Asn Thr Ile Phe Ala
    2060                2065                2070

tcc ctg gct gtt cgc cat ggc ttc cac aag ttc ttc cgg cac ctc 6264
Ser Leu Ala Val Arg His Gly Phe His Lys Phe Phe Arg His Leu
    2075                2080                2085

tcg ccg ggc ctt aac gat gtg att gac cgt ttt gtg cgg atc cag 6309
Ser Pro Gly Leu Asn Asp Val Ile Asp Arg Phe Val Arg Ile Gln

2090                2095                2100

cag gag  aat gga cac tgc atc  agt gag gag tac tac  tta ttg tcc      6354
Gln Glu  Asn Gly His Cys Ile  Ser Glu Glu Tyr Tyr  Leu Leu Ser
    2105                2110                2115

gag gag  gag tgc gat gac gcc  gag gac gtt gag gtg  ccc aag gca      6399
Glu Glu  Glu Cys Asp Asp Ala  Glu Asp Val Glu Val  Pro Lys Ala
    2120                2125                2130

ttg ggc  gac gtt ttc gag tcg  atc gca ggt gcc att  ttt ctc gac      6444
Leu Gly  Asp Val Phe Glu Ser  Ile Ala Gly Ala Ile  Phe Leu Asp
    2135                2140                2145

tca aac  atg tcg ctg gac gtg  gtt tgg cac gta tat  agc aac atg      6489
Ser Asn  Met Ser Leu Asp Val  Val Trp His Val Tyr  Ser Asn Met
    2150                2155                2160

atg agc  ccg gag atc gag cag  ttc agc aac tca gtg  cca aaa tcg      6534
Met Ser  Pro Glu Ile Glu Gln  Phe Ser Asn Ser Val  Pro Lys Ser
    2165                2170                2175

ccc att  cgg gag ctc ctc gag  ctg gag ccg gaa acc  gcc aag ttc      6579
Pro Ile  Arg Glu Leu Leu Glu  Leu Glu Pro Glu Thr  Ala Lys Phe
    2180                2185                2190

ggc aag  ccc gag aag ctg gcg  gat ggg cga cgg gtg  cgc gtt acc      6624
Gly Lys  Pro Glu Lys Leu Ala  Asp Gly Arg Arg Val  Arg Val Thr
    2195                2200                2205

gtg gat  gtc ttc tgc aaa gga  acc ttc cgt ggc atc  gga cgc aac      6669
Val Asp  Val Phe Cys Lys Gly  Thr Phe Arg Gly Ile  Gly Arg Asn
    2210                2215                2220

tat cgc  att gcc aag tgc acg  gcg gcc aaa tgc gca  ttg cgc caa      6714
Tyr Arg  Ile Ala Lys Cys Thr  Ala Ala Lys Cys Ala  Leu Arg Gln
    2225                2230                2235

ctc aaa aag cag ggc ttg ata gcc aaa aaa gac taa 6750
Leu Lys Lys Gln Gly Leu Ile Ala Lys Lys Asp
    2240                2245

<210> 4
<211> 2249
<212> PRT
<213> Drosophila melanogaster

<400> 4

Met Ala Phe His Trp Cys Asp Asn Asn Leu His Thr Thr Val Phe Thr
1               5                   10                  15

Pro Arg Asp Phe Gln Val Glu Leu Leu Ala Thr Ala Tyr Glu Arg Asn
            20                  25                  30

Thr Ile Ile Cys Leu Gly His Arg Ser Ser Lys Glu Phe Ile Ala Leu
        35                  40                  45

Lys Leu Leu Gln Glu Leu Ser Arg Arg Ala Arg Arg His Gly Arg Val
    50                  55                  60

Ser Val Tyr Leu Ser Cys Glu Val Gly Thr Ser Thr Glu Pro Cys Ser
65                  70                  75                  80

Ile Tyr Thr Met Leu Thr His Leu Thr Asp Leu Arg Val Trp Gln Glu
                85                  90                  95

Gln Pro Asp Met Gln Ile Pro Phe Asp His Cys Trp Thr Asp Tyr His
            100                 105                 110

Val Ser Ile Leu Arg Pro Glu Gly Phe Leu Tyr Leu Leu Glu Thr Arg
        115                 120                 125

Glu Leu Leu Leu Ser Ser Val Glu Leu Ile Val Leu Glu Asp Cys His
    130                 135                 140

Asp Ser Ala Val Tyr Gln Arg Ile Arg Pro Leu Phe Glu Asn His Ile
145                 150                 155                 160

Met Pro Ala Pro Pro Ala Asp Arg Pro Arg Ile Leu Gly Leu Ala Gly
                165                 170                 175

Pro Leu His Ser Ala Gly Cys Glu Leu Gln Gln Leu Ser Ala Met Leu
            180                 185                 190

Ala Thr Leu Glu Gln Ser Val Leu Cys Gln Ile Glu Thr Ala Ser Asp
        195                 200                 205

Ile Val Thr Val Leu Arg Tyr Cys Ser Arg Pro His Glu Tyr Ile Val
    210                 215                 220

Gln Cys Ala Pro Phe Glu Met Asp Glu Leu Ser Leu Val Leu Ala Asp
225                 230                 235                 240

Val Leu Asn Thr His Lys Ser Phe Leu Leu Asp His Arg Tyr Asp Pro

                245                 250                 255

Tyr Glu Ile Tyr Gly Thr Asp Gln Phe Met Asp Glu Leu Lys Asp Ile
            260                 265                 270

Pro Asp Pro Lys Val Asp Pro Leu Asn Val Ile Asn Ser Leu Leu Val
        275                 280                 285

Val Leu His Glu Met Gly Pro Trp Cys Thr Gln Arg Ala Ala His His
    290                 295                 300

Phe Tyr Gln Cys Asn Glu Lys Leu Lys Val Lys Thr Pro His Glu Arg
305                 310                 315                 320

His Tyr Leu Leu Tyr Cys Leu Val Ser Thr Ala Leu Ile Gln Leu Tyr
                325                 330                 335

Ser Leu Cys Glu His Ala Phe His Arg His Leu Gly Ser Gly Ser Asp
            340                 345                 350

Ser Arg Gln Thr Ile Glu Arg Tyr Ser Ser Pro Lys Val Arg Arg Leu
        355                 360                 365

Leu Gln Thr Leu Arg Cys Phe Lys Pro Glu Glu Val His Thr Gln Ala
    370                 375                 380

Asp Gly Leu Arg Arg Met Arg His Gln Val Asp Gln Ala Asp Phe Asn
385                 390                 395                 400

Arg Leu Ser His Thr Leu Glu Ser Lys Cys Arg Met Val Asp Gln Met
                405                 410                 415

Asp Gln Pro Pro Thr Glu Thr Arg Ala Leu Val Ala Thr Leu Glu Gln
            420                 425                 430

Ile Leu His Thr Thr Glu Asp Arg Gln Thr Asn Arg Ser Ala Ala Arg
        435                 440                 445

Val Thr Pro Thr Pro Thr Pro Ala His Ala Lys Pro Lys Pro Ser Ser
    450                 455                 460

Gly Ala Asn Thr Ala Gln Pro Arg Thr Arg Arg Arg Val Tyr Thr Arg
465                 470                 475                 480

Arg His His Arg Asp His Asn Asp Gly Ser Asp Thr Leu Cys Ala Leu
                485                 490                 495

Ile Tyr Cys Asn Gln Asn His Thr Ala Arg Val Leu Phe Glu Leu Leu
            500                 505                 510

Ala Glu Ile Ser Arg Arg Asp Pro Asp Leu Lys Phe Leu Arg Cys Gln
        515                 520                 525

Tyr Thr Thr Asp Arg Val Ala Asp Pro Thr Thr Glu Pro Lys Glu Ala
    530                 535                 540

Glu Leu Glu His Arg Arg Gln Glu Glu Val Leu Lys Arg Phe Arg Met
545                 550                 555                 560

His Asp Cys Asn Val Leu Ile Gly Thr Ser Val Leu Glu Glu Gly Ile
                565                 570                 575

Asp Val Pro Lys Cys Asn Leu Val Val Arg Trp Asp Pro Pro Thr Thr
            580                 585                 590

Tyr Arg Ser Tyr Val Gln Cys Lys Gly Arg Ala Arg Ala Ala Pro Ala
        595                 600                 605

Tyr His Val Ile Leu Val Ala Pro Ser Tyr Lys Ser Pro Thr Val Gly
    610                 615                 620

Ser Val Gln Leu Thr Asp Arg Ser His Arg Tyr Ile Cys Ala Thr Gly
625                 630                 635                 640

Asp Thr Thr Glu Ala Asp Ser Asp Ser Asp Asp Ser Ala Met Pro Asn
                645                 650                 655

Ser Ser Gly Ser Asp Pro Tyr Thr Phe Gly Thr Ala Arg Gly Thr Val
            660                 665                 670

Lys Ile Leu Asn Pro Glu Val Phe Ser Lys Gln Pro Pro Thr Ala Cys
        675                 680                 685

Asp Ile Lys Leu Gln Glu Ile Gln Asp Glu Leu Pro Ala Ala Ala Gln
    690                 695                 700

Leu Asp Thr Ser Asn Ser Ser Asp Glu Ala Val Ser Met Ser Asn Thr
705                 710                 715                 720

Ser Pro Ser Glu Ser Ser Thr Glu Gln Lys Ser Arg Arg Phe Gln Cys
                725                 730                 735

Glu Leu Ser Ser Leu Thr Glu Pro Glu Asp Thr Ser Asp Thr Thr Ala
            740                 745                 750

Glu Ile Asp Thr Ala His Ser Leu Ala Ser Thr Thr Lys Asp Leu Val
        755                 760                 765

His Gln Met Ala Gln Tyr Arg Glu Ile Glu Gln Met Leu Leu Ser Lys
    770                 775                 780

Cys Ala Asn Thr Glu Pro Pro Glu Gln Glu Gln Ser Glu Ala Glu Arg
785                 790                 795                 800

Phe Ser Ala Cys Leu Ala Ala Tyr Arg Pro Lys Pro His Leu Leu Thr
                805                 810                 815

Gly Ala Ser Val Asp Leu Gly Ser Ala Ile Ala Leu Val Asn Lys Tyr
            820                 825                 830

Cys Ala Arg Leu Pro Ser Asp Thr Phe Thr Lys Leu Thr Ala Leu Trp

        835                 840                 845

Arg Cys Thr Arg Asn Glu Arg Ala Gly Val Thr Leu Phe Gln Tyr Thr
    850                 855                 860

Leu Arg Leu Pro Ile Asn Ser Pro Leu Lys His Asp Ile Val Gly Leu
865                 870                 875                 880

Pro Met Pro Thr Gln Thr Leu Ala Arg Arg Leu Ala Ala Leu Gln Ala
                885                 890                 895

Cys Val Glu Leu His Arg Ile Gly Glu Leu Asp Asp Gln Leu Gln Pro
            900                 905                 910

Ile Gly Lys Glu Gly Phe Arg Ala Leu Glu Pro Asp Trp Glu Cys Phe
        915                 920                 925

Glu Leu Glu Pro Glu Asp Glu Gln Ile Val Gln Leu Ser Asp Glu Pro
    930                 935                 940

Arg Pro Gly Thr Thr Lys Arg Arg Gln Tyr Tyr Tyr Lys Arg Ile Ala
945                 950                 955                 960

Ser Glu Phe Cys Asp Cys Arg Pro Val Ala Gly Ala Pro Cys Tyr Leu
                965                 970                 975

Tyr Phe Ile Gln Leu Thr Leu Gln Cys Pro Ile Pro Glu Glu Gln Asn
            980                 985                 990

Thr Arg Gly Arg Lys Ile Tyr Pro Pro Glu Asp Ala Gln Gln Gly Phe
        995                 1000                1005

Gly Ile Leu Thr Thr Lys Arg Ile Pro Lys Leu Ser Ala Phe Ser
    1010                1015                1020

Ile Phe Thr Arg Ser Gly Glu Val Lys Val Ser Leu Glu Leu Ala
    1025                1030                1035

Lys Glu Arg Val Ile Leu Thr Ser Glu Gln Ile Val Cys Ile Asn
    1040                1045                1050

Gly Phe Leu Asn Tyr Thr Phe Thr Asn Val Leu Arg Leu Gln Lys
    1055                1060                1065

Phe Leu Met Leu Phe Asp Pro Asp Ser Thr Glu Asn Cys Val Phe
    1070                1075                1080

Ile Val Pro Thr Val Lys Ala Pro Ala Gly Gly Lys His Ile Asp
    1085                1090                1095

Trp Gln Phe Leu Glu Leu Ile Gln Ala Asn Gly Asn Thr Met Pro
    1100                1105                1110

Arg Ala Val Pro Asp Glu Glu Arg Gln Ala Gln Pro Phe Asp Pro
    1115                1120                1125

Gln Arg Phe Gln Asp Ala Val Val Met Pro Trp Tyr Arg Asn Gln
    1130                1135                1140

Asp Gln Pro Gln Tyr Phe Tyr Val Ala Glu Ile Cys Pro His Leu
    1145                1150                1155

Ser Pro Leu Ser Cys Phe Pro Gly Asp Asn Tyr Arg Thr Phe Lys
    1160                1165                1170

His Tyr Tyr Leu Val Lys Tyr Gly Leu Thr Ile Gln Asn Thr Ser
    1175                1180                1185

Gln Pro Leu Leu Asp Val Asp His Thr Ser Ala Arg Leu Asn Phe
    1190                1195                1200

Leu Thr Pro Arg Tyr Val Asn Arg Lys Gly Val Ala Leu Pro Thr
    1205                1210                1215

Ser Ser Glu Glu Thr Lys Arg Ala Lys Arg Glu Asn Leu Glu Gln
    1220                1225                1230

Lys Gln Ile Leu Val Pro Glu Leu Cys Thr Val His Pro Phe Pro
    1235                1240                1245

Ala Ser Leu Trp Arg Thr Ala Val Cys Leu Pro Cys Ile Leu Tyr
    1250                1255                1260

Arg Ile  Asn Gly Leu Leu Leu  Ala Asp Asp Ile Arg  Lys Gln Val
1265                1270                1275

Ser Ala  Asp Leu Gly Leu Gly  Arg Gln Gln Ile Glu  Asp Glu Asp
1280                1285                1290

Phe Glu  Trp Pro Met Leu Asp  Phe Gly Trp Ser Leu  Ser Glu Val
1295                1300                1305

Leu Lys  Lys Ser Arg Glu Ser  Lys Gln Lys Glu Ser  Leu Lys Asp
1310                1315                1320

Asp Thr  Ile Asn Gly Lys Asp  Leu Ala Asp Val Glu  Lys Lys Pro
1325                1330                1335

Thr Ser  Glu Glu Thr Gln Leu  Asp Lys Asp Ser Lys  Asp Asp Lys
1340                1345                1350

Val Glu  Lys Ser Ala Ile Glu  Leu Ile Ile Glu Gly  Glu Glu Lys
1355                1360                1365

Leu Gln  Glu Ala Asp Asp Phe  Ile Glu Ile Gly Thr  Trp Ser Asn
1370                1375                1380

Asp Met  Ala Asp Asp Ile Ala  Ser Phe Asn Gln Glu  Asp Asp Asp
1385                1390                1395

Glu Asp  Asp Ala Phe His Leu  Pro Val Leu Pro Ala  Asn Val Lys

    1400                 1405                 1410

Phe Cys Asp Gln Gln Thr Arg Tyr Gly Ser Pro Thr Phe Trp Asp
    1415                1420                1425

Val Ser Asn Gly Glu Ser Gly Phe Lys Gly Pro Lys Ser Ser Gln
    1430                1435                1440

Asn Lys Gln Gly Gly Lys Gly Lys Ala Lys Gly Pro Ala Lys Pro
    1445                1450                1455

Thr Phe Asn Tyr Tyr Asp Ser Asp Asn Ser Leu Gly Ser Ser Tyr
    1460                1465                1470

Asp Asp Asp Asp Asn Ala Gly Pro Leu Asn Tyr Met His His Asn
    1475                1480                1485

Tyr Ser Ser Asp Asp Asp Asp Val Ala Asp Asp Ile Asp Ala Gly
    1490                1495                1500

Arg Ile Ala Phe Thr Ser Lys Asn Glu Ala Glu Thr Ile Glu Thr
    1505                1510                1515

Ala Gln Glu Val Glu Lys Arg Gln Lys Gln Leu Ser Ile Ile Gln
    1520                1525                1530

Ala Thr Asn Ala Asn Glu Arg Gln Tyr Gln Gln Thr Lys Asn Leu
    1535                1540                1545

Leu Ile Gly Phe Asn Phe Lys His Glu Asp Gln Lys Glu Pro Ala
    1550                1555                1560

Thr Ile Arg Tyr Glu Glu Ser Ile Ala Lys Leu Lys Thr Glu Ile
    1565                1570                1575

Glu Ser Gly Gly Met Leu Val Pro His Asp Gln Gln Leu Val Leu
    1580                1585                1590

Lys Arg Ser Asp Ala Ala Glu Ala Gln Val Ala Lys Val Ser Met
    1595                1600                1605

Met Glu Leu Leu Lys Gln Leu Leu Pro Tyr Val Asn Glu Asp Val
    1610                1615                1620

Leu Ala Lys Lys Leu Gly Asp Arg Arg Glu Leu Leu Leu Ser Asp
    1625                1630                1635

Leu Val Glu Leu Asn Ala Asp Trp Val Ala Arg His Glu Gln Glu
    1640                1645                1650

Thr Tyr Asn Val Met Gly Cys Gly Asp Ser Phe Asp Asn Tyr Asn
    1655                1660                1665

Asp His His Arg Leu Asn Leu Asp Glu Lys Gln Leu Lys Leu Gln
    1670                1675                1680

Tyr Glu 'Arg Ile Glu Ile Glu  Pro Pro Thr Ser Thr  Lys Ala Ile
1685                1690                1695

Thr Ser  Ala Ile Leu Pro Ala  Gly Phe Ser Phe Asp  Arg Gln Pro
1700                1705                1710

Asp Leu  Val Gly His Pro Gly  Pro Ser Pro Ser Ile  Ile Leu Gln
1715                1720                1725

Ala Leu  Thr Met Ser Asn Ala  Asn Asp Gly Ile Asn  Leu Glu Arg
1730                1735                1740

Leu Glu  Thr Ile Gly Asp Ser  Phe Leu Lys Tyr Ala  Ile Thr Thr
1745                1750                1755

Tyr Leu  Tyr Ile Thr Tyr Glu  Asn Val His Glu Gly  Lys Leu Ser
1760                1765                1770

His Leu  Arg Ser Lys Gln Val  Ala Asn Leu Asn Leu  Tyr Arg Leu
1775                1780                1785

Gly Arg  Arg Lys Arg Leu Gly  Glu Tyr Met Ile Ala  Thr Lys Phe
1790                1795                1800

Glu Pro  His Asp Asn Trp Leu  Pro Pro Cys Tyr Tyr  Val Pro Lys
1805                1810                1815

Glu Leu Glu Lys Ala Leu Ile Glu Ala Lys Ile Pro Thr His His
1820 1825 1830

Trp Lys Leu Ala Asp Leu Leu Asp Ile Lys Asn Leu Ser Ser Val
1835 1840 1845

Gln Ile Cys Glu Met Val Arg Glu Lys Ala Asp Ala Leu Gly Leu
1850 1855 1860

Glu Gln Asn Gly Gly Ala Gln Asn Gly Gln Leu Asp Asp Ser Asn
1865 1870 1875

Asp Ser Cys Asn Asp Phe Ser Cys Phe Ile Pro Tyr Asn Leu Val
1880 1885 1890

Ser Gln His Ser Ile Pro Asp Lys Ser Ile Ala Asp Cys Val Glu
1895 1900 1905

Ala Leu Ile Gly Ala Tyr Leu Ile Glu Cys Gly Pro Arg Gly Ala
1910 1915 1920

Leu Leu Phe Met Ala Trp Leu Gly Val Arg Val Leu Pro Ile Thr
1925 1930 1935

Arg Gln Leu Asp Gly Gly Asn Gln Glu Gln Arg Ile Pro Gly Ser
1940 1945 1950

Thr Lys Pro Asn Ala Glu Asn Val Val Thr Val Tyr Gly Ala Trp

1955 1960 1965

Pro Thr Pro Arg Ser Pro Leu Leu His Phe Ala Pro Asn Ala Thr
1970 1975 1980

Glu Glu Leu Asp Gln Leu Leu Ser Gly Phe Glu Glu Phe Glu Glu
1985 1990 1995

Ser Leu Gly Tyr Lys Phe Arg Asp Arg Ser Tyr Leu Leu Gln Ala
2000 2005 2010

Met Thr His Ala Ser Tyr Thr Pro Asn Arg Leu Thr Asp Cys Tyr
2015 2020 2025

Gln Arg Leu Glu Phe Leu Gly Asp Ala Val Leu Asp Tyr Leu Ile
2030 2035 2040

Thr Arg His Leu Tyr Glu Asp Pro Arg Gln His Ser Pro Gly Ala
2045 2050 2055

Leu Thr Asp Leu Arg Ser Ala Leu Val Asn Asn Thr Ile Phe Ala
2060 2065 2070

Ser Leu Ala Val Arg His Gly Phe His Lys Phe Phe Arg His Leu
2075 2080 2085

Ser Pro Gly Leu Asn Asp Val Ile Asp Arg Phe Val Arg Ile Gln
2090 2095 2100

Gln Glu  Asn Gly His Cys Ile  Ser Glu Glu Tyr Tyr  Leu Leu Ser
2105                2110                2115

Glu Glu  Glu Cys Asp Asp Ala  Glu Asp Val Glu Val  Pro Lys Ala
2120                2125                2130

Leu Gly  Asp Val Phe Glu Ser  Ile Ala Gly Ala Ile  Phe Leu Asp
2135                2140                2145

Ser Asn  Met Ser Leu Asp Val  Val Trp His Val Tyr  Ser Asn Met
2150                2155                2160

Met Ser  Pro Glu Ile Glu Gln  Phe Ser Asn Ser Val  Pro Lys Ser
2165                2170                2175

Pro Ile  Arg Glu Leu Leu Glu  Leu Glu Pro Glu Thr  Ala Lys Phe
2180                2185                2190

Gly Lys  Pro Glu Lys Leu Ala  Asp Gly Arg Arg Val  Arg Val Thr
2195                2200                2205

Val Asp  Val Phe Cys Lys Gly  Thr Phe Arg Gly Ile  Gly Arg Asn
2210                2215                2220

Tyr Arg  Ile Ala Lys Cys Thr  Ala Ala Lys Cys Ala  Leu Arg Gln
2225                2230                2235

Leu Lys Lys Gln Gly Leu Ile Ala Lys Lys Asp
2240 2245